(19) Europäisches Patentamt
European Patent Office
Office européen des brevets

(11) EP 1 024 785 B1

(12) **EUROPEAN PATENT SPECIFICATION**

(45) Date of publication and mention of the grant of the patent:
**15.01.2003 Bulletin 2003/03**

(21) Application number: **98953803.8**

(22) Date of filing: **20.10.1998**

(51) Int Cl.[7]: **A61K 7/50**, A61K 7/46, A61K 7/00

(86) International application number:
**PCT/US98/22212**

(87) International publication number:
**WO 99/021532 (06.05.1999 Gazette 1999/18)**

(54) **CLEANSING AND CONDITIONING ARTICLE FOR SKIN OR HAIR HAVING IMPROVED FRAGRANCE DELIVERY**

REINIGUNGS- UND KONDITIONIERUNGSGEGENSTAND FÜR DIE HAUT ODER DAS HAAR, MIT ERHÖHTER DUFTFREISETZUNG

PRODUIT DEMAQUILLANT ET REVITALISANT POUR LA PEAU OU LES CHEVEUX PRESENTANT DES QUALITES AMELIOREES DE DIFFUSION DE PARFUM

(84) Designated Contracting States:
**AT BE CH CY DE DK ES FI FR GB GR IE IT LI LU NL PT SE**

(30) Priority: **24.10.1997 US 957174**

(43) Date of publication of application:
**09.08.2000 Bulletin 2000/32**

(73) Proprietor: **THE PROCTER & GAMBLE COMPANY**
**Cincinnati, Ohio 45202 (US)**

(72) Inventors:
- **HASENOEHRL, Erik, John**
  **Loveland, Ohio 45140 (US)**
- **GOTTLIEB, Emily, Elizabeth**
  **Cincinnati, OH 45236 (US)**

(74) Representative: **McKinney, Victoria et al**
**Patent Department,**
**Procter & Gamble Technical Centres Limited,**
**Rusham Park**
**Whitehall Lane**
**Egham, Surrey TW20 9NW (GB)**

(56) References cited:
**WO-A-94/22501**
**FR-A- 899 469**
**GB-A- 2 012 165**
**GB-A- 2 218 430**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

**Description**

TECHNICAL FIELD

The present invention relates to a substantially dry, disposable, personal cleansing product useful for both cleansing and conditioning the skin/hair and providing improved fragrance delivery. These articles are used by the consumer by wetting the dry article with water. The article comprises a water insoluble substrate, a lathering surfactant, and a fragrance-releasing complex. Preferably, the articles of the present invention further comprise a conditioning component.

BACKGROUND OF THE INVENTION

The present invention relates to personal cleansing and conditioning articles for the skin or hair having improved fragrance delivery. Fragrances, e.g., perfumes, are a desirable part of personal cleansing articles for two main reasons: they cover up the unpleasant raw material malodors; and they provide an olfactory aesthetic benefit. Fragrances are also used in personal cleansing articles to serve as a signal that the product is effective (e.g., that the skin is clean and fresh).

Personal cleansing articles have traditionally been marketed in a variety of forms such as bar soaps, creams, lotions, and gels GB-A-2 218 430 discloses a cleaning article comprising a substrate embedded into which is a gel having surfactant properties. These cleansing formulations have attempted to satisfy a number of criteria to be acceptable to consumers. These criteria include cleansing effectiveness, skin feel, mildness to skin, hair, and ocular mucosae, and lather volume. Ideal personal cleansers should gently cleanse the skin or hair, cause little or no irritation, and not leave the skin or hair overly dry after frequent use. However, these traditional forms of personal cleansing articles have the inherent problem of balancing cleansing efficacy against delivering a conditioning benefit. Although one solution to this problem is to use separate cleansing and conditioning articles, this is not always convenient or practical and many consumers would prefer to use a single article which can both cleanse and condition the skin or hair.

In a typical cleansing composition the conditioning ingredients are difficult to formulate because many conditioners are incompatible with the surfactants, resulting in an undesirable non-homogenous mixture. To obtain a homogeneous mixture with conditioning ingredients, and to prevent the loss of conditioning ingredients before deposition, additional ingredients, e.g. emulsifiers, thickeners, and gellants are often added to suspend the conditioning ingredients within the surfactant mixture. This results in an aesthetically pleasing homogenous mixture, but often results in poor deposition of conditioning ingredients, because the conditioners are emulsified and not efficiently released during cleansing. Also, many conditioning agents have the disadvantage of suppressing lather generation. Lather suppression is a problem because many consumers seek cleansing articles that provide a rich, creamy, and generous lather.

Therefore, it is seen that conventional cleansing articles which attempt to combine surfactants and conditioning ingredients suffer from disadvantages inherently resulting from the incompatibilities of surfactants and conditioners. One effective solution is an article that provides effective cleansing and consistent conditioning in a convenient, inexpensive, and sanitary disposable personal cleansing article having the desirable properties of a washcloth. These articles provide the convenience of not needing to use both a separate cleansing and conditioning article. These articles are also highly convenient to use because it is in the form of a substantially dry article that is wetted before use, and, therefore, obviating the need to carry cumbersome bottles, bars, jars, tubes, and other forms of both cleansing and conditioning articles. Disposable articles are also a more sanitary alternative to the use of a sponge, washcloth, or other cleansing implement intended for multiple reuse, because such implements develop bacterial growth, unpleasant odors, and other undesirable characteristics related to repeated use.

Fragrances such as perfumes can be added directly to the above described disposable articles. There are, however, several disadvantages when perfumes are added as a neat oil onto or impregnated into substantially dry articles. One problem is that some perfume ingredients are not stable and, thus, are subject to damage and/or loss. They can also undergo an oxidative or other chemical reaction (e.g., by oxygen, light, heat etc.) and cause undesired discoloration of the articles containing them.

A further disadvantage arising from the direct addition of perfumes to substantially dry cleansing articles is that perfume components are, in general, volatile and, therefore, easily lost from the product during processing or storage. The loss of the highly volatile fraction of the perfume is especially high. As a result, in the past, personal cleansing products tended to employ perfumes composed mainly of less volatile, high boiling (having high boiling points), perfume components to maximize survival of the fragrance during processing and storage of the product and thus provide better in-use and after-use fragrance benefits. This was not the most desirable situation, however, because some of the volatile, low boiling perfume ingredients can provide a fresh and clean impression, and it is highly desirable that these ingredients be delivered and present in the personal cleansing product.

Another problem arising from the direct addition of perfume to substantially dry, disposable articles is that

there is no flexibility to simultaneously optimize fragrance display in the neat product (e.g., the disposable article) and during use of the product. For example, the optimum fragrance level during use may result in the neat product smelling too strong. Likewise, the optimum fragrance level in the disposable article may lead to less satisfactory results during use. It would be desirable to be able to adjust the fragrance display independently in the neat product and in use.

Perfumes are ingredients that have commonly been added to personal cleansing products to impart aesthetically attractive aromas, as mentioned hereinbefore. Perfumes can be designed and selected to make a variety of impressions on the user. Unfortunately, any particular perfume will typically convey only a single or continuous overall message, and would not clearly communicate the multiple functions of a multiple function cleansing product or be enhanced during the products usage to reinforce the performance of the product. Therefore, it is highly desirable that dual fragrance characters be delivered in the personal cleansing products to convey the distinctiveness of the product or multiple, distinct functions of the product.

The present inventors have surprisingly found that use of a perfume-releasing complex provides to the dry, disposable cleansing and conditioning article (i) improved fragrance stability, (ii) enhanced in-use fragrance bloom of the disposable cleansing product, and (iii) the ability of dual fragrance delivery. The perfume component can comprise a perfume complexed with a variety of organic and inorganic materials.

It is, therefore, an object of the present invention to provide such substantially dry, disposable, personal cleansing articles.

It is another an object of the present invention to provide personal cleansing and conditioning articles which consistently delivery fragrance wherein the articles are used in combination with water.

These and other objects of this invention will become apparent in light of the following disclosure.

## SUMMARY OF THE INVENTION

The present invention relates to a disposable, single use personal care cleansing and conditioning product which is rinsed from the skin or hair and which has desirable fragrance delivery properties, comprising: (A) a water insoluble substrate, (B) at least one lathering surfactant added onto or impregnated into the substrate, and (C) from 0.015% to 15%, by weight of said water insoluble substrate, of a fragrance-releasing complex added onto or impregnated into said substrate, wherein said product is substantially dry prior to use. The fragrance-releasing complex comprises (i) from 10% to 90%, by weight of the complex, of a porous fragrance carrier, and (ii) from 1% to 90%, by weight of the complex, of a fragrance impregnated within said carrier.

The present invention also relates to a method of manufacturing a disposable, single use personal care cleansing and conditioning product which is rinsed from the skin or hair and which has desirable fragrance delivery properties, comprising the steps of separately or simultaneously adding onto or impregnating into a water insoluble substrate (A) at least one lathering surfactant added onto or impregnated into said substrate, and (B) from 0.015% to 15%, by weight of said water insoluble substrate, of a fragrance-releasing complex added onto or impregnated into said substrate, wherein said product is substantially dry prior to use. The fragrance-releasing complex comprises (i) from 10% to 90%, by weight of the complex, of a porous fragrance carrier, and (ii) from 1% to 90%, by weight of the complex, of a fragrance impregnated within said carrier.

In further embodiments the fragrance-releasing complex is encapsulated with a coating material selected from the group consisting of paraffin waxes, microcrystalline waxes, animal waxes, vegetable waxes, saturated fatty acids and fatty alcohols, fatty esters, cellulose esters, polyalkylene glycol, polyvinyl alcohol, and mixtures thereof, and wherein the coating material is present in an amount ranging from 2% to 50% of the fragrance-releasing complex.

In further still embodiments, the present invention further comprises a conditioning component added onto or impregnated into the substrate. The conditioning component comprises materials selected from the group consisting of water soluble conditioning agents, oil soluble conditioning agents, conditioning emulsions, lipid hardening materials, and mixtures thereof. The conditioning component preferably has a lipid hardness value of greater than 0.02 kg. The conditioning component also preferably has a surface to saturation ratio of greater than 1.25 at any point on the surface of the substrate.

All percentages and ratios used herein, unless otherwise indicated, are by weight and all measurements made are at 25°C, unless otherwise designated. The invention hereof can comprise, consist of, or consist essentially of, the essential as well as optional ingredients and components described therein.

## BRIEF DESCRIPTION OF THE DRAWINGS

Figure 1 is a plane view illustration of a cleansing and conditioning article of the present invention, the article including an apertured paper layer and a nonwoven layer, with the paper layer shown facing the viewer, and with

a portion of the apertured paper layer shown cut away to show generally parallel, spaced apart zones of adhesive extending generally parallel to the machine directions of the paper layer and the nonwoven.

Figure 2 is an illustration of a portion of the cleansing and conditioning article shown in Figure 1, Figure 2 being enlarged relative to Figure 1 to illustrate the creping ridges in the paper layer.

Figure 3A is a cross-sectional illustration of the cleansing and conditioning article of the present invention taken along the direction indicated by line 3-3 in Figure 1, and showing the article prior to wetting of the first layer.

Figure 3B is a cross-sectional illustration taken along the direction indicated by line 3-3 in Figure 1, and showing the article after wetting of the first layer.

Figure 4 is an illustration of a paper machine which can be used to make paper webs that can be used in forming the substrate portion of the cleansing and conditioning articles herein.

Figure 5 is an illustration of a forming element which can be used to form a paper web with apertures.

## DETAILED DESCRIPTION OF THE INVENTION

The present invention delivers fragrances in an efficient and cost-effective manner for a substantially dry, disposable, cleansing and conditioning article. In particular, the personal cleansing articles of the present invention can convey dual fragrance characteristics and can exhibit desirable fragrance characteristics in their neat (e.g., dry) form as well as during use. Thus, the personal cleansing articles of the present invention provide (i) improved fragrance stability, (ii) enhanced in-use fragrance bloom of the disposable cleansing product, and (iii) the ability of dual fragrance delivery while being highly efficacious for cleansing the skin or hair. The articles can also contain conditioning active ingredients and other non-conditioning active ingredients to be deposited onto the skin or hair.

By a "lathering surfactant" is meant a surfactant, which when combined with water and mechanically agitated generates a foam or lather. Preferably, these surfactants should be mild, which means that these surfactants provide sufficient cleansing or detersive benefits but do not overly dry the skin or hair (e.g., removing too much natural oil and/or moisture), and yet meet the lathering criteria described above.

The terms "disposable" or "single use", are used herein in their ordinary sense to mean a article that is disposed or discarded after one usage event.

The term "conditioning component," as used herein, means a combination of the conditioning agents. This combination can also include lipid hardening materials.

The term "water-activated," as used herein, means that the present invention is presented to the consumer in dry form to be used after it is wetted with water. It is found that these articles produce a lather or are "activated" by contacting them with water and then further subjecting the article to mechanical forces, such as rubbing.

The term "substantially dry," as used herein, means that prior to use the article is substantially free of water and generally feels dry to the touch. Thus, the articles of the present invention will generally comprise less than about 10% by weight of water, preferably less than about 5% by weight of water, and more preferably less than about 1% by weight of water, the forgoing measured in a dry environment, e.g., low humidity. One of ordinary skill in the art would recognize that the water content of a article such as in the present invention can vary with the relative humidity of the environment.

The term "surface to saturation ratio" is a measurement of the proportion of the conditioning agent and active ingredient that is on the surface of the substrate versus inside the substrate. One of ordinary skill in the art of analytical chemistry would be well versed in measurements obtained from Attenuated Total Reflectance (ATR) FT-IR Spectroscopy. What is believed to be a complete disclosure is provided in the section titled "Method to Measure Surface Application of Active Ingredients and Conditioning Agents."

The term "mild" as used herein in reference to the lathering surfactants and articles of the present invention means that the articles of the present invention demonstrate skin mildness comparable to a mild alkyl glyceryl ether sulfonate (AGS) surfactant based synthetic bar, i.e. synbar. Methods for measuring mildness, or inversely the irritancy, of surfactant containing articles, are based on a skin barrier destruction test. In this test, the milder the surfactant, the lesser the skin barrier is destroyed. Skin barrier destruction is measured by the relative amount of radiolabeled (tritium labeled) water ($3H-H_2O$) which passes from the test solution through the skin epidermis into the physiological buffer contained in the diffusate chamber. This test is described by T.J. Franz in the J. Invest. Dermatol., 1975, 64, pp. 190-195; and in U.S. Patent No. 4,673,525, to Small et al., issued June 16, 1987. Other testing methodologies for determining surfactant mildness well known to one skilled in the art can also be used.

The term "deposition consistency," as used herein, means that deposition of the conditioning agents comprising the conditioning component will be relatively unvarying no matter how the consumer prepares to use, and the actual uses, the cleansing and conditioning article (e.g., lathering the side of the substrate carrying the conditioning component versus lathering the substrate side with the surfactant). The articles of the present invention will have a deposition consistency of greater than 60%, preferably greater than 65%, more preferably greater than 70%, and most preferably greater than 75%. The deposition consistency measurement is the quotient obtained by dividing the amount

of deposition of conditioning agents that occurs via "non-ideal lathering and use" by the amount of deposition of conditioning agents that occurs via "ideal lathering and use." Non-ideal lathering, as used herein, means that lathering is achieved by rubbing together or against itself the surface of the article containing the conditioning agents and then contacting the skin or hair with the same surface. This causes inefficient deposition of the conditioning agents because some of the conditioning agents become emulsified by the surfactant. Ideal lathering, as used herein, means that lathering is achieved by rubbing together or against itself the surface of the article containing surfactant, but not containing conditioning agents, and then contacting the skin or hair with the surface containing the conditioning component. The same reference points would apply if both surfaces of the substrate are treated with the conditioning agents (e.g. deposition obtained from lathering and contacting the skin with the same lathered surface containing emulsified conditioning agents versus contacting the skin with the non-lathered surface which contains non-emulsified conditioning agents). Deposition consistency is maximized when the lipid hardness value of the conditioning component is greater than 0.02 kg.

The personal care articles of the present invention comprise the following essential components: (A) a water insoluble substrate, (B) at least one lathering surfactant added onto or impregnated into the substrate, and (C) a fragrance-releasing complex added onto or impregnated into said substrate. The articles of the present invention can further comprise a conditioning component added onto or impregnated into the substrate.

I. WATER INSOLUBLE SUBSTRATE

The products of the present invention comprise a water insoluble substrate. By "water insoluble" is meant that the substrate does not dissolve in or readily break apart upon immersion in water. The water insoluble substrate is the implement or vehicle for delivering the lathering surfactant and the conditioning component of the present invention to the skin or hair to be cleansed and conditioned. Without being limited by theory, it is believed that the substrate, by providing mechanical forces and agitation provides a lather generating effect and also aids in the deposition of the conditioning component.

A wide variety of materials can be used as the substrate. The following nonlimiting characteristics are desirable: (i) sufficient wet strength for use, (ii) sufficient abrasivity, (iii) sufficient loft and porosity, (iv) sufficient thickness, and (v) appropriate size.

Nonlimiting examples of suitable insoluble substrates which meet the above criteria include nonwoven substrates, woven substrates, hydroentangled substrates, air entangled substrates, natural sponges, synthetic sponges, polymeric netted meshes, and the like. Preferred embodiments employ nonwoven substrates since they are economical and readily available in a variety of materials. By nonwoven is meant that the layer is comprised of fibers which are not woven into a fabric but rather are formed into a sheet, mat, or pad layer. The fibers can either be random (i.e., randomly aligned) or they can be carded (i.e. combed to be oriented in primarily one direction). Furthermore, the nonwoven substrate can be composed of a combination of layers of random and carded fibers.

Nonwoven substrates may be comprised of a variety of materials both natural and synthetic. By natural is meant that the materials are derived from plants, animals, insects or byproducts of plants, animals, and insects. By synthetic is meant that the materials are obtained primarily from various man-made materials or from natural materials which have been further altered. The conventional base starting material is usually a fibrous web comprising any of the common synthetic or natural textile-length fibers, or mixtures thereof.

Nonlimiting examples of natural materials useful in the present invention are silk fibers, keratin fibers and cellulosic fibers. Nonlimiting examples of keratin fibers include those selected from the group consisting of wool fibers, camel hair fibers, and the like. Nonlimiting examples of cellulosic fibers include those selected from the group consisting of wood pulp fibers, cotton fibers, hemp fibers, jute fibers, flax fibers, and mixtures thereof.

Nonlimiting examples of synthetic materials useful in the present invention include those selected from the group consisting of acetate fibers, acrylic fibers, cellulose ester fibers, modacrylic fibers, polyamide fibers, polyester fibers, polyolefin fibers, polyvinyl alcohol fibers, rayon fibers, polyurethane foam, and mixtures thereof. Examples of some of these synthetic materials include acrylics such as acrilan, creslan, and the acrylonitrile-based fiber, orlon; cellulose ester fibers such as cellulose acetate, arnel, and acele; polyamides such as nylons (e.g., nylon 6, nylon 66, nylon 610, and the like); polyesters such as fortrel, kodel, and the polyethylene terephthalate fiber, dacron; polyolefins such as polypropylene, polyethylene; polyvinyl acetate fibers; polyurethane foams and mixtures thereof. These and other suitable fibers and the nonwoven materials prepared therefrom are generally described in Riedel, "Nonwoven Bonding Methods and Materials," Nonwoven World (1987); The Encyclopedia Americana, vol. 11, pp. 147-153, and vol. 26, pp. 566-581 (1984); U.S. Patent No. 4,891,227, to Thaman et al., issued January 2, 1990; and U.S. Patent No. 4,891,228.

Nonwoven substrates made from natural materials consist of webs or sheets most commonly formed on a fine wire screen from a liquid suspension of the fibers. See C.A. Hampel et al., The Encyclopedia of Chemistry, third edition, 1973, pp. 793-795 (1973); The Encyclopedia Americana, vol. 21, pp. 376-383 (1984); and G.A. Smook, Handbook of Pulp and Paper Technologies, Technical Association for the Pulp and Paper Industry (1986).

Substrates made from natural materials useful in the present invention can be obtained from a wide variety of commercial sources. Nonlimiting examples of suitable commercially available paper layers useful herein include Airtex$^R$, an embossed airlaid cellulosic layer having a base weight of about 71 gsy, available from James River, Green Bay, WI; and Walkisoft$^R$, an embossed airlaid cellulosic having a base weight of about 89 g/m$^2$ (75 gsy), available from Walkisoft U.S.A., Mount Holly, NC.

Methods of making nonwoven substrates are well known in the art. Generally, these nonwoven substrates can be made by air-laying, water-laying, meltblowing, coforming, spinbonding, or carding processes in which the fibers or filaments are first cut to desired lengths from long strands, passed into a water or air stream, and then deposited onto a screen through which the fiber-laden air or water is passed. The resulting layer, regardless of its method of production or composition, is then subjected to at least one of several types of bonding operations to anchor the individual fibers together to form a self-sustaining web. In the present invention the nonwoven layer can be prepared by a variety of processes including hydroentanglement, thermally bonding or thermo-bonding, and combinations of these processes. Moreover, the substrates of the present invention can consist of a single layer or multiple layers. In addition, a multilayered substrate can include films and other nonfibrous materials.

Nonwoven substrates made from synthetic materials useful in the present invention can also be obtained from a wide variety of commercial sources. Nonlimiting examples of suitable nonwoven layer materials useful herein include HEF 40-047, an apertured hydroentangled material containing about 50% rayon and 50% polyester, and having a basis weight of about 51 g/m$^2$ (43 grams per square yard (gsy) available from Veratec, Inc., Walpole, MA; HEF 140-102, an apertured hydroentangled material containing about 50% rayon and 50% polyester, and having a basis weight of about 56 gsy, available from Veratec, Inc., Walpole, MA; Novonet$^R$ 149-616, a thermo-bonded grid patterned material containing about 100% polypropylene, and having a basis weight of about 60 g/m$^2$ (50 gsy), available from Veratec, Inc., Walpole, MA; Novonet$^R$ 149-801, a thermo-bonded grid patterned material containing about 69% rayon, about 25% polypropylene, and about 6% cotton, and having a basis weight of about 89 g/m$^2$ (75 gsy), available from Veratec, Inc. Walpole, MA; Novonet$^R$ 149-191, a thermo-bonded grid patterned material containing about 69% rayon, about 25% polypropylene, and about 6% cotton, and having a basis weight of about 119 g/m$^2$ (100 gsy), available from Veratec, Inc. Walpole, MA; HEF Nubtex$^R$ 149-801, a nubbed, apertured hydroentangled material, containing about 100% polyester, and having a basis weight of about 83 g/m$^2$ (70 gsy), available from Veratec, Inc. Walpole, MA; Keybak$^R$ 951V, a dry formed apertured material, containing about 75% rayon, about 25% acrylic fibers, and having a basis weight of about 51 g/m$^2$ (43 gsy), available from Chicopee, New Brunswick, NJ; Keybak$^R$ 1368, an apertured material, containing about 75% rayon, about 25% polyester, and having a basis weight of about 46 g/m$^2$ (39 gsy), available from Chicopee, New Brunswick, NJ; Duralace$^R$ 1236, an apertured, hydroentangled material, containing about 100% rayon, and having a basis weight from about 48 - 137 g/m$^2$ (40 gsy to about 115 gsy), available from Chicopee, New Brunswick, NJ; Duralace$^R$ 5904, an apertured, hydroentangled material, containing about 100% polyester, and having a basis weight from about 48 - 137 g/m$^2$ (40 gsy to about 115 gsy), available from Chicopee, New Brunswick, NJ; Sontara 8868, a hydroentangled material, containing about 50% cellulose and about 50% polyester, and having a basis weight of about 71 g/m$^2$ (60 gsy), available from Dupont Chemical Corp.

Alternatively, the water insoluble substrate can be a polymeric mesh sponge as described in European Patent No. EP 702550 A1 published March 27, 1996. The polymeric sponge comprises a plurality of plies of an extruded tubular netting mesh prepared from a strong flexible polymer, such as addition polymers of olefin monomers and polyamides of polycarboxylic acids. Although these polymeric sponges are designed to be used in conjunction with a liquid cleanser, these types of sponges can be used as the water insoluble substrate in the present invention.

The substrate can be made into a wide variety of shapes and forms including flat pads, thick pads, thin sheets, ball-shaped implements, irregularly shaped implements, and having sizes ranging from a surface area of about a square inch to about hundreds of square inches. The exact size will depend upon the desired use and product characteristics. Especially convenient are square, circular, rectangular, or oval pads having a surface area of from about 6.5 - 929.0 cm$^2$ (1 in$^2$ to about 144 in$^2$), preferably from about 64.5 - 774.2 cm$^2$ (10 in$^2$ to about 120 in$^2$), and more preferably from about 193.5 - 516.1 cm$^2$ (30 in$^2$ to about 80 in$^2$), and a thickness of from about 0.0025 - 1.27 cm (1 mil to about 500 mil), preferably from about 0.0127 - 0.635 cm (5 mil to about 250 mil), and more preferably from about 0.0254 - 0.254 cm (10 mil to about 100 mil).

The water insoluble substrates of the present invention can comprise two or more layers, each having different textures and abrasiveness. The differing textures can result from the use of different combinations of materials or from the use of different manufacturing processes or a combination thereof. A dual textured substrate can be made to provide the advantage of having a more abrasive side for exfoliation and a softer, absorbent side for gentle cleansing. In addition, separate layers of the substrate can be manufactured to have different colors, thereby helping the user to further distinguish the surfaces.

A. Water Insoluble Substrate Having at Least a Portion That is Wet Extensible

The articles of the present invention can preferably comprise a water insoluble substrate wherein at least a first portion of said substrate is wet extensible and at least a second portion of said substrate is less wet extensible than said first portion. By wet extensible is meant that the material has a tendency to elongate at least in one direction when wetted. A test for measuring extensibility is provided below.

It is highly desirable to provide a cleansing and conditioning article having the qualities of a washcloth. These desirable features can be realized in disposable articles by providing proper texture, thickness (caliper), and bulk (volume per unit weight). A relatively high value of texture is desirable for aiding in cleansing of skin and hair. Relatively high values of caliper and bulk are desirable for providing volume in the article for receiving and containing liquids. Typically, such washcloth-like articles have a substrate which includes one or more materials or layers. It has been surprisingly found that the present substrates, wherein at least a first portion of said substrate is wet extensible and at least a second portion of said substrate is less wet extensible than said first portion, provide these desirable washcloth-like qualities.

One embodiment of a water insoluble substrate having at least a portion that is wet extensible is illustrated in Figures 1-2. In this embodiment, the present invention comprises a multiple layer disposable wiping article 20. Figures 1 and 2 illustrate a two layer, or two ply, embodiment of the present invention. Alternatively, the disposable wiping article can include more than two layers.

The disposable cleansing and conditioning article 20 comprises a substrate designated generally by reference numeral 22. The substrate 22 comprises a first layer 100 and a second layer 200. The first layer 100 is apertured, the first layer 100 comprising a plurality of apertures 102 which extend through the thickness of the first layer 100. In Figure 1, apertures 102 are shown on only a portion of the first layer 100 for clarity.

The first layer 100 is wet extensible when the first layer is wetted. Extensibility is measured according to the "Wet Extensibility Test" described below, and is reported as a percentage. The second layer 200 is relatively less wet extensible when wetted than the first layer 100.

Selected portions of the first layer 100 are joined, directly or indirectly, to second layer 200 to inhibit wet extension of the first layer in the plane of the first layer. In Figures 1 and 2, selected portions of the first layer 100 are joined to the second layer 200 to provide bonded regions designated 110 and unbonded regions 114.

In the embodiment shown in Figure 1, the bonded regions 110 are generally parallel, spaced apart regions which extend along substantially the full length of the article 20, and define generally parallel, spaced apart unbonded regions 114 of the first layer 100. In Figure 1, the unbonded regions 114 extend along substantially the full length of the article 20. An adhesive, designated by reference numeral 300 in Figures 1 and 2, can be used to join the first layer 100 to the second layer 200.

When the first layer is wetted, there is a tendency for the first layer 100 to expand along one or more directions in the plane of the first layer. (The plane of the first layer is parallel to the plane of Figure 1). However, because of the relatively lower wet extensibility of the second layer 200, the second layer constrains extension of the first layer 100 in the plane of the first layer. As a result, the unbonded regions 114 of the first layer 100 deform, such as by buckling or puckering in the Z-direction, perpendicular to the plane of the first layer 100.

Figure 3A is a cross-sectional illustration of the cleansing and conditioning article 20 prior to wetting of the first layer 100. As shown in Figure 3A, the wiping article is generally flat prior to wetting. Figure 3B is cross-sectional illustration similar to that of Figure 3A, but showing the article 20 after wetting of the first layer 100. Figure 3B shows out of plane deformation of the first layer 100 upon wetting of the first layer 100. The Z-direction is indicated in Figures 3A and 3B. The deformation of the wetted first layer 100 provides the article 100 with elevated ridges 120 which increase the wet texture, wet caliper (thickness) and wet bulk of the article 20. In particular, the article 20 has a wet caliper to dry caliper ratio which is greater than 1.0, and preferably at least about 1.1. The wet caliper to dry caliper ratio is a measure of the thickness of the article 20, when wetted, relative to the thickness of the dry article 20 prior to wetting. The wet caliper to dry caliper ratio is measured according to the procedure "Wet Caliper to Dry Caliper Ratio" provided below.

The elevated ridges 120 also provide pockets 150 disposed between the unbonded portions of the first layer 100 and the underlying portions of the second layer 200. The apertures 102 provide a flow path through which liquids and/or small particles can enter the pockets 150.

Additionally, since the article 20 is used with, or includes a lathering agent, such as a surfactant, the apertures 102 can aid in the incorporation of air during the lathering process, thereby improving lather generation. For instance, a portion of the article 20 can be coated with or otherwise treated with a surfactant composition, as described more fully below. The article 20 can be wetted with water to activate the surfactant, and the airflow generated through the apertures 102 during use of the article (e.g. washing or wiping) can help to generate lather.

The size and number of the apertures 102 can influence the speed of lather generation and the quality of lather produced. A relatively small number of relatively large apertures 102 will tend to reduce the time required to

generate lather, but will yield relatively large lather bubbles with a translucent appearance. On the other hand, a relatively larger number of relatively smaller apertures 102 will tend to reduce bubble size, thereby increasing lather creaminess and opacity, but at the expense of increasing the time required to generate lather. Between 4 and 100 apertures per inch can provide suitable lather speed and quality.

First Layer: Referring to the components of the article 20 in more detail, suitable materials from which the first layer 100 can be formed include foreshortened (such as by creping) wetlaid paper webs. Other suitable materials can include woven materials, nonwoven materials, foams, battings, and the like.

The first layer 100 should be constructed to have a wet extensibility of at least 4 percent, more preferably at least 10 percent, and still more preferably at least about 20 percent. In one embodiment, the first layer has a wet extensibility of at least about 25 percent. Preferably, the difference between the wet extensibility of the first layer and the wet extensibility of the second layer (the wet extensibility of the second layer subtracted from the wet extensibility of the first layer) is at least about 4 percent, more preferably at least about 10 percent, and still more preferably at least about 20 percent.

The fibers or filaments of the first layer 100 can be natural (e.g. cellulosic fibers such as wood pulp fibers, cotton linters, rayon, and bagasse fibers) or synthetic (e.g. polyolefins, polyamides or polyesters), or combinations thereof.

In one preferred embodiment, the first layer 100 comprises a wetlaid paper web of cellulosic wood pulp fibers which is foreshortened at least about 4 percent, more preferably at least about 10 percent, and still more preferably at least about 20 percent, by dry creping. Referring to Figure 2, the first layer 100 is shown comprising crepe ridges 105 corresponding to the foreshortening of the first layer 100. The machine direction (MD) and cross machine direction (CD) are indicated in Figures 1 and 2. The machine direction corresponds to the direction of manufacture of the paper web of first layer 100. The crepe ridges 105 are generally perpendicular to the machine direction, and generally parallel to the cross machine direction of the paper web of first layer 100.

The paper web of the first layer 100 can have a basis weight of between about 25 to about 45 grams per square meter. In one embodiment, the basis weight of the first layer 100 is about 33 grams per square meter.

The apertures 102 can be formed in the first layer 100 in an suitable manner. For instance, the apertures 102 can be formed in the first layer 100 during formation of the paper web of the first layer 100, or alternatively, after the paper web of the first layer 100 is manufactured. In one embodiment, the paper web of the first layer 100 is produced according to the teachings of one or more of the following U.S. Patents: U.S. 5,245,025 issued Sept. 14, 1993 to Trokhan et al.; U.S. 5,277,761 issued January 11, 1994 to Phan et al.; and U.S. 5,654,076 issued August 5, 1997 to Trokhan et al. In particular, U.S. 5,277,761 at Column 10 discloses formation of a paper web having a apertures.

Prior to wetting of the first layer, the creped first layer 100 can have between 4 and 300 apertures 102 per square inch, and more preferably between 4 and 100 apertures 102 per square inch. Wetting a creped paper web causes the web, if unrestrained, to expand in at least one direction, such as the machine direction, so that the number of apertures 102 per square inch after wetting can be smaller than the number of apertures per square inch prior to wetting. Similarly, when apertures are formed in a paper web, and the paper web is subsequently creped, the number of apertures per square inch prior to creping will be smaller than the number of apertures per square inch after creping. Accordingly references to paper web dimensions refer to dimensions after creping and prior to wetting.

The apertures 102 can comprise between about 15 and about 75 percent of the total surface of the first layer 100. The apertures 102 shown in Figure 2 are bilaterally staggered (staggered in both the machine and cross machine directions) in a repeating, nonrandom pattern. In one embodiment, the first layer 100 comprises a paper web which is dry creped 25 percent (25 percent foreshortening) with greater than about 25 percent wet extensibility, and has about 40 to about 50 apertures, 102, per 6.45 $cm^2$ (square inch), the apertures 102 having a length 103 (Figure 2) of about 0.25 - 0.46 cm (0.10 to about 0.18 inch) and a width 104 of about 0.18 - 0.38 cm (0.07 to about 0.15 inch).

The paper web is manufactured by first forming an aqueous papermaking furnish. The furnish comprises papermaking fibers, and can further comprise various additives. U.S. Patent 5,223, 096 issued June 29, 1993 to Phan et al. is mentioned for the purpose of disclosing various wood pulps and papermaking additives.

A suitable paper web for making the first layer 100 can be manufactured according to the following description. A papermaking furnish is prepared from water and highly refined Kraft pulp derived from northern softwoods (NSK), the paper furnish having a fiber consistency of about 0.2 percent (dry fiber weight divided by the total weight of the furnish equals 0.002). A dry strength additive such as carboxymethyl cellulose (CMC) is added to the 100 % NSK furnish in the amount of about 6 pounds of CMC solids per ton of dry papermaking fibers. A wet strength additive such as Kymene 557H (available from Hercules, Inc. of Wilmington, Del.) is added to the furnish in the amount of about 24 pounds of Kymene solids per ton of dry papermaking fibers.

Referring to Figure 4, the furnish is deposited from a headbox 500 of a papermaking machine to a forming element 600 at a fiber consistency of about 0.2 percent. The forming element 600 is in the form of a continuous belt in Figure 4. The slurry of papermaking fibers is deposited on the forming element 600, and water is drained from the slurry through the forming element 600 to form an embryonic web of papermaking fibers designated by reference

numeral 543 in Figure 4.

Figure 5 shows a portion of the forming element 600. The forming element 600 has two mutually opposed faces. The face which is shown in Figure 5 is the face which contacts the papermaking fibers of the web being formed. A description of a forming element of the type shown in Figure 5 is provided in the above referenced U.S. Patents 5,245,025; 5,277,761; and 5,654,076.

The forming element 600 has flow restriction members in the form of resin protuberances 659. The forming element 600 shown comprises a patterned array of protuberances 659 joined to a reinforcing structure 657, which may comprise a foraminous element, such as a woven screen or other apertured framework. The protuberances 659 extend above the reinforcing structure 657.

A suitable forming element 600 has about 37 protuberances 659 per square inch of surface of the forming element 600, with the protuberances 659 covering about 35 percent of the surface of the forming element 600, as viewed in Figure 5, and the protuberances extending 0.0638 cm (0.0255 inches) above the surface of the reinforcing structure 657. The protuberances can have a machine direction length X of about 0.46 cm (0.18 inch) and a cross machine direction width Y of about 0.36 cm (0.14 inch).

The reinforcing structure 657 is substantially fluid pervious, while the protuberances 659 are substantially fluid impervious. Accordingly, as the liquid in the papermaking furnish drains through the forming element, the papermaking fibers in the furnish will be retained on the reinforcing structure 657, leaving apertures in the embryonic web 543 corresponding generally in size, shape and location to the size, shape and location of the protuberances 659.

Referring back to Figure 4, the embryonic web 543 is transferred to a conventional dewatering felt 550 with the aid of a vacuum pick up shoe 560. The web 543 is transferred to the felt 550 at a fiber consistency of about 4 percent. The web 543 is carried on the felt 550 to a nip 570 formed between a vacuum pressure roll 572 and a Yankee dryer drum 575. The web 543 is dried on the Yankee drum 575 to a fiber consistency of about 96 percent, at which point the web is creped from the Yankee drum 575 with a doctor blade 577 having a bevel angle of about 25 degrees and an impact angle of about 81 degrees. The web is wound on a reel at a rate (lineal feet per second) which is 25 percent slower than the surface speed of the Yankee drum (reel speed equals 0.75 times the Yankee speed) to foreshorten the web about 25 percent. The foreshortened web can have a basis weight of about 33 grams per square meter, and a thickness of about 0.030 - 0.033 cm [12 to 13 mils (0.012 to 0.013 inch)] as measured with a confining pressure of 613 g/cm$^2$ (95 grams per square inch) and a load foot having a diamer of 5.08 cm (2 inches). The resulting foreshortened web can be used to form a first layer 100 having a wet extensibility of at least about 25 percent.

Second Layer: Referring back to Figures 1, 3A, and 3B, the first layer 100 is joined to the second layer 200 to constrain extension of selected portions of the first layer 100 when the first layer is wetted. The second layer 200 has a lower wet extensibility than that of the first layer 100.

Suitable materials from which the second layer 200 can be formed include woven materials, nonwoven materials, foams, battings, and the like. Particularly preferred materials are nonwoven webs having fibers or filaments distributed randomly as in "air-laying" or certain "wet-laying" processes, or with a degree of orientation, as in certain "wet-laying" and "carding" processes.

One material from which the second layer 200 can be formed is a nonwoven web formed by hydroentanglement of fibers. A suitable hydroentangled web is a nonwoven, hydroentangled web comprising about 50 percent by weight rayon fibers and 50 percent by weight polyester fibers, and having a basis weight of 62 grams per square meter. A suitable hydroentangled nonwoven web is commercially available from PGI Nonwovens of Benson, N.C. under the designation Chicopee 9931.

Bonding: Again referring to Figures 1, 3A, and 3B, selected portions of the first layer 100 are joined directly (or indirectly such as through a third component) to the second layer 200 in a predetermined bonding pattern to provide a plurality of bonded and unbonded regions of the first layer 100. In Figures 1 and 2, the bonded regions are designated 110, and the unbonded regions are designated 114. Each of the first and second layers 100 and 200 can have a machine direction, and the first and second layers can be bonded so that the machine direction of the first layer is generally parallel to the machine direction of the second layer.

The first layer 100 and the second layer 200 can be joined using any suitable method, including but not limited to adhesive bonding, mechanical bonding, thermal bonding, mechanical-thermal bonding, ultrasonic bonding, and combinations thereof.

In one embodiment, the first layer 100 and the second layer 200 can be joined with an adhesive 300 applied to selective portions of the second layer 200. The adhesive is preferably water insoluble so that the article 20 can be wetted with water without delamination of the first and second layers. The adhesive is preferably also surfactant tolerant. By "surfactant tolerant" it is meant that the bonding characteristics of the adhesive are not degraded by the presence of surfactants. Suitable adhesives include EVA (ethylene vinyl acetate) based hot melt adhesives. One suitable adhesive is a hot melt adhesive commercially available as H1382-01 from Findley Adhesives of Wauwatos, Wisconsin.

With reference to Figures 1 and 2, the hot melt adhesive can be applied to the nonwoven second layer 200 in bands which extend generally parallel to the machine direction of the nonwoven second layer 200. The hot melt

adhesive can be applied in stripes 310 having a width W (Figure 1) of about 0.32 - 2.54 cm (1/8 inch to about 1 inch). The spacing D between adjacent adhesive stripes can be about 0.32 - 5.08 cm (1/8 inch to about 2 inches). In Figure 1, five stripes 310A, 310B, 310C, 310D, and 310 E are shown. In one embodiment, the width W of stripes 310A, 310C, and 310E can be about 1.27 cm (1/2 inch), the witdth W of stripes 310B and 310D can be about 0.64 cm (1/4 inch), and the spacing D between adjacent stripes can be about 2.54 cm (1 inch).

The adhesive can be applied to the nonwoven second layer 200 using a slot coating applicator. A suitable slot coating applicator is a Nordson MX series hot melter with extrusion head commercially available from the Nordson Company of Norcross, Ga. The H1382-01 adhesive referenced above can be applied to the second layer 200 at a temperature of about 350 Fahrenheit, at an application level of about 0.03 grams of adhesive per square inch. Immediately following application of the adhesive to the nonwoven second layer 200, the nonwoven second layer 200 and the paper first layer 100 can be bonded together by pressing the two layers 100 and 200 together with the adhesive disposed between the second layer 200 and the first layer 100. One suitable means for pressing the two layers 100 and 200 together is by passing the two layers through a nip formed between two rollers, with the rollers loaded to provide adequate nip pressure for bonding.

The resulting laminate of the first and second layers can have an average dry caliper of about 0.072 cm [28.5 mils (0.0285 inch)], an average wet caliper of about 0.081 cm [32.1 mils (0.0321 inch)], and a wet caliper to dry caliper ratio of aobut 1.1. The dry caliper, wet caliper, and wet caliper to dry caliper ratio are measured as described below under "Wet Caliper to Dry Caliper Ratio."

The resulting laminate of the first and second layers has spaced apart, generally rectangular bonded regions 110 and spaced apart, generally rectangular unbonded regions 114. Alternatively, the first and second layers 100 and 200 can be bonded together to provide different patterns of bonded and unbonded regions. For instance, the adhesive could be applied in a continuous network to the second layer 100, to provide a continuous bonded region 110 and discrete unbonded regions 114 having any suitable shape, including but not limited to circles, ovals, triangles, diamonds, and the like.

Wet Extensiblity Test: The wet extensibility of a layer, such as the layer 100 or the layer 200, is determined using the following procedure. Samples are conditioned at 21°C (70 degrees Fahrenheit) and 50 percent relative humidity for two hours prior to testing.

First, the direction of greatest wet extensibility in the plane of the layer is determined. For dry creped paper webs, this direction will be parallel to the machine direction, and generally perpendicular to the crepe ridges.

If the direction of greatest wet extensibility is not known, the direction can be determined by cutting seven samples from a sheet with sample lengths oriented between 0 degrees and 90 degrees, inclusive, with respect to a reference line drawn on the sheet. The samples are then measured as set forth below to determine the direction of greatest wet extensibility.

Once the direction of the greatest wet extensibility is determined, 8 samples are cut to have a length of about 17.8 cm (7 inches) measured parallel to the direction of greatest wet extensibility, and a width of at least 2.5 cm (1 inch). The samples are cut from unbonded portions of the layers 100 and 200, or, if unbonded portions having the above dimensions cannot be cut from the article 20, then samples are cut from the layers 100 and 200 prior to bonding the layers together. Two marks are placed on each sample, such as with an ink pen. The marks are spaced apart 12.7 cm (5 inches) as measured parallel to the direction of greatest wet extensibility. This 12.7 cm (5 inch) length is the initial dry test length of the sample.

Each sample is thoroughly wetted by submerging the sample in distilled water for 30 seconds in a water bath. Each sample is removed from the water bath and immediately supported to hang vertically so that a line through the two marks is generally vertical. The wet sample is supported such that the support does not interfere with extension between the two marks (e.g. with a clip which does not contact the sample between the two marks). The wet test length of the sample is the distance between the two marks. The distanceis measured within 30 seconds of removing the sample from the water bath.

For each sample, the sample wet extension is calculated as follows:

Sample Wet Extension

= [wet test length - initial dry test length (12.7 cm (5 inches))]/(initial dry test length)x100.

For example, for a measured wet length of 16.5 cm (6.5 inches) and a dry length of 12.7 cm (5.0 inches), the wet extension is [(6.5-5)/5] x 100 = 30 percent. The wet extensibility of the samples is the average of the 8 calculated values of sample wet extension.

Wet Caliper to Dry Caliper Ratio: The wet caliper to dry caliper ratio is measured using the following procedure. Samples are conditioned at 70 degrees Fahrenheit and 50 percent relative humidity for two hours prior to testing.

The dry caliper of the article 20 is measured using a confining pressure of 613 $g/cm^2$ (95 grams per square inch) and a load foot having a diameter of 5.1 cm (2 inches). The dry caliper is measured for eight samples. For each sample, the caliper is measured with the load foot centered on an unbonded region of the first layer 100. The eight caliper measurements are averaged to provide an average dry caliper.
Each sample is then wetted by submerging the sample in a distilled water bath for 30 seconds. The sample is then removed from the water bath. The caliper of the wet sample is measured within 30 seconds of removing the sample from the bath. The wet caliper is measured in the same location in which the dry caliper was previously measured. The eight wet caliper measurements are averaged to provide an average wet caliper.
The wet caliper to dry caliper ratio is the average wet caliper divided by the average dry caliper.

## II. LATHERING SURFACTANT

The articles of the present invention comprise from about 0.5% to about 12.5%, preferably from about 0.75% to about 11%, and more preferably from about 1% to about 10%, based on the weight of the water insoluble substrate, of a lathering surfactant.
By a lathering surfactant is meant a surfactant, which when combined with water and mechanically agitated generates a foam or lather. Preferably, these surfactants or combinations of surfactants should be mild, which means that these surfactants provide sufficient cleansing or detersive benefits but do not overly dry the skin or hair, and yet meet the lathering criteria described above.
A wide variety of lathering surfactants are useful herein and include those selected from the group consisting of anionic lathering surfactants, nonionic lather surfactants, amphotheric lathering surfactants, and mixtures thereof. Cationic surfactants can also be used as optional components, provided they do not negatively impact the overall lathering characteristics of the required, lathering surfactants.

### A. Anionic Lathering Surfactants

Nonlimiting examples of anionic lathering surfactants useful in the compositions of the present invention are disclosed in McCutcheon's, Detergents and Emulsifiers, North American edition (1986), published by allured Publishing Corporation; McCutcheon's, Functional Materials, North American Edition (1992); and U.S. Patent No. 3,929,678, to Laughlin et al., issued December 30, 1975.
A wide variety of anionic lathering surfactants are useful herein. Nonlimiting examples of anionic lathering surfactants include those selected from the group consisting of sarcosinates, sulfates, lactylates, isethionates, taurates, phosphates, and mixtures thereof. Amongst the isethionates, the alkoyl isethionates are preferred, and amongst the sulfates, the alkyl and alkyl ether sulfates are preferred. The alkoyl isethionates typically have the formula $RCO\text{-}OCH_2CH_2SO_3M$ wherein R is alkyl or alkenyl of from 10 to 30 carbon atoms, and M is a water-soluble cation such as ammonium, sodium, potassium and triethanolamine. Nonlimiting examples of these isethionates include those alkoyl isethionates selected from the group consisting of ammonium cocoyl isethionate, sodium cocoyl isethionate, sodium lauroyl isethionate, and mixtures thereof.
The alkyl and alkyl ether sulfates typically have the respective formulae $ROSO_3M$ and $RO(C_2H_4O)_xSO_3M$, wherein R is alkyl or alkenyl of from about 10 to about 30 carbon atoms, x is from about 1 to about 10, and M is a water-soluble cation such as ammonium, sodium, potassium and triethanolamine. Another suitable class of anionic surfactants are the water-soluble salts of the organic, sulfuric acid reaction articles of the general formula:

$$R_1\text{--}SO_3\text{--}M$$

wherein $R_1$ is chosen from the group consisting of a straight or branched chain, saturated aliphatic hydrocarbon radical having from about 8 to about 24, preferably about 10 to about 16, carbon atoms; and M is a cation. Still other anionic synthetic surfactants include the class designated as succinamates, olefin sulfonates having about 12 to about 24 carbon atoms, and b-alkyloxy alkane sulfonates. Examples of these materials are sodium lauryl sulfate and ammonium lauryl sulfate.
Other anionic materials include the sarcosinates, nonlimiting examples of which include sodium lauroyl sarcosinate, sodium cocoyl sarcosinate, and ammonium lauroyl sarcosinate.
Other anionic materials useful herein are soaps (i.e. alkali metal salts, e.g., sodium or potassium salts) of fatty acids, typically having from about 8 to about 24 carbon atoms, preferably from 10 to 20 carbon atoms. The fatty acids used in making the soaps can be obtained from natural sources such as, for instance, plant or animal-derived glycerides (e.g., palm oil, coconut oil, soybean oil, castor oil, tallow, lard, etc.) The fatty acids can also be synthetically prepared. Soaps are described in more detail in U.S. Patent No. 4,557,853, cited above.

Other anionic materials include phosphates such as monoalkyl, dialkyl, and trialkylphosphate salts.

Other anionic materials include alkanoyl sarcosinates corresponding to the formula $RCON(CH_3)CH_2CH_2CO_2M$ wherein R is alkyl or alkenyl of about 10 to about 20 carbon atoms, and M is a water-soluble cation such as ammonium, sodium, potassium and trialkanolamine (e.g., triethanolamine), a preferred example of which is sodium lauroyl sarcosinate.

Also useful are taurates which are based on taurine, which is also known as 2-aminoethanesulfonic acid. Examples of taurates include N-alkyltaurines such as the one prepared by reacting dodecylamine with sodium isethionate according to the teaching of U.S. Patent 2,658,072 which is incorporated herein by reference in its entirety.

Also useful are lactylates. Nonlimiting examples of lactylates include sodium lauroyl lactylate, sodium cocoyl lactylate, ammonium lauroyl lactylate, caprilic lactylate, and triethanolamine ("TEA") lauroyl lactylate.

Nonlimiting examples of preferred anionic lathering surfactants useful herein include those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium lauroyl sarcosinate, and mixtures thereof.

Especially preferred for use herein is ammonium lauryl sulfate, ammonium laureth sulfate, sodium lauroyl lactylate, caprilic lactylate, and triethanolamine lauroyl lactylate.

B. Nonionic Lathering Surfactants

Nonlimiting examples of nonionic lathering surfactants for use in the compositions of the present invention are disclosed in McCutcheon's, Detergents and Emulsifiers, North American edition (1986), published by allured Publishing Corporation; and McCutcheon's, Functional Materials, North American Edition (1992).

Nonionic lathering surfactants useful herein include those selected from the group consisting of alkyl glucosides, alkyl polyglucosides, polyhydroxy fatty acid amides, alkoxylated fatty acid esters, lathering sucrose esters, amine oxides, and mixtures thereof.

Alkyl glucosides and alkyl polyglucosides are useful herein, and can be broadly defined as condensation articles of long chain alcohols, e.g. C8-30 alcohols, with sugars or starches or sugar or starch polymers, i.e., glycosides or polyglycosides. These compounds can be represented by the formula $(S)_n$-O-R wherein S is a sugar moiety such as glucose, fructose, mannose, and galactose; n is an integer of from about 1 to about 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a C8-20 alkyl group, and n is an integer of from about 1 to about 9. Commercially available examples of these surfactants include decyl polyglucoside (available as APG 325 CS from Henkel) and lauryl polyglucoside (available as APG 600CS and 625 CS from Henkel). Also useful are sucrose ester surfactants such as sucrose cocoate and sucrose laurate.

Other useful nonionic surfactants include polyhydroxy fatty acid amide surfactants, more specific examples of which include glucosamides, corresponding to the structural formula:

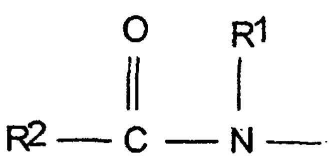

wherein: $R^1$ is H, $C_1$-$C_4$ alkyl, 2-hydroxyethyl, 2-hydroxy- propyl, preferably $C_1$-$C_4$ alkyl, more preferably methyl or ethyl, most preferably methyl; $R^2$ is $C_5$-$C_{31}$ alkyl or alkenyl, preferably $C_7$-$C_{19}$ alkyl or alkenyl, more preferably $C_9$-$C_{17}$ alkyl or alkenyl, most preferably $C_{11}$-$C_{15}$ alkyl or alkenyl; and Z is a polhydroxyhydrocarbyl moiety having a linear hydrocarbyl chain with a least 3 hydroxyls directly connected to the chain, or an alkoxylated derivative (preferably ethoxylated or propoxylated) thereof. Z preferably is a sugar moiety selected from the group consisting of glucose, fructose, maltose, lactose, galactose, mannose, xylose, and mixtures thereof. An especially preferred surfactant corresponding to the above structure is coconut alkyl N-methyl glucoside amide (i.e., wherein the $R^2CO$- moiety is derived from coconut oil fatty acids). Processes for making compositions containing polyhydroxy fatty acid amides are disclosed, for example, in G.B. Patent Specification 809,060, published February 18, 1959, by Thomas Hedley & Co., Ltd.; U.S. Patent No. 2,965,576, to E.R. Wilson, issued December 20, 1960; U.S. Patent No. 2,703,798, to A.M. Schwartz, issued March 8, 1955; and U.S. Patent No. 1,985,424, to Piggott, issued December 25, 1934.

Other examples of nonionic surfactants include amine oxides. Amine oxides correspond to the general formula $R_1R_2R_3NO$, wherein $R_1$ contains an alkyl, alkenyl or monohydroxy alkyl radical of from about 8 to about 18 carbon

atoms, from 0 to about 10 ethylene oxide moieties, and from 0 to about 1 glyceryl moiety, and $R_2$ and $R_3$ contain from about 1 to about 3 carbon atoms and from 0 to about 1 hydroxy group, e.g., methyl, ethyl, propyl, hydroxyethyl, or hydroxypropyl radicals. The arrow in the formula is a conventional representation of a semipolar bond. Examples of amine oxides suitable for use in this invention include dimethyl-dodecylamine oxide, oleyldi(2-hydroxyethyl) amine oxide, dimethyloctylamine oxide, dimethyl-decylamine oxide, dimethyl-tetradecylamine oxide, 3,6,9-trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.

Nonlimiting examples of preferred nonionic surfactants for use herein are those selected form the group consisting of C8-C14 glucose amides, C8-C14 alkyl polyglucosides, sucrose cocoate, sucrose laurate, lauramine oxide, cocoamine oxide, and mixtures thereof.

C. Amphoteric Lathering Surfactants

The term "amphoteric lathering surfactant," as used herein, is also intended to encompass zwitterionic surfactants, which are well known to formulators skilled in the art as a subset of amphoteric surfactants.

A wide variety of amphoteric lathering surfactants can be used in the compositions of the present invention. Particularly useful are those which are broadly described as derivatives of aliphatic secondary and tertiary amines, preferably wherein the nitrogen is in a cationic state, in which the aliphatic radicals can be straight or branched chain and wherein one of the radicals contains an ionizable water solubilizing group, e.g., carboxy, sulfonate, sulfate, phosphate, or phosphonate.

Nonlimiting examples of amphoteric surfactants useful in the compositions of the present invention are disclosed in McCutcheon's, Detergents and Emulsifiers, North American edition (1986), published by allured Publishing Corporation; and McCutcheon's, Functional Materials, North American Edition (1992).

Nonlimiting examples of amphoteric or zwitterionic surfactants are those selected from the group consisting of betaines, sultaines, hydroxysultaines, alkyliminoacetates, iminodialkanoates, aminoalkanoates, and mixtures thereof.

Examples of betaines include the higher alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, cetyl dimethyl betaine (available as Lonzaine 16SP from Lonza Corp.), lauryl bis-(2-hydroxyethyl) carboxymethyl betaine, oleyl dimethyl gamma-carboxypropyl betaine, lauryl bis-(2-hydroxypropyl)alpha-carboxyethyl betaine, coco dimethyl sulfopropyl betaine, lauryl dimethyl sulfoethyl betaine, lauryl bis-(2-hydroxyethyl) sulfopropyl betaine, amidobetaines and amidosulfobetaines (wherein the $RCONH(CH_2)_3$ radical is attached to the nitrogen atom of the betaine), oleyl betaine (available as amphoteric Velvetex OLB-50 from Henkel), and cocamidopropyl betaine (available as Velvetex BK-35 and BA-35 from Henkel).

Examples of sultaines and hydroxysultaines include materials such as cocamidopropyl hydroxysultaine (available as Mirataine CBS from Rhone-Poulenc).

Preferred for use herein are amphoteric surfactants having the following structure:

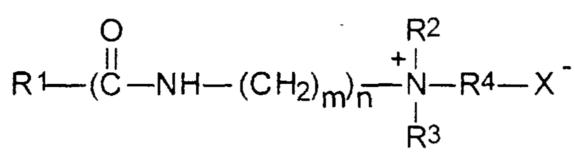

wherein $R^1$ is unsubstituted, saturated or unsaturated, straight or branched chain alkyl having from about 9 to about 22 carbon atoms. Preferred $R^1$ has from about 11 to about 18 carbon atoms; more preferably from about 12 to about 18 carbon atoms; more preferably still from about 14 to about 18 carbon atoms; m is an integer from 1 to about 3, more preferably from about 2 to about 3, and more preferably about 3; n is either 0 or 1, preferably 1; $R^2$ and $R^3$ are independently selected from the group consisting of alkyl having from 1 to about 3 carbon atoms, unsubstituted or monosubstituted with hydroxy, preferred $R^2$ and $R^3$ are $CH_3$; X is selected from the group consisting of $CO_2$, $SO_3$ and $SO_4$; $R^4$ is selected from the group consisting of saturated or unsaturated, straight or branched chain alkyl, unsubstituted or monosubstituted with hydroxy, having from 1 to about 5 carbon atoms. When X is $CO_2$, $R^4$ preferably has 1 or 3 carbon atoms, more preferably 1 carbon atom. When X is $SO_3$ or $SO_4$, $R^4$ preferably has from about 2 to about 4 carbon atoms, more preferably 3 carbon atoms.

Examples of amphoteric surfactants of the present invention include the following compounds:

Cetyl dimethyl betaine (this material also has the CTFA designation cetyl betaine)

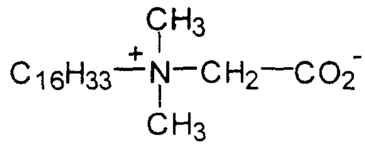

Cocamidopropylbetaine

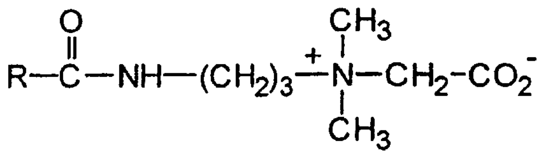

wherein R has from about 9 to about 13 carbon atoms

Cocamidopropyl hydroxy sultaine

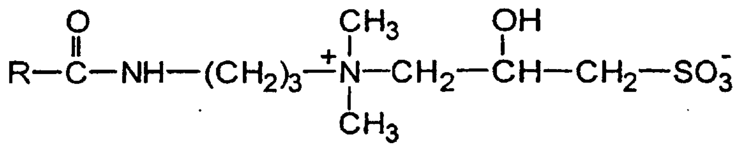

wherein R has from about 9 to about 13 carbon atoms,

Examples of other useful amphoteric surfactants are alkyliminoacetates, and iminodialkanoates and aminoalkanoates of the formulas $RN[CH_2)_mCO_2M]_2$ and $RNH(CH_2)_mCO_2M$ wherein m is from 1 to 4, R is a $C_8$-$C_{22}$ alkyl or alkenyl, and M is H, alkali metal, alkaline earth metal ammonium, or alkanolammonium. Also included are imidazolinium and ammonium derivatives. Specific examples of suitable amphoteric surfactants include sodium 3-dodecyl-aminopropionate, sodium 3-dodecylaminopropane sulfonate, N-higher alkyl aspartic acids such as those produced according to the teaching of U.S. Patent 2,438,091; and the articles sold under the trade name "Miranol" and described in U.S. Patent 2,528,378. Other examples of useful amphoterics include amphoteric phosphates, such as coamidopropyl PG-dimonium chloride phosphate (commercially available as Monaquat PTC, from Mona Corp.). Also useful are amphoacetates such as disodium lauroamphodiacetate, sodium lauroamphoacetate, and mixtures thereof.

Preferred lathering surfactants for use herein are the following, wherein the anionic lathering surfactant is selected from the group consisting of ammonium lauroyl sarcosinate, sodium trideceth sulfate, sodium lauroyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cetyl sulfate, and mixtures thereof; wherein the nonionic lathering surfactant is selected from the group consisting of lauramine oxide, cocoamine oxide, decyl polyglucose, lauryl polyglucose, sucrose cocoate, C12-14 glucosamides, sucrose laurate, and mixtures thereof; and wherein the amphoteric lathering surfactant is selected from the group consisting of disodium lauroamphodiacetate, sodium lauroamphoacetate, cetyl dimethyl betaine, cocoamidopropyl betaine, cocoamidopropyl hydroxy sultaine, and mixtures thereof.

III. FRAGRANCE COMPONENT

The products of the present invention comprise a fragrance component which increases consumer acceptance by providing an olfactory aesthetic benefit and/or which serves as a signal that the product is effective. These products deliver fragrances in an efficient, stable, and cost-effective manner. Generally, any fragrance or fragrance complex technology can be used in the products of the present invention. Preferred fragrance technologies are those which (i)

protect the fragrance from loss, oxidation, and chemical reaction, before use by the consumer and (ii) effectively release the fragrance upon wetting with an aqueous solution, e.g., water.

A. Fragrance-releasing complex

The personal cleansing articles of the present invention comprise from about 0.01% to about 20%, preferably from about 0.015% to about 15%, most preferably from about 0.02% to about 10%, by weight of the substrate, of a fragrance-releasing complex. The fragrance-releasing complex comprises a porous fragrance carrier and a fragrance which is impregnated within the fragrance carrier. Preferred fragrance-releasing complexes are those which (i) protect the fragrance from loss, oxidation, and chemical reaction, before use by the consumer and (ii) effectively release the fragrance upon wetting with an aqueous solution, e.g., water.

The ratio of the fragrance to the fragrance carrier within the fragrance-releasing complex typically ranges from 5:1 to 1:10, preferably from 5:1 to 1:2, more preferably from 2:1 to 1:2, depending on the absorbency of the fragrance carrier. When a fragrance carrier with a high absorbency is used, the ratio of fragrance:fragrance carrier is 5:1. When a carrier of low absorbency is used, the ratio of fragrance:fragrance carrier is 1:10. For fragrance carriers with moderate absorbencies, the ratio of fragrance:fragrance carrier lies between 5:1 and 1:10. Some typical ratios of fragrance: fragrance carrier for certain key fragrance carriers are listed in the table below:

| Fragrance Carrier | Ratio Fragrance:Fragrance Carrier |
|---|---|
| Silica | 2:1 |
| Zeolite | 1:6 |
| β-Cyclodextrin | 1:6 |

The Fragrance: The fragrance-releasing complex comprising the personal cleansing products of the present invention comprises from about 1% to about 90%, preferably from about 15% to about 80%, more preferably from about 40% to about 70%, by weight of the complex, of a fragrance. As used herein, the term "fragrance" can include perfume ingredients, cooling agents and other tactile agents, or a combination thereof.

The perfume ingredients employed in the personal cleansing products of the present invention are the conventional ones known in the art. Even perfume ingredients which are unstable due to volatility (as exhibited by changes in intensity) or discoloration when used in their neat form are stable and suitable for use in the personal cleansing products of the present invention when they are impregnated in a fragrance carrier as hereinafter described. As used herein, a fragrance is considered to be "stable" if the fragrance does not exhibit appreciable changes in color or intensity and does not exhibit appreciable loss due to volatility after 10 days at 120°F.

Suitable perfume compounds and compositions can be found in the art including U.S. Pat. Nos.: 4,145,184, Brain and Cummins, issued Mar. 20, 1979; 4,209,417, Whyte, issued June 24, 1980; 4,515,705, Moeddel, issued May 7, 1985; and 4,152,272, Young, issued May 1, 1979, U.S. 5,378,468 Suffis et al., issued January 3, 1995; U. S. 5,266,592 Grub et al., issued November 30, 1993; U. S. 5,081,111 Akimoto et al., issued January 14, 1992; U. S. 4,994,266 Wells, issued February 19, 1991; U.S. 4,524,018 Yemoto et al., issued June 18, 1985; U. S. 3,849,326 Jaggers et al., issued November 19, 1974; U. S. 3,779,932 Jaggers et al., issued December 18, 1973; JP 07-179,328 published July 18, 1995; JP 05-230496 published September 7, 1993; WO 96/38528 published December 5, 1996; WO 96/14827 published May 23, 1996; WO 95/04809 published February 16, 1995; and WO 95/16660 published June 22, 1995. In addition, P.M. Muller, D. Lamparsky Perfumes Art, Science, & Technology Blackie Academic & Professional, (New York, 1994).

Perfumes can be classified according to their volatility. The highly volatile, low boiling, perfume ingredients typically have boiling points of about 250°C or lower. The moderately volatile perfume ingredients are those having boiling points of from about 250°C to about 300°C. The less volatile, high boiling, perfume ingredients are those having boiling points of about 300°C or higher. Many of the perfume ingredients as discussed hereinafter, along with their odor and/or flavor characters, and their physical and chemical properties, such as boiling point and molecular weight, are given in "Perfume and Flavor Chemicals (Aroma Chemicals)," Steffen Arctander, published by the author, 1969.

Examples of the highly volatile, low boiling, perfume ingredients are: anethole, benzaldehyde, benzyl acetate, benzyl alcohol, benzyl formate, iso-bornyl acetate, camphene, cis-citral (neral), citronellal, citronellol, citronellyl acetate, paracymene, decanal, dihydrolinalool, dihydromyrcenol, dimethyl phenyl carbinol, eucalyptol, geranial, geraniol, geranyl acetate, geranyl nitrile, cis-3-hexenyl acetate, hydroxycitronellal, d-limonene, linalool, linalool oxide, linalyl acetate, linalyl propionate, methyl anthranilate, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, phenyl ethyl alcohol, alpha-pinene, beta-pinene, gamma-terpinene, alpha-terpineol, beta-ter-pineol, terpinyl acetate, and vertenex (para-tertiary-butyl cyclohexyl acetate). Some natural oils also contain large percentages of highly volatile perfume ingredients. For example, lavandin contains as major components: linalool; linalyl acetate; geraniol; and citronellol. Lemon oil and orange terpenes both contain about 95% of d-limonene.

Examples of moderately volatile perfume ingredients are: amyl cinnamic aldehyde, iso-amyl salicylate, beta-caryophyllene, cedrene, cinnamic alcohol, coumarin, dimethyl benzyl carbinyl acetate, ethyl vanillin, eugenol, iso-eugenol, flor acetate, heliotropine, 3-cis-hexenyl salicylate, hexyl salicylate, lilial (para-tertiarybutyl-alpha-methyl hydrocinnamic aldehyde), gamma-methyl ionone, nerolidol, patchouli alcohol, phenyl hexanol, beta-selinene, trichloromethyl phenyl carbinyl acetate, triethyl citrate, vanillin, and veratraldehyde. Cedarwood terpenes are composed mainly of alpha-cedrene, beta-cedrene, and other $C_{15}H_{24}$ sesquiterpenes.

Examples of the less volatile, high boiling, perfume ingredients are: benzophenone, benzyl salicylate, ethylene brassylate, galaxolide (1,3,4,6,7,8-hexahydro-4,6,6,7,8,8-hexamethyl-cyclopenta-gama-2-benzopyran), hexyl cinnamic aldehyde, lyral (4-(4-hydroxy-4-methyl pentyl)-3-cyclohexene-10-carboxaldehyde), methyl cedrylone, methyl dihydro jasmonate, methyl-beta-naphthyl ketone, musk indanone, musk ketone, musk tibetene, and phenylethyl phenyl acetate.

As hereinbefore indicated, the fragrance employed in the personal cleansing products of the present invention can also comprise a cooling agent or a combination of cooling agents. Cooling agents are compounds which directly effect those nerve endings responsible for hot or cold sensations. Suitable cooling agents are menthol, menthol-based or acyclic carboximides, camphor, Eucalyptus, and menthol-based or acyclic ketals (acetals). The cooling agents particularly preferred for use in the personal cleansing products of the present invention are those selected from the group consisting of 3-1-menthoxy propane-1,2-diol, N-substituted-p-menthane-3-carboxamides and acyclic carboxamides and mixtures thereof.

3-1-menthoxy propane 1,2-diol is fully described in detail in U.S. Patent 4,459,425, issued July 10, 1984 to Amano et. al. This volatile aromatic is commercially available, as TK-10 from Takasago Perfumery Co., Ltd., Tokyo, Japan.

The N-substituted-p-menthane-3-carboxamides are fully described in U.S. Patent 4,136,163 to Watson et al., issued January 23, 1979 incorporated herein by reference in its entirety. The most preferred cooling agent of this class is N-ethyl-p-menthane-3-carboxamide which is commercially available as WS-3 from Wilkinson Sword Limited.

Useful acyclic carboxamides are fully described in U.S. Patent 4,230,688 to Rowsell et al., issued October 28 1980. The most preferred cooling agent of this class is N,2,3-trimethyl-2-isopropyl-butanamide which is commercially available as WS-23 from Wilkinson Sword Limited.

Preferred for use herein is a mixture of 3-1-menthoxy propane 1,2-diol, N-ethyl-p-menthane-3-carboxamide and N,2,3-trimethyl-2-isopropylbutanamide in a ratio of 1:75:42, respectively.

<u>The Fragrance Carrier</u>: The fragrance-releasing complex employed in the personal cleansing products of the present invention comprises from about 10% to about 90%, preferably from about 20% to about 85%, more preferably from about 30% to about 60%, by weight of the complex, of a porous fragrance carrier. The fragrance carrier is typically present in the personal cleansing products of the present invention at a level ranging from about 0.01% to about 10%, preferably from about 0.1% to about 5%, more preferably from about 0.2% to about 2%, by weight of the substrate. The fragrance carriers improve fragrance delivery. By "improving fragrance delivery" is meant that the fragrance stability and in-use fragrance bloom are improved.

The fragrance carriers employed in the personal cleansing products of the present invention comprise particles having a diameter of from about 0.001 micron to about 50 microns, preferably from about 0.01 to about 20 microns, more preferably from about 0.1 to about 10 microns. As used herein, a "carrier particle" means a particle which entraps a fragrance (e.g., perfume oil) in the dry (e.g., neat) personal cleansing product and releases entrapped fragrance when the product gets wet. Suitable carrier particles include, but are not limited to, cyclodextrins, silicas (e.g., fumed silica, colloidal silica, spheroidal silica, amorphous silicas, precipitated silicas, and calcium silicate), starches (e.g., porous starch and agglomerated starch), polymethacrylate copolymers, and mixtures thereof, as described in U.S. Pat. No. 5,236,615, to Trinh et al., issued Aug. 17, 1993; U.S. Pat. No. 5,139,687, to Borgher, Sr. et al., issued Aug. 18, 1992; U.S. Pat. No. 5,552,378, to Trinh et al., issued Sept. 3, 1996; U.S. Pat. No. 5,246, 611, to Trinh, issued Sept. 21, 1993; U.S. Pat. No. 5,185,155, to Behan et al., issued Feb. 9, 1993; U.S. Pat. No. 5,112,612, to Garvey et al., issued May 12, 1992; U.S. Pat. No. 5292,533, to McMahon et al., issued Mar. 8, 1994;U.S. Pat. No. 5,466,460, to McMahon et al., issued Nov. 14, 1995; U.S. Pat. No. 5,376,287, to Borcher, Sr. et al., issued Dec. 27, 1994.

One type of inorganic carriers suitable for use in the present invention include amorphous silica, precipitated silica, fumed silica and aluminosilicates such as zeolite and alumina with a pore volume of at least 0.1 ml/g consisting of pores with a diameter between 4 and 100 A, which by their nature are hydrophilic. Preferably, amorphous Silica gel is used because of its high oil absorbency. Silica gel particles include SyloidR silicas such as Numbers: 72; 74; 221; 234; 235; 244; etc. SyloidR silicas are available from W.R. Grace & Co., Davison Chemical Division, P.O. Box 2117, Baltimore, Maryland 21203. Such particles have surface areas of from about 250 to about 340 $m^2/g$; pore volumes of from about 1.1 to about 1.7 cc/g; and average particle sizes of from about 2.5 to about 6 microns. Fumed silica particles

have primary particle diameters of from about 0.007 to about 0.025 micron and include Cab-O-SilR Numbers: L-90; LM-130; LM-5; M-5; PTG; MS-55; HS-5; and EH-5. Cab-O-SilR silicas are available from Cabot Corp., P.O. Box 188, Tuscola, Illinois 61953. It is preferred that there be only minimal amounts of other materials present when the perfume is added to the silica particles to maximize adsorption. It is especially preferred that only small amounts, e.g., less than about 10% of organic materials, including waxes, be present.

Another type of inorganic carrier suitable for use in the present invention include cyclodextrin. As used herein, the term "cyclodextrin" (CD) includes any of the known cyclodextrins such as unsubstituted cyclodextrins containing from six to twelve glucose units, especially, alpha-, beta-, gamma-cyclodextrins, and mixtures thereof, and/or their derivatives, and/or mixtures thereof, that are capable of forming inclusion complexes with perfume ingredients. Alpha-, beta-, and gamma-cyclodextrins can be obtained from, among others, American Maize-Products Company (Amaizo), Corn Processing Division, Hammond, Indiana; and Roquette Corporation, Gurnee, Illinois. There are many derivatives of cyclodextrins that are known. Representative derivatives are those disclosed in U.S. Pat. Nos: 3,426,011, Parmerter et al., issued Feb. 4, 1969; 3,453,257, 3,453,258, 3,453,259, and 3,453,260, all in the names of Parmerter et al., and all issued Jul. 1, 1969; 3,459,731, Gramera et al., issued Aug. 5, 1969; 3,553,191, Parmerter et al., issued Jan. 5, 1971; 3,565,887, Parmerter et al., issued Feb. 23, 1971; 4,535,152, Szejtli et al., issued Aug. 13, 1985; 4,616,008, Hirai et al., issued Oct. 7, 1986; 4,638,058, Brandt et al., issued Jan. 20, 1987; 4,746,734, Tsuchiyama et al., issued May 24, 1988; and 4,678,598, Ogino et al., issued Jul. 7. Examples of cyclodextrin derivatives suitable for use herein are methyl-β-CD, hydroxyethyl-β-CD, and hydroxypropyl-β-CD of different degrees of substitution (D.S.), available from Amaizo and from Aldrich Chemical Company, Milwaukee, Wisconsin. Water-soluble derivatives are also highly desirable.

The individual cyclodextrins can also be linked together, e.g., using multifunctional agents to form oligomers, cooligomers, polymers, copolymers, etc. Examples of such materials are available commerically from Amaizo and from Aldrich Chemical Compnay (β-CD/epichlorohydrin copolymers).

It is also desireable to use mixtures of cyclodextrins and/or precursor compounds to provide a mixture of complexes. Such mixtures, e.g., can provide more even odor profiles by encapsulating a wider range of perfume ingredients and/or preventing formation of large crystals of said complexes. Mixtues of cyclodextrins can conveniently be obtained by using intermediate products from known processes for the preparation of cyclodextrins including those processes described in U.S. Pat. Nos.: 3,425,910, to Armbruster et al., issued Feb. 4, 1969; 3,812,011, to Okada et al., issued May 21, 1974; 4,317,881 to Yagi et al., issued Mar. 2, 1982; 4,418,144, to Okada et al., issued Nov. 29, 1983; and 4,738,923, to Ammeraal, issued Apr. 19, 1988. Preferably at least a major portion of the cyclodextrins are alpha-cyclodextrin, beta-cyclodextrin, and/or gamma-cyclodextrin, more preferably beta-cyclodextrin. Some cyclodextrin mixtures are commercially available from, e.g., Ensuiko Sugar Refining Company, Yokohama, Japan.

The fragrance carriers comprising the fragrance-release complexes of the present invention can be incorporated into the personal cleansing products as is or they can be encapsulated in, e.g., waxy materials, such as fatty acids.

Complex Formation: The complexes of this invention are formed in any of the ways known in the art. Typically, the complexes are formed either by bringing the perfume and the cyclodextrin together as solutions in suitable solvents, preferably water, or in suspension or by kneading the ingredients together in the presence of a suitable, preferably minimal, amount of solvent, preferably water. Other polar solvents such as ethylene glycol, propylene glycol, diethylene glycol, triethylene glycol, 2-methoxy ethanol, 2-ethoxy ethanol, glycerine, dimethylsulfoxide, dimethylformamide, 1,2-propanediol, ethanol, methanol, isopropanol, etc., and mixtures of said polar solvents with themselves and/or with water can be used as solvents for complex formation. The use of such solvents in complex formation has been disclosed in an article in Chemistry Letters by A. Harada and S. Takahashi, pp. 2089-2090 (1984).

The suspension/kneading method is particularly desirable because less solvent is needed and therefore less separation of the solvent is required. Suitable processes are disclosed in the patents incorporated hereinbefore by reference. Additional disclosures of complex formation can be found in Atwood, J.L., J.E.D. Davies & D.D. MacNichol, (Ed.): Inclusion Compounds, Vol. III, Academic Press (1984), especially Chapter 11; Atwood, J.L. and J.E.D. Davies (Ed.): Proceedings of the Second International Symposium of Cyclodextrins Tokyo, Japan, (July, 1984); Cyclodextrin Technology, J. Szejtli, Kluwer Academic Publishers (1988).

In general, the fragrance releasing complexes have a molar ratio of fragrance to carrier (e.g., cyclodextrin) of about 1:1. However, the molar ratio can be either higher or lower, depending on the molecular size of the active compound and the identity of the cyclodextrin compound. The molar ratio can be determined easily by forming a saturated solution of the carrier and adding the fragrance to form the complex. In general the complex will precipitate readily. If not, the complex can usually be precipitated by the addition of electrolyte, change of pH, cooling, etc. The complex can then be analyzed to determine the ratio of fragrance to carrier.

The actual complexes are determined by the size of the cavity in the carrier and the size of the fragrance molecule. Although the normal complex is one molecule of fragrance in one molecule of carrier, complexes can be formed between one molecule of fragrance and two molecules of carrier when the fragrance molecule is large and contains two portions that can fit in the carrier. Highly desirable complexes can be formed using mixtures of carrier

sizes since some fragrances are normally mixtures of materials that vary widely in size. It is usually desirable that at least a majority of the material be alpha-, beta-, and/or gamma-cyclodextrin, more preferably beta-cyclodextrin.

Processes for the production of carriers and complexes are described in U.S. Pat. Nos.: 3,812,011, Okada, Tsuyama, and Tsuyama, issued May 21, 1974; 4,317,881, Yagi, Kouno and Inui, issued Mar. 2, 1982; 4,418,144, Okada, Matsuzawa, Uezima, Nakakuki, and Horikoshi, issued Nov. 29, 1983; 4,378,923, Ammeraal, issued Apr. 19, 1988. Materials obtained by any of these variations are acceptable for the purposes of this invention. It is also acceptable to initially isolate the inclusion complexes directly from the reaction mixture by crystallization.

Continuous operation usually involves the use of supersaturated solutions, and/or suspension/kneading, and/or temperature manipulation, e.g., heating and then either cooling, freeze-drying, etc. The complexes can be dried or not depending on the next step in the process for making the desired composition. Incorporation of the complex/solvent (water) mixture in some carriers, e.g., polyalkylene glycol, eliminates the need for a drying step. Thus, it is desirable to use the wet, undried complex slurry with a liquid carrier for improved handling and ease of incorporation into subsequent compositions. In general, the fewest possible process steps are used to avoid loss of active and excessive processing costs.

Encapsulation of the Fragrance releasing-complex: It is preferred to encapsulate the fragrance-releasing-complex with a coating material. The preferred coating materials include both water-insoluble and water-soluble materials. Water-soluble materials are more preferred because of they allow quick release of fragrance in the presence of an aqeous solution, e.g., water. Suitable encapsulation materials and techniques are described in U.S. Pat. No. 5,236,615, to Trinh et al., issued Aug. 17, 1993; U.S. Pat. No. 5,139,687, to Borgher, Sr. et al., issued Aug. 18, 1992; U.S. Pat. No. 5,552,378, to Trinh et al., issued Sept. 3, 1996; U.S. Pat. No. 5,246, 611, to Trinh, issued Sept. 21, 1993; U.S. Pat. No. 5,185,155, to Behan et al., issued Feb. 9, 1993; U.S. Pat. No. 5,112,612, to Garvey et al., issued May 12, 1992; U.S. Pat. No. 5292,533, to McMahon et al., issued Mar. 8, 1994;U.S. Pat. No. 5,466,460, to McMahon et al., issued Nov. 14, 1995; U.S. Pat. No. 5,376,287, to Borcher, Sr. et al., issued Dec. 27, 1994.

Nonlimiting examples of suitable water-insoluble materials are typically selected from waxy materials such as paraffinic waxes, microcrystalline waxes, animal waxes, vegetable waxes, saturated fatty acids and fatty alcohols having from 12 to 40 carbon atoms in their alkyl chain, and fatty esters such as fatty acid triglycerides, fatty acid esters of sorbitan and fatty acid esters of fatty alcohols, or from water-insoluble polymers. Typical specific suitable waxy coating materials include lauric, myristic, palmitic, stearic, arachidic and behenic acids, stearyl and behenyl alcohol, microcrystalline wax, beeswax, spermaceti wax, candelilla wax, sorbitan tristearate, sorbitan tetralaurate, tripalmitin, trimyristin and octacosane. A preferred waxy material is coconut fatty acid.

Nonlimiting examples of suitable water-soluble materials include, but are not limited to, polyalkylene glycol materials, water-soluble polymers, glycerol esters, and polyols.

Especially preferred are polyalkylene glycols that are liquid or molten at less than about 100°C, especially polyalkylene glycol materials such as:

(A) Polyalkylene glycols and/or mixed polyalkylene glycols having average molecular weights (MW) of from about 400 to about 20,000, preferably between about 600 and about 10,000. Examples include:

polyethylene glycols, preferably having molecular weights of from about 1,000 to about 400,000, more preferably having molecular weights of from about about 4,400 to about 400,000. Suitable examples polyethylene glycols ("PEG") include, but is not limited to, PEG 600, PEG 1450, PEG 3350, PEG 4600, and PEG 8000, as designated by CTFA Cosmetic Ingredient Handbook, Second Edition, (1992);

polypropylene glycols, preferably having molecular weights of from about 600 to about 4,000; poly(tetramethylene glycol), preferably having molecular weights of from about 1,000 to about 10,000;

mixed polyalkylene glycols such as poly(ethylene oxide-propylene oxide). Examples: average MW 1,100, E/P ratio 0.15:1; average MW 3,440, E/P ratio 0.33:1; average MW 2,920, E/P ratio 0.8:1; average MW 13,333, E/P ratio 3:1; and average MW 8,750, E/P ratio 5:1; and

mixed polyalkylene glycol block copolymers such as $HO-[CH_2CH_2O]_x-[CH_2CH(CH_3)O]_y-[CH_2CH_2O]_x-H$ and/or $HO-[CH(CH_3)CH_2O]_y-[CH_2CH_2O]_x-[CH_2CH(CH_3)O]_y-H$ wherein the sum of the y's ranges from about 15 to about 70, and the ratio of the sum of the x's to the sum of the y's is from about 1:10 to about 11:10, preferably from about 1:2 to about 1:1. Examples include materials made by BASF Corporation and sold under the trade names of PluronicR and Pluronic RR surfactants, respectively.

(B) $C_1$-$C_{22}$, preferably $C_1$-$C_4$ alkylated polyalkylene glycols [poly(alkylene glycol) mono- and dialkyl ethers], $RO-(R^2O)_n-H$ and/or $RO-(R^2O)_n-R$, with each R being methyl, ethyl, propyl, or butyl; each $R^2$ being a $C_2$-$C_4$ alkylene group; and n ranging from 1 to about 200, with the percentage of polyalkylene glycol being preferably more than about 50%. Specific examples include:

$RO-[CH_2CH(CH_3)O]_m-H$, with R being methyl, ethyl, propyl, or butyl; and m being from 1 to about 200 (MW from about 90 to about 20,000);

$RO-(CH_2CH_2O)_n-H$, with each R being methyl, ethyl, propyl, or butyl, preferably methyl; and n being from about 2 to about 200 (MW from about 120 to about 9,000), preferably from about 15 to about 150 (MW from about 700 to about 6,700), more preferably from about 15 to about 100 (MW from about 700 to about 4,500); and/or

$RO-(CH_2CH_2O)_n-R$, with each R being methyl, ethyl, propyl, or butyl; and n being from about 2 to about 200 (MW from about 134 to about 9,000), preferably from about 15 to about 150 (MW from about 700 to about 6,700), more preferably from about 15 to about 100 (MW from about 700 to about 4,500).

(C) Polyalkoxylated materials having an average molecular weight of from about 200 to about 20,000 and the weight percent of the polyalkoxy portion being from about 50% to about 99%. Specific examples include: Tetronic ® and Tetronic R ®; and Varstat 66 ®. Tetronic ® and Tetronic R ® are block copolymeric surfactants, manufactured by BASF Corporation. Tetronic ® surfactants have the general formula:

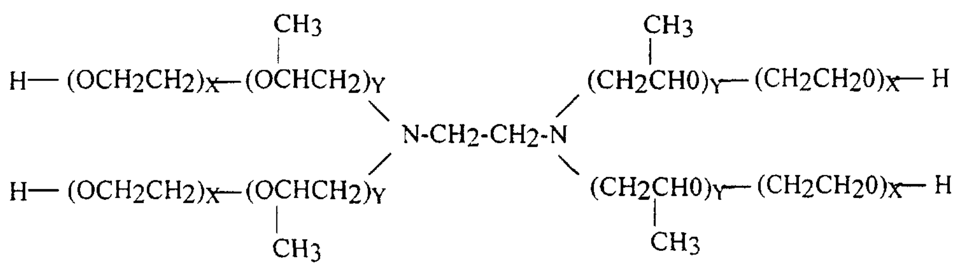

and Tetronic R ® surfactants have the general formula:

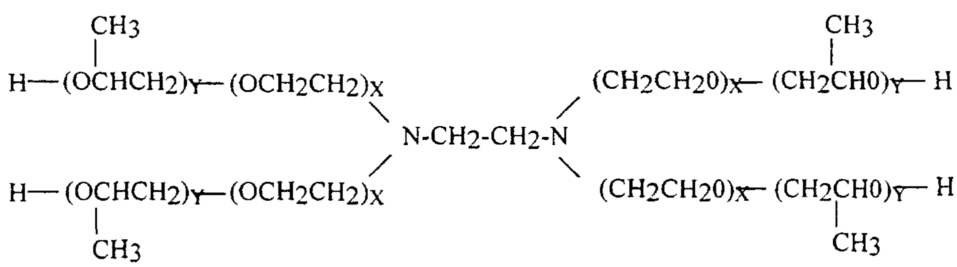

wherein the sum of the y's ranges from about 8 to about 120, and the ratio of the sum of the x's to the sum of the y's is from about 1:10 to about 11:10, preferably from about 1:2 to about 1:1.

Varstat 66R, sold by Sherex Chemical Company, has the formula

$$[H-(OCH_2CH_2)_p-N\oplus(C_2H_5)(R^3)-(CH_2CH_2O)_q-H]C_2H_5SO_4^{\ominus}$$

with $R^3$ being a $C_{12}$-$C_{18}$ alkyl or alkenyl radical, and with p + q being preferably from about 10 to about 30. Surfynol 465 ®, sold by Air Products and Chemicals, Inc., is an ethylene oxide adduct of 2,4,7,9,tetramethyl-5-decyn-4,7-diol of the formula

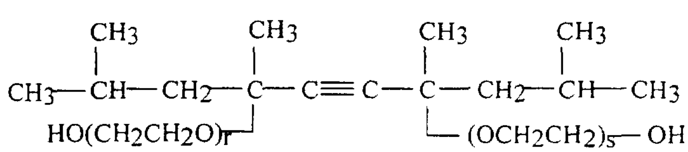

with r + s being about 8. In Surfynol 465 ® the weight percent of the polyethylene oxide portion is about 65%. The encapsulation materials can contain other moieties so long as they do not disrupt the complex excessively.

The weight ratio of the complex to the encapsulation material is from about 1:1 to about 1:5, preferably from about 2:3 to about 1:3. The level of the encapsulation material has to be relatively high so that the complex can be supported and the mixture of complex and encapsulation material can be relatively fluid when the carrier is in a liquid state.

Preferred encapsulation materials are those that are solid at room temperature but can become molten or fluid below about 100°C, more preferably those that can become molten or fluid below about 80°C.

Specific examples are:

polyethylene glycols with an average MW of from about 600 to about 20,000;
poly(tetramethylene glycols) with an average MW of from about 1,000 to about 10,000; and
poly(ethylene glycol) methyl ether with an average MW of from about 600 to about 20,000.

The complexes herein are desirably formed by a process, of the type described hereinbefore, in which cyclodextrin is mixed with the fragrance, preferably perfume, in a limited amount of water, then the water is dried off by air or by lyophilization, as described hereinafter. The complex is then admixed with the liquid encapsulation material, or preferably with the molten normally solid encapsulation material, at a ratio of the complex to the encapsulation material of from about 1:1 to about 1:5, to form pumpable fluid complex compositions for further processing.

A preferred composition and process comprises applying the molten mixtures of (a) dry frangrance/carrier complex and (b) the normally solid hydrophilic polyethylene glycol material onto a solid substrate surface, then letting the droplets solidify on said surface. Said droplets are readily dissolvable by water or other aqueous media such as body fluids (e.g., sweat, saliva, urine, menses, etc.) to release the active.

Said hydrophilic polyethylene glycol materials have the general formula $RO-(CH_2CH_2O)_n-R$ wherein each R is a hydrogen radical, a $C_1$-$C_{22}$ alkyl or alkenyl radical, or mixtures of such radicals, and n is from about 13 to about 450 (average MW of from about 600 to about 20,000) with the percentage of polyethylene glycol preferably being more than about 50%. Preferred R groups include a hydrogen radical, $C_1$-$C_4$ alkyl radicals, or mixtures of such radicals. More preferred polyethylene glycol materials are the hydrophilic polyethylene glycols, poly(ethylene glycol) methyl ethers, or mixtures thereof, with average MW's of from about 600 to about 20,000 (n from about 13 to about 450), preferably from about 1,000 to about 9,000 (n from about 20 to about 200), more preferably from about 1,400 to about 4,500 (n from about 30 to about 100). The weight ratio of the complex to the polyethylene glycol material is from about 1:1 to about 1:5, preferably from about 1:2 to about 1:4.

Other preferred compositions and processes involve prilling molten mixtures of (a) dry fragrance/carrier complex and normally solid hydrophilic polyethylene glycol material as described above by, e.g., spray drying, marumarizing, etc., into solid prills with particle sizes of from about 10 microns to about 1,000 microns, preferably from about 50 microns to about 600 microns. Said solid prills can then be used, e.g., either (a) attached to a solid substrate surface by distributing the prills on said surface, melting said prills, and then resolidifying to bind said prills to said surface or (b) placed in a water-insoluble, but porous, pouch or enclosure. These articles will readily release the active when treated with water or other aqueous media.

Another preferred composition and process comprises forming the complex in the presence of a limited amount of solvent, e.g., water, then without the solvent (water) being removed, the normally solid polyethylene glycol materials are admixed in molten form with the complex/water mixture to form a pumpable mixture that can be used directly to form solid compositions by mixing with molten materials that would ordinarily not be compatible with complex/water mixture alone.

In the above composition and process which utilize a mixture of encapsulation material and solvent to suspend the complex, the ratio of encapsulation material to complex typically varies from about 0.5 to about 3, preferably from about 0.6 to about 2, and more preferably from about 0.75 to about 1. The ratio of solvent plus encapsulation material to complex typically varies from about 1:1 to about 5:1, preferably from about 1:1 to about 3:1. Preferably there is more encapsulation material than solvent, the solvent is water, and/or the encapsulation material is polyethylene glycol or alkylated polyethylene glycol, preferably having a molecular weight of from about 600 to about 20,000, and more preferably from about 1,000 to about 9,000.

The process using a mixture of encapsulation material and solvent is also desirable because removal of the solvent adds an additional step, or steps, and can result in loss of some active, e.g., perfume.

The polyalkylene glycol materials preferably do not have any hydrophobic end group that will displace the active from the cyclodextrin. The polyalkylene glycols can contain other monomers in the chains, but the level of other monomers should be kept low to avoid displacement of the active from the cyclodextrin complex. Surprisingly, the complexes are effectively dispersed in the above carrier (solvent) but are not destroyed, e.g., by the carrier displacing the complexed active, e.g., perfume. Solvents such as ethylene glycol, propylene glycol, ethanol, glycerin, and molten sorbitol can form pumpable slurries, but will at least partially dissolve the complexes and thereby release the active.

Once the complexes are dispersed in the encapsulation material, the complexes can be applied directly to

substrates by using the suspension of complex in the carrier to achieve good distribution. E.g., the fragrance/carrier in the encapsulation material can be sprayed and/or spread onto the desired surface. Propellants, or air under pressure, can be used to form a dispersion of the encapsulation material and complex. The complexes can release some of the active (perfume) when exposed to water in the atmosphere, but, surprisingly, a large amount of active, even volatile perfume active, remains in the complexes attached to the surface.

When the encapsulation material is used to enrobe and/or protect the complex and/or to attach the complex to a substrate, the carrier is preferably solid at normally encountered temperatures. Polypropylene glycols are not solids so they will normally be used only as part of a mixture of encapsulation materials. Whether a specific carrier or mixture of carriers is solid can be readily determined by inspection.

Examples of polymeric materials which can be used for the coating of the particles herein are cellulose ethers such as ethyl, propyl or butyl cellulose; cellulose esters such as cellulose acetate, propionate, butyrate or acetate-butyrate; polyalkylene glycol such as ethylene, propylene, tetramethylene glycol; ureaformaldehyde resins, polyvinyl alcohol, polyvinyl chloride, polyvinylidene chloride, polyethylene, polypropylene, polyacrylates, polymethacrylates, polymethyl-methacrylates and nylon. Such materials and their equivalents are described in greater detail in any conventional handbook of synthetic organic plastics, for example, in Modern Plastics Encyclopaedia Volume, Vol. 62, No. 10A (for 1985-1986) at pages 768-787, published by McGraw-Hill, New York, N.Y. (October 1985). A preferred polymeric material is ethyl cellulose. The polymeric coating materials can be plasticized with known plasticizing agents such as phthalate, adipate and sebacate esters, polyols (e.g., ethylene glycol), tricresyl phosphate, castor oil and camphor. These polymeric coatings are preferred for the superior protection they provide.

The coating when present, is generally present in an amount of from about 2% to about 50%, preferably from about 20% to about 40% by weight of the fragrance-releasing complex.

The coating material can comprise a mixture of water-soluble coating materials, water-insoluble coating materials, and polymeric coating materials. In such mixtures the waxy coating material will typically comprise from about 10% to about 90% of the mixture and the polymeric material about 10% to about 90%.

The function of the coating which surrounds the fragrance-releasing complex is to provide further improved stability, as well as to allow for dual delivery of fragrances wherein different fragrances can be impregnated in various complexes.

Impregnation of the Fragrance within the Carrier: At least a portion of the fragrance employed in the personal cleansing products of the present invention is impregnated within the hereinbefore-described fragrance carrier. To impregnate the fragrance within the fragrance carrier, the fragrance and the carrier are mixed together under shear conditions to provide a homogeneous mixture.

Neat Fragrances: In addition to the fragrances impregnated within the fragrance carrier, the personal cleansing products of the present invention can also optionally contain fragrances present in their neat form (e.g., not impregnated within a fragrance carrier). Incorporating a neat fragrance into the personal cleansing products herein can contribute to unique fragrance impressions for the product. For example, a personal cleansing product which contains both a fragrance impregnated within a fragrance carrier and a neat fragrance can 1) give a dual fragrance impression (e.g., can exhibit different fragrance impressions for the dry(neat) product versus the in-use product), or 2) can optimize the fragrance impression for both the neat product and the in-use product.

The fragrances which can be used as neat fragrances for the personal cleansing products of the present invention are the same as those hereinbefore described for incorporation into the fragrance-releasing complex. The neat fragrance is typically present in an amount ranging from about 0.01% to about 10%, preferably from about 0.01% to about 2%, more preferably from about 0.1% to about 2% by weight of the substrate. The total fragrance (e.g., neat fragrance and fragrance incorporated into fragrance-releasing complex) typically present in the products of the present invention ranges from about 0.01 to about 10%, preferably from about 0.1 to about 5%, more preferably from about 0.4 to about 2%.

## IV. CONDITIONING COMPONENT

The articles of the present invention can preferably comprise a conditioning component which is useful for providing a conditioning benefit to the skin or hair during the use of the article. The conditioning component comprises from about 0.05% to about 99%, preferably from about 0.1% to about 50%, and more preferably from about 1% to about 25% by weight of said water insoluble substrate.

The conditioning component of the present invention can comprise: a water soluble conditioning agent; an oil soluble conditioning agent; conditioning emulsion; lipid hardening material; or any combination or permutation of the four. The oil soluble conditioning agent is selected from one or more oil soluble conditioning agents such that the weighted arithmetic mean solubility parameter of the oil soluble conditioning agent is less than or equal to 10.5. The water soluble conditioning agent is selected from one or more water soluble conditioning agents such that the weighted arithmetic mean solubility parameter of the water soluble conditioning agent is greater than 10.5. It is recognized, based

on this mathematical definition of solubility parameters, that it is possible, for example, to achieve the required weighted arithmetic mean solubility parameter, i.e. less than or equal to 10.5, for an oil soluble conditioning agent comprising two or more compounds if one of the compounds has an individual solubility parameter greater than 10.5. Conversely, it is possible to achieve the appropriate weighted arithmetic mean solubility parameter, i.e. greater than 10.5, for a water soluble conditioning agent comprising two or more compounds if one of the compounds has an individual solubility parameter less than or equal to 10.5.

Solubility parameters are well known to the formulation chemist of ordinary skill in the art and are routinely used as a guide for determining compatibilities and solubilities of materials in the formulation process.

The solubility parameter of a chemical compound, δ, is defined as the square root of the cohesive energy density for that compound. Typically, a solubility parameter for a compound is calculated from tabulated values of the additive group contributions for the heat of vaporization and molar volume of the components of that compound, using the following equation:

$$\delta = \left[ \frac{\sum_i E_i}{\sum_i m_i} \right]^{1/2}$$

wherein $\Sigma_i E_i$ = the sum of the heat of vaporization additive group contributions, and

$\Sigma_i m_i$ = the sum of the molar volume additive group contributions

Standard tabulations of heat of vaporization and molar volume additive group contributions for a wide variety of atoms and groups of atoms are collected in Barton, A.F.M. Handbook of Solubility Parameters, CRC Press, Chapter 6, Table 3, pp. 64-66 (1985), which is incorporated by reference herein in its entirety. The above solubility parameter equation is described in Fedors, R.F., "A Method for Estimating Both the Solubility Parameters and Molar Volumes of Liquids", Polymer Engineering and Science, vol. 14, no. 2, pp. 147-154 (February 1974).

Solubility parameters obey the law of mixtures such that the solubility parameter for a mixture of materials is given by the weighted arithmetic mean (i.e. the weighted average) of the solubility parameters for each component of that mixture. See, Handbook of Chemistry and Physics, 57th edition, CRC Press, p. C-726 (1976-1977).

Formulation chemists typically report and use solubility parameters in units of $(cal/cm^3)^{1/2}$. The tabulated values of additive group contributions for heat of vaporization in the Handbook of Solubility Parameters are reported in units of kJ/mol. However, these tabulated heat of vaporization values are readily converted to cal/mol using the following well-known relationships:

1 J/mol = 0.239006 cal/mol and 1000 J = 1 kJ. See Gordon, A.J. et al., The Chemist's Companion, John Wiley & Sons, pp. 456-463, (1972).

Solubility parameters have also been tabulated for a wide variety of chemical materials. Tabulations of solubility parameters are found in the above-cited Handbook of Solubility Parameters. Also, see "Solubility Effects In Product, Package, Penetration, And Preservation", C.D. Vaughan, Cosmetics and Toiletries, vol. 103, October 1988, pp. 47-69.

A. Oil Soluble Conditioning Agents

Nonlimiting examples of conditioning agents useful as oil soluble conditioning agents include those selected from the group consisting of mineral oil, petrolatum, $C_7$-$C_{40}$ branched chain hydrocarbons, $C_1$-$C_{30}$ alcohol esters of $C_1$-$C_{30}$ carboxylic acids, $C_1$-$C_{30}$ alcohol esters of $C_2$-$C_{30}$ dicarboxylic acids, monoglycerides of $C_1$-$C_{30}$ carboxylic acids, diglycerides of $C_1$-$C_{30}$ carboxylic acids, triglycerides of $C_1$-$C_{30}$ carboxylic acids, ethylene glycol monoesters of $C_1$-$C_{30}$ carboxylic acids, ethylene glycol diesters of $C_1$-$C_{30}$ carboxylic acids, propylene glycol monoesters of $C_1$-$C_{30}$ carboxylic acids, propylene glycol diesters of $C_1$-$C_{30}$ carboxylic acids, $C_1$-$C_{30}$ carboxylic acid monoesters and polyesters of sugars, polydialkylsiloxanes, polydiarylsiloxanes, polyalkarylsiloxanes, cylcomethicones having 3 to 9 silicon atoms, vegetable oils, hydrogenated vegetable oils, polypropylene glycol C4-C20 alkyl ethers, di C8-C30 alkyl ethers, and mixtures thereof.

Mineral oil, which is also known as petrolatum liquid, is a mixture of liquid hydrocarbons obtained from petroleum. See The Merck Index, Tenth Edition, Entry 7048, p. 1033 (1983) and International Cosmetic Ingredient Dictionary, Fifth Edition, vol. 1, p.415-417 (1993).

Petrolatum, which is also known as petroleum jelly, is a colloidal system of nonstraight-chain solid hydrocarbons and high-boiling liquid hydrocarbons, in which most of the liquid hydrocarbons are held inside the micelles. See The Merck Index, Tenth Edition, Entry 7047, p. 1033 (1983); Schindler, Drug. Cosmet, Ind., **89**, 36-37, 76, 78-80, 82 (1961); and International Cosmetic Ingredient Dictionary, Fifth Edition, vol. 1, p. 537 (1993).

Straight and branched chain hydrocarbons having from about 7 to about 40 carbon atoms are useful herein. Nonlimiting examples of these hydrocarbon materials include dodecane, isododecane, squalane, cholesterol, hydrogenated polyisobutylene, docosane (i.e. a $C_{22}$ hydrocarbon), hexadecane, isohexadecane (a commercially available hydrocarbon sold as Permethyl® 101A by Presperse, South Plainfield, NJ). Also useful are the C7-C40 isoparaffins, which are C7-C40 branched hydrocarbons.

Also useful are C1-C30 alcohol esters of C1-C30 carboxylic acids and of C2-C30 dicarboxylic acids, including straight and branched chain materials as well as aromatic derivatives. Also useful are esters such as monoglycerides of C1-C30 carboxylic acids, diglycerides of C1-C30 carboxylic acids, triglycerides of C1-C30 carboxylic acids, ethylene glycol monoesters of C1-C30 carboxylic acids, ethylene glycol diesters of C1-C30 carboxylic acids, propylene glycol monoesters of C1-C30 carboxylic acids, and propylene glycol diesters of C1-C30 carboxylic acids. Straight chain, branched chain and aryl carboxylic acids are included herein. Also useful are propoxylated and ethoxylated derivatives of these materials. Nonlimiting examples include diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palmitate, myristyl myristate, stearyl stearate, cetyl stearate, behenyl behenrate, dioctyl maleate, dioctyl sebacate, diisopropyl adipate, cetyl octanoate, diisopropyl dilinoleate, caprilic/capric triglyceride, PEG-6 caprylic/capric triglyceride, PEG-8 caprylic/capric triglyceride, and mixtures thereof.

Also useful are various C1-C30 monoesters and polyesters of glycerin and related materials. These esters are derived from glycerin and one or more carboxylic acid moities. Depending on the constituent acid and glycerin, these esters can be in either liquid or solid form at room temperature. Nonlimiting examples of solid esters include: glyceryl tribehenate, glyceryl stearate, glyceryl palmitate, glyceryl distearate, glyceryl dipalmitate.

Also useful are various C1-C30 monoesters and polyesters of sugars and related materials. These esters are derived from a sugar or polyol moiety and one or more carboxylic acid moities. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a 1:2 molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 2:6 molar ratio; and the octaester of sucrose wherein the esterifying carboxylic acid moieties are laurate, linoleate and behenate in a 1:3:4 molar ratio. A preferred solid material is sucrose polyester in which the degree of esterification is 7-8, and in which the fatty acid moieties are C18 mono- and/or di-unsaturated and behenic, in a molar ratio of unsaturates:behenic of 1:7 to 3:5. A particularly preferred solid sugar polyester is the octaester of sucrose in which there are about 7 behenic fatty acid moieties and about I oleic acid moiety in the molecule. Other materials include cottonseed oil or soybean oil fatty acid esters of sucrose. The ester materials are further described in, U.S. Patent No. 2,831,854, U.S. Patent No. 4,005,196, to Jandacek, issued January 25, 1977; U.S. Patent No. 4,005,195, to Jandacek, issued January 25, 1977, U.S. Patent No. 5,306,516, to Letton et al., issued April 26, 1994; U.S. Patent No. 5,306,515, to Letton et al., issued April 26, 1994; U.S. Patent No. 5,305,514, to Letton et al., issued April 26, 1994; U.S. Patent No. 4,797,300, to Jandacek et al., issued January 10, 1989; U.S. Patent No. 3,963,699, to Rizzi et al, issued June 15, 1976; U.S. Patent No. 4,518,772, to Volpenhein, issued May 21, 1985; and U.S. Patent No. 4,517,360, to Volpenhein, issued May 21, 1985.

Nonvolatile silicones such as polydialkylsiloxanes, polydiarylsiloxanes, and polyalkarylsiloxanes are also useful oils. These silicones are disclosed in U.S. Patent No. 5,069,897, to Orr, issued December 3, 1991. The polyalkylsiloxanes correspond to the general chemical formula $R_3SiO[R_2SiO]_xSiR_3$ wherein R is an alkyl group (preferably R is methyl or ethyl, more preferably methyl) and x is an integer up to about 500, chosen to achieve the desired molecular weight. Commercially available polyalkylsiloxanes include the polydimethylsiloxanes, which are also known as dimethicones, nonlimiting examples of which include the Vicasil® series sold by General Electric Company and the Dow Corning® 200 series sold by Dow Corning Corporation. Specific examples of polydimethylsiloxanes useful herein include Dow Corning® 225 fluid having a viscosity of 10 centistokes and a boiling point greater than 200°C, and Dow Corning® 200 fluids having viscosities of 50, 350, and 12,500 centistokes, respectively, and boiling points greater than

200°C. Also useful are materials such as trimethylsiloxysilicate, which is a polymeric material corresponding to the general chemical formula $[(CH_2)_3SiO_{1/2}]_x[SiO_2]_y$, wherein x is an integer from about 1 to about 500 and y is an integer from about 1 to about 500. A commercially available trimethylsiloxysilicate is sold as a mixture with dimethicone as Dow Corning® 593 fluid. Also useful herein are dimethiconols, which are hydroxy terminated dimethyl silicones. These materials can be represented by the general chemical formulas $R_3SiO[R_2SiO]_xSiR_2OH$ and $HOR_2SiO[R_2SiO]_xSiR_2OH$ wherein R is an alkyl group (preferably R is methyl or ethyl, more preferably methyl) and x is an integer up to about 500, chosen to achieve the desired molecular weight. Commercially available dimethiconols are typically sold as mixtures with dimethicone or cyclomethicone (e.g. Dow Corning® 1401, 1402, and 1403 fluids). Also useful herein are polyalkylaryl siloxanes, with polymethylphenyl siloxanes having viscosities from about 15 to about 65 centistokes at 25°C being preferred. These materials are available, for example, as SF 1075 methylphenyl fluid (sold by General Electric Company) and 556 Cosmetic Grade phenyl trimethicone fluid (sold by Dow Coming Corporation).

Vegetable oils and hydrogenated vegetable oils are also useful herein. Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated sesame oil, hydrogenated sunflower seed oil, and mixtures thereof.

Also useful are C4-C20 alkyl ethers of polypropylene glycols, C1-C20 carboxylic acid esters of polypropylene glycols, and di-C8-C30 alkyl ethers. Nonlimiting examples of these materials include PPG-14 butyl ether, PPG-15 stearyl ether,, dioctyl ether, dodecyl octyl ether, and mixtures thereof.

B. Water Soluble Conditioning Agents

Nonlimiting examples of conditioning agents useful as water soluble conditioning agents include those selected from the group consisting of polyhydric alcohols, polypropylene glycols, polyethylene glycols, ureas, pyrolidone carboxylic acids, ethoxylated and/or propoxylated C3-C6 diols and triols, alpha-hydroxy C2-C6 carboxylic acids, ethoxylated and/or propoxylated sugars, polyacrylic acid copolymers, sugars having up to about 12 carbons atoms, sugar alcohols having up to about 12 carbon atoms, and mixtures thereof. Specific examples of useful water soluble conditioning agents include materials such as urea; guanidine; glycolic acid and glycolate salts (e.g. ammonium and quaternary alkyl ammonium); lactic acid and lactate salts (e.g. ammonium and quaternary alkyl ammonium); sucrose, fructose, glucose, eruthrose, erythritol, sorbitol, mannitol, glycerol, hexanetriol, propylene glycol, butylene glycol, hexylene glycol, and the like; polyethylene glycols such as PEG-2, PEG-3, PEG-30, PEG-50, polypropylene glycols such as PPG-9, PPG-12, PPG-15, PPG-17, PPG-20, PPG-26, PPG-30, PPG-34; alkoxylated glucose; hyaluronic acid; and mixtures thereof. Also useful are materials such as aloe vera in any of its variety of forms (e.g., aloe vera gel), chitin, starch-grafted sodium polyacrylates such as Sanwet (RTM) IM-1000, IM-1500, and IM-2500 (available from Celanese Superabsorbent Materials, Portsmouth, VA); lactamide monoethanolamine; acetamide monoethanolamine; and mixtures thereof. Also useful are propoxylated glycerols as described in propoxylated glycerols described in U.S. Patent No. 4,976,953, to Orr et al., issued December 11, 1990.

C. Conditioning Emulsions

The conditioning component of the present invention may also comprise a conditioning emulsion which is useful for providing a conditioning benefit to the skin or hair during the use of the article. The term "conditioning emulsion" as used herein means the combination of an internal phase comprising a water soluble conditioning agent that is enveloped by an external phase comprising an oil soluble agent. In preferred embodiments, the conditioning emulsion would further comprise an emulsifier. The conditioning emulsion comprises from about 0.25% to about 150%, preferably from about 0.5% to about 100%, and more preferably from about 1% to about 50% by weight of said water insoluble substrate. By a conditioning emulsion is meant a combination of an internal phase comprising a water soluble conditioning agent that is enveloped by an external phase comprising an oil soluble agent. In preferred embodiments, the conditioning emulsion would further comprise an emulsifier.

The conditioning emulsion comprises (i) an internal phase comprising water soluble conditioning agents as described above, and (ii) an external phase comprising oil soluble agents (e.g., conditioning and non-conditioning agents) as described hereinbefore and hereinafter. In further embodiments, the conditioning emulsion further comprises an emulsifier capable of forming an emulsion of said internal and external phases. Although an emulsifier capable of forming an emulsion of the internal and external phases is preferred in the present invention, it is recognized in the art of skin care formulations that a water soluble conditioning agent can be enveloped by an oil soluble agent without an emulsifier. As long as the water soluble conditioning agent is enveloped by the oil soluble agent, thereby protected

from being rinsed away during the cleansing process, the composition would be within the scope of the present invention.

The internal phase can optionally comprise other water-soluble or dispersible materials that do not adversely affect the stability of the conditioning emulsion. One such material is a water-soluble electrolyte. The dissolved electrolyte minimizes the tendency of materials present in the lipid phase to also dissolve in the water phase. Any electrolyte capable of imparting ionic strength to the internal phase can be used. Suitable electrolytes include the water soluble mono-, di- or trivalent inorganic salts such as water-soluble halides, e.g., chlorides, nitrates and sulfates of alkali metals and alkaline earth metals. Examples of such electrolytes include sodium chloride, calcium chloride, sodium sulfate, magnesium sulfate, and sodium bicarbonate. The electrolyte will typically be included in a concentration in the range of from about 1 to about 20% of the internal phase.

Other water-soluble or dispersible materials that can be present in the internal phase include thickeners and viscosity modifiers. Suitable thickeners and viscosity modifiers include water-soluble polyacrylic and hydrophobically modified polyacrylic resins such as Carbopol and Pemulen, starches such as corn starch, potato starch, tapioca, gums such as guar gum, gum arabic, cellulose ethers such as hydroxypropyl cellulose, hydroxyethyl cellulose, carboxymethyl cellulose, and the like. These thickeners and viscosity modifiers will typically be included in a concentration in the range of from about 0.05 to about 0.5% of the internal phase.

Other water soluble or dispersible materials that can be present in the internal water phase include polycationic polymers to provide steric stabilization at the water-lipid interface and nonionic polymers that also stabilize the water-in-lipid-emulsion. Suitable polycationic polymers include Reten 201, Kymene 557H® and Acco 7112. Suitable nonionic polymers include polyethylene glycols (PEG) such as Carbowax. These polycationic and nonionic polymers will typically be included in a concentration in the range of from about 0.1 to about 1.0% of the internal phase.

Preferred embodiments of the present invention which contain conditioning emulsions comprise an emulsifier capable of forming an emulsion of the internal and external phases. In the emulsions of the present invention, the emulsifier is included in an effective amount. What constitutes an "effective amount" will depend on a number of factors including the respective amounts of the oil soluble agents, the type of emulsifier used, the level of impurities present in the emulsifier, and like factors. Typically, the emulsifier comprises from about 0.1% to about 20%, preferably from about 1% to about 10%, and more preferably from about 3% to about 6% by weight of the conditioning emulsion.

The emulsifiers useful in the present invention typically are oil soluble or miscible with the oil soluble external phase materials, especially at the temperature at which the lipid material melts. It also should have a relatively low HLB value. Emulsifiers suitable for use in the present invention have HLB values typically in the range of from about 1 to about 7 and can include mixtures of different emulsifiers. Preferably, these emulsifiers will have HLB values from about 1.5 to about 6, and more preferably from about 2 to about 5.

A wide variety of emulsifiers are useful herein and include, but not limited to, those selected from the group consisting of sorbitan esters, glyceryl esters, polyglyceryl esters, methyl glucose esters, sucrose esters, ethoxylated fatty alcohols, hydrogenated castor oil ethoxylates, sorbitan ester ethoxylates, polymeric emulsifiers, and silicone emulsifiers.

Sorbitan esters are useful in the present invention. Preferable are sorbitan esters of C16-C22 saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN® 80), sorbitan sesquioleate (e.g., Arlacel® 83), sorbitan monoisostearate (e.g., CRILL® 6 made by Croda), sorbitan stearates (e.g., SPAN® 60), sorbitan triooleate (e.g., SPAN® 85), sorbitan tristearate (e.g., SPAN® 65), sorbitan dipalmitates (e.g., SPAN® 40), and sorbitan isostearate. Sorbitan monoisostearate and sorbitan sesquioleate are particularly preferred emulsifiers for use in the present invention.

Other suitable emulsifiers for use in the present invention include, but is not limited to, glyceryl monoesters, preferably glyceryl monoesters of C16-C22 saturated, unsaturated and branched chain fatty acids such as glyceryl oleate, glyceryl monostearate, glyceryl monopalmitate, glyceryl monobehenate, and mixtures thereof; polyglyceryl esters of C16-C22 saturated, unsaturated and branched chain fatty acids, such as polyglyceryl-4 isostearate, polyglyceryl-3 oleate, diglycerol monooleate, tetraglycerol monooleate and mixtures thereof; methyl glucose esters, preferably methyl glucose esters of C16-C22 saturated, unsaturated and branched chain fatty acids such as methyl glucose dioleate, methyl glucose sesquiisostearate, and mixtures thereof; sucrose fatty acid esters, preferably sucrose esters of C12-C22 saturated, unsaturated and branched chain fatty acids such as sucrose stearate, sucrose trilaurate, sucrose distearate (e.g., Crodesta® F10), and mixtures thereof; C12-C22 ethoxylated fatty alcohols such as oleth-2, oleth-3, steareth-2, and mixtures thereof; hydrogenated castor oil ethoxylates such as PEG-7 hydrogenated castor oil; sorbitan ester ethoxylates such as PEG-40 sorbitan peroleate, Polysorbate-80, and mixtures thereof; polymeric emulsifiers such as ethoxylated dodecyl glycol copolymer; and silicone emulsifiers such as laurylmethicone copolyol, cetyldimethicone, dimethicone copolyol, and mixtures thereof.

In addition to these primary emulsifiers, the compositions of the present invention can optionally contain a coemulsifier to provide additional water-lipid emulsion stability. Suitable coemulsifiers include, but is not limited to,

phosphatidyl cholines and phosphatidyl choline-containing compositions such as lecithins; long chain C16-C22 fatty acid salts such as sodium stearate; long chain C16-C22 dialiphatic, short chain C1-C4 dialiphatic quaternary ammonium salts such as ditallow dimethyl ammonium chloride and ditallow dimethyl ammonium methylsulfate; long chain C16-C22 dialkoyl(alkenoyl)-2-hydroxyethyl, short chain C1-C4 dialiphatic quaternary ammonium salts such as ditallowoyl-2-hydroxyethyl dimethyl ammonium chloride; the long chain C16-C22 dialiphatic imidazolinium quaternary ammonium salts such as methyl-1-tallow amido ethyl-2-tallow imidazolinium methylsulfate and methyl-1-oleyl amido ethyl-2-oleyl imidazolinium methylsulfate; short chain C1-C4 dialiphatic, long chain C16-C22 monoaliphatic benzyl quaternary ammonium salts such as dimethyl stearyl benzyl ammonium chloride, and synthetic phospholipids such as stearamidopropyl PG-dimonium chloride (Phospholipid PTS from Mona Industries).

D. Lipid Hardness

In emobidments further comprising a conditioning component, the article can preferably have a minimum lipid hardness value of about 0.02 kg. Consumer habits in using disposable articles having two surfaces vary considerably. When preparing to use such articles, consumers will generally wet the article and then "lather" it before contacting the article with their skin or hair. "Lathering" is achieved by rubbing the surfaces of the article on or against each other prior to use of the article. If the surface containing the conditioning agents is first used in lathering and the same surface is then used to contact the skin or hair, deposition of the conditioning agents is considerably decreased due to emulsification of the conditioning agents by the surfactant. If, however, the surface not containing the conditioning agents (e.g., a surfactant-containing surface) is rubbed together to produce the lather and the surface containing the conditioning agents is then used to contact the skin or hair, maximum deposition of conditioning agents is achieved. If both surfaces of the article are treated with the conditioning agents, the same inconsistent deposition can result. Maximum deposition of conditioning agents would result only if a non-lathered surface containing conditioning agents is contacted with the skin or hair.

It has been surprisingly found that if the conditioning component (the combination of the conditioning agents) has a minimum lipid hardness value of 0.02 kg., this inconsistent deposition of conditioning agents is considerably diminished. It is believed that increasing conditioning component hardness decreases transfer within the substrate and also decreases emulsification of the conditioning agents by the surfactants during the lathering step. As a result, more of the conditioning agents remain available for mechanical transfer via contact with the skin or hair.

The lipid hardness value is a physical hardness measurement of the combination of all conditioning agents within the conditioning component. It is believed that increasing the lipid hardness value increases deposition consistency of the conditioning agents despite variations in lathering techniques employed by the consumer. It is believed that increasing conditioning component hardness decreases transfer within the substrate and also decreases emulsification of the conditioning agents by the surfactants during the lathering step. As a result, more of the conditioning agents remain available for mechanical transfer via contact with the skin or hair.

The conditioning component of the present invention has a lipid hardness value of greater than about 0.02 kg, preferably greater than about 0.05, and more preferably greater than about 0.10. Preferably, the lipid hardness value of the conditioning component should not be greater than about 5.00 kg., more preferably about 4.00 kg, most preferably 3.00, because hardness levels beyond this point can negatively affect deposition of the conditioning agents in the conditioning component to the skin or hair.

Lipid Hardness Test: The lipid hardness value is measured by a test traditionally used to measure bar soap hardness. A Chatillon force gauge is employed to measure the hardness value of a 0.14 - 0.24 kg (5-8 oz). sample of the conditioning component. Several readings are taken, each on a fresh sample, to obtain an average value. The Chatillon force gauge model no. DFIS100 is manufactured by Chatillon Corporation which is located in Greensboro, North Carolina.

Materials Used to Increase Lipid Hardness Value: The cleansing and conditioning articles of the present invention may comprise a hardening material used in combination with the conditioning agents comprising the conditioning component described hereinbefore. Many materials can be used as both a conditioning agent and as a lipid hardening material. In fact, any solid conditioning agent, described hereinbefore, may be used as a lipid hardening material. The amount of the hardening material needed to achieve the minimum lipid hardness value of 0.02 kg. is dependent upon the particular material used and can be easily determined by one of ordinary skill in the art. The hardening material can be used as an individual hardening material or a combination of hardening materials, and is included at concentrations ranging from about 0.1% to about 99.9%, preferably from about 0.5% to about 75%, more preferably from about 1% to about 50%, even more preferably from about 2% to about 25%, by weight of the conditioning component.

As used herein the term "hardening materials" refers to those materials which have a melting point above about 30°C, preferably above about 30°C to about 250° C, more preferably from about 37°C to about 100°C, even more preferably from about 37°C to about 80°C.

Any material may be used to increase the lipid hardness value of the conditioning component provided that the following criteria are met: (i) the material must be soluble in the conditioning agents of the conditioning component and (ii) the material must have a melting point of greater than 20° C (e.g., be a solid at room temperature). Examples of suitable hardening materials include, but are not limited to, petrolatum, highly branched hydrocarbons, fatty alcohols, fatty acid esters, vegetable oils, hydrogenated vegetable oils, polypropylene glycols, alpha-hydroxy fatty acids, fatty acids having from about 10 to about 40 carbon atoms, alkyl amides of di and/or tri-basic carboxylic acids, n-acyl amino acid derivatives, and mixtures thereof. Hardening materials useful in the present invention are further described in U. S. Patent No. 4,919,934, to Deckner et al., issued April 24 1990.

Suitable highly branched hydrocarbons for use herein include hydrocarbon compounds having from about 17 to about 40 carbon atoms. Nonlimiting examples of these hydrocarbon compounds include squalane, cholesterol, lanolin, docosane (i.e. a $C_{22}$ hydrocarbon), and isoparaffins.

Suitable fatty alcohols for use herein include monohydric alcohols, ethoxylated fatty alcohols, and fatty alcohol esters, excluding the ethoxylated fatty alcohols and fatty alcohol esters useful as emulsifiers herein. Specific examples of commercially available fatty alcohols include, but are not limited to, Unilin 550, Unilin 700, Unilin 425, Unilin 400, Unilin 350, and Unilin 325, all supplied by Petrolite. Suitable ethoxylated fatty alcohols include, but are not limited, Unithox 325, Unithox 400, and Unithox 450, Unithox 480, Unithox 520, Unithox 550, Unithox 720, Unithox 750, all of which are available from Petrolite. Non-limiting examples of suitable esters of fatty alcohols include tri-isostearyl citrate, ethyleneglycol di-12-hydroxystearate, tristearylcitrate, stearyl octanoate, stearyl heptanoate, trilaurylcitrate.

Suitable fatty acid esters for use herein include ester waxes, monoglycerides, diglycerides, triglycerides and mixtures thereof. Non-limiting examples of suitable ester waxes include stearyl stearate, stearyl behenate, palmityl stearate, stearyl octyldodecanol, cetyl esters, cetearyl behenate, behenyl behenate, ethylene glycol distearate, ethylene glycol dipalmitate, and beeswax. Examples of commercial ester waxes include Kester waxes from Koster Keunen, Crodamol SS from Croda and Demalcare SPS from Rhone Poulenc.

Vegetable oils and hydrogenated vegetable oils which are solid or semi-solid at ambient temperatures of from about 20°C to about 25°C are also useful herein as hardening materials. Examples of suitable vegetable oils and hydrogenated vegetable oils include butterfat, chicken fat, goose fat, horse fat, lard (fatty tissue) oil, rabbit fat, sardine oil, tallow (beef), tallow (mutton), chinese vegetable tallow, babassu oil, cocoa butter, coconut oil, palm oil, palm kernal oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated sesame oil, hydrogenated sunflower seed oil, derivatives thereof and mixtures thereof.

Suitable polypropylene glycols for use herein include $C_4$-$C_{16}$ alkyl ethers of polypropylene glycols, and $C_1$-$C_{16}$ carboxylic acid esters of polypropylene glycols. Nonlimiting examples of these materials include PPG-14 butyl ether, PPG-15 stearyl ether, PPG-9, PPG-12, PPG-15, PPG-17, PPG-20, PPG-26, PPG-30, PPG-34, and mixtures thereof.

Examples of suitable alpha-hydroxy fatty acids and fatty acids having from about 10 to about 40 carbon atoms include 12-hydroxystearic acid, 12-hydroxylauric acid, 16-hydroxyhexadecanoic acid, behenic acid, eurcic acid, stearic acid, caprylic acid, lauric acid, isostearic acid, and mixtures thereof. Examples of some suitable fatty acids are further described in U.S. Patent 5,429,816, issued to Hofrichter et al. on July 4,1995; and U.S. Patent 5,552,136, issued to Motley on September 3, 1996.

Suitable alkyl amides of di and/or tri-basic carboxylic acids for use herein include disubstituted or branched monoamides, monosubstituted or branched diamides, triamides, and mixtures thereof. Some specific examples of alkyl amides of di- and tri-basic carboxylic acids include, but are not limited to, alkyl amides of citric acid, tricarballylic acid, aconitic acid, nitrilotriacetic acid and itaconic acid such as 1,2,3-propane tributylamide, 2-hydroxy-1,2,3-propane tributylamide, 1-propene-1,2,3-trioctylamide, N,N',N"-tri(methyldecylamide)amine, 2 docecyl-N,N'-dibutylsuccinamide, and mixtures thereof. Other suitable amides include the n-acyl amino acid derivatives described in U.S. Patent 5,429,816, issued to Hofrichter et al. on July 4, 1995.

Also suitable for use in the present invention are waxes having a HLB of from about 1 to about 10, preferably from about 6 and most preferably from about 5. The HLB (short for "Hydrophile-Lipophile Balance") value system is fully described, and values for various materials are provided, in the publication *The Time-Saving Guide to Emulsifier Selection* (published by ICI Americas Inc., Wilmington, Del.; 1984).

Useful ester waxes include $C_{10}$-$C_{40}$ fatty acid, diesters, of $C_{10}$-$C_{40}$ fatty acids where the alcohol is propylene glycol, ethylene glycol, polyethylene glycol, polypropylene glycol, polyglycerin, or glycerin, triglycerides or diglycerides of $C_{10}$-$C_{40}$ fatty acids, pentaerythritol tri- ortetra- esters of $C_{10}$-$C_{40}$ fatty acids, $C_{10}$-$C_{40}$ fatty acids of sorbitan triesters, $C_{10}$-$C_{40}$ fatty acids of sucrose polyesters having 3-8 moles of substitution, myristyl myristate, paraffin, synthetic waxes such as Fischer-Tropsche waxes, microcrystalline waxes, castor wax, partially hydrogenated vegetable oils, behenyl behenrate and myristyl propionate and mixtures thereof.

Useful diester waxes include Synchrowax ERL-C (C18-36 acid glycolester) (available from Croda) and propylene glycol diester waxes including ethylene glycol distearate and glycol distearate. Useful triglyceride waxes include

Shea Butter, Cocoa Butter, Synchrowax HGL-C (C18-36 acid triglyceride), Synchrowax HRC (tribehenin), Synchrowax HRS-C [tribehenin (and) calcium behenate] (all available from Croda Inc.), Tristearin, trimyristate and fully hydrogenated vegetable oils and mixtures thereof. Preferred is a mixture of diester and triglyceride waxes in a ratio of from about 5:1 to about 1:1 and more preferably from about 4:1 to about 1:1.

Waxes useful in the compositions of this invention are disclosed in the following: U.S. Pat No. 5,219,558 to Woodin, Jr. et al., issued June 15, 1993; U.S. Pat. No. 4,049,792, to Elsnau, issued Sept. 20, 1977; U.S. Pat. No. 4,151,272, to Geary et al., issued Apr. 24, 1975; U.S. Pat. No. 4,229,432, to Geria, issued Oct. 21, 1980; U.S. Pat No. 4,280,994, to Turney, issued Jul. 28, 1981; U.S. Pat. No. 4,126,679, to Davy et al., issued Nov. 21, 1978; and European Patent Application Publication Number 117,070 to May, published Aug. 29, 1984, "The Chemistry and Technology of Waxes", A. H. Warth, 2nd Edition, reprinted in 1960, Reinhold Publishing Corporation, pp. 391-393 and 421; "The Petroleum Chemicals Industry", R. F. Goldstein and A. L. Waddeam, 3rd Edition (1967), E &F.N. Span Ltd., pp. 33-40; "The Chemistry and Manufacture of Cosmetics", M. G. DeNavarre, 2nd Edition (1970), Van Nostrand & Company, pp. 354-376; and in "Encyclopedia of Chemical Technology:, Vol. 24, Kirk-Othmer, 3rd Edition (1979) pp. 466-481.

Additional non-limiting examples of useful hardening materials are those selected from the group consisting of sorbitan esters, glyceryl esters, polyglyceryl esters, methyl glucose esters, sucrose esters, ethoxylated fatty alcohols, hydrogenated castor oil ethoxylates, sorbitan ester ethoxylates, polymeric emulsifiers, and silicone emulsifiers.

Sorbitan esters are useful in the present invention. Preferable are sorbitan esters of C16-C22 saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN® 80), sorbitan sesquioleate (e.g., Arlacel® 83), sorbitan monoisostearate (e.g., CRILL® 6 made by Croda), sorbitan stearates (e.g., SPAN® 60), sorbitan triooleate (e.g., SPAN® 85), sorbitan tristearate (e.g., SPAN® 65), sorbitan dipalmitates (e.g., SPAN® 40), and sorbitan isostearate. Sorbitan monoisostearate and sorbitan sesquioleate are particularly preferred emulsifiers for use in the present invention.

Other suitable hardeners for use in the present invention include, but is not limited to, glyceryl monoesters, preferably glyceryl monoesters of C16-C22 saturated, unsaturated and branched chain fatty acids such as glyceryl oleate, glyceryl monostearate, glyceryl monopalmitate, glyceryl monobehenate, and mixtures thereof; polyglyceryl esters of C16-C22 saturated, unsaturated and branched chain fatty acids, such as polyglyceryl-4 isostearate, polyglyceryl-3 oleate, diglycerol monooleate, tetraglycerol monooleate and mixtures thereof; methyl glucose esters, preferably methyl glucose esters of C16-C22 saturated, unsaturated and branched chain fatty acids such as methyl glucose dioleate, methyl glucose sesquiisostearate, and mixtures thereof; sucrose fatty acid esters, preferably sucrose esters of C12-C22 saturated, unsaturated and branched chain fatty acids such as sucrose stearate, sucrose trilaurate, sucrose distearate (e.g., Crodesta® F10), and mixtures thereof; C12-C22 ethoxylated fatty alcohols such as oleth-2, oleth-3, steareth-2, and mixtures thereof; hydrogenated castor oil ethoxylates such as PEG-7 hydrogenated castor oil; sorbitan ester ethoxylates such as PEG-40 sorbitan peroleate, Polysorbate-80, and mixtures thereof; polymeric emulsifiers such as ethoxylated dodecyl glycol copolymer; and silicone emulsifiers such as laurylmethicone copolyol, cetyldimethicone, dimethicone copolyol, and mixtures thereof.

Other useful hardeners include, but is not limited to, phosphatidyl cholines and phosphatidyl choline-containing compositions such as lecithins; long chain C16-C22 fatty acid salts such as sodium stearate; long chain C16-C22 dialiphatic, short chain C1-C4 dialiphatic quaternary ammonium salts such as ditallow dimethyl ammonium chloride and ditallow dimethyl ammonium methylsulfate; long chain C16-C22 dialkoyl(alkenoyl)-2-hydroxyethyl, short chain C1-C4 dialiphatic quaternary ammonium salts such as ditallowoyl-2-hydroxyethyl dimethyl ammonium chloride; the long chain C16-C22 dialiphatic imidazolinium quaternary ammonium salts such as methyl-1-tallow amido ethyl-2-tallow imidazolinium methylsulfate and methyl-1-oleyl amido ethyl-2-oleyl imidazolinium methylsulfate; short chain C1-C4 dialiphatic, long chain C16-C22 monoaliphatic benzyl quaternary ammonium salts such as dimethyl stearyl benzyl ammonium chloride, and synthetic phospholipids such as stearamidopropyl PG-dimonium chloride (Phospholipid PTS from Mona Industries).

## V. WEIGHT RATIOS AND WEIGHT PERCENTAGES

In the present invention, the weight ratio of the lathering surfactant to the conditioning component is less than about 40:7, preferably less than about 5:1, more preferably less than about 2.5:1, and more preferably less than about 1:1.

In certain embodiments of the present invention, the cleansing and conditioning component, which is defined as comprising a lathering surfactant and a conditioning component further comprising an oil soluble conditioning agent and a water soluble conditioning agent, the lathering surfactant comprises from about 1% to about 75%, preferably from about 10% to about 65%, and more preferably from about 15% to about 45%, by weight of the cleansing and conditioning component, and the conditioning component comprises from about 15% to about 99%, preferably from about 20% to about 75%, and more preferably from about 25% to about 55%, by weight of the cleansing and conditioning

component.

## VI. SURFACE TO SATURATION RATIO

### A. Method to Measure Surface Application of Conditioning Agents

The products of the present invention can preferably have the conditioning agent substantially on the surface of the substrate. By "substantially on the surface of the substrate" is meant that the surface to saturation ratio is greater than about 1.25, preferably greater than about 1.50, more preferably greater than about 2.00, even more preferably greater than about 2.70, and most preferably 3.00. The surface to saturation ratio is a ratio of the measurement of conditioning agent on the surface of the substrate versus the measurement of the conditioning agent within the substrate. These measurements are obtained from Attenuated Total Reflectance (ATR) FT-IR Spectroscopy the use of which is well known to one skilled in the art of analytical chemistry. The same method can be applied to measure the combination of conditioning agent and active ingredients.

The procedure to obtain the measurements are as follows:

Instrumental Setup: A BioRad FTS-7 spectrometer, manufactured by Bio Rad Labs, Digital Laboratory Division, located in Cambridge, MA, is used to collect the infrared spectra. Typically, the measurements consist of 100 scans at 4 $cm^{-1}$ resolution. The collection optics consist of a flat 60 deg ZnSe ATR crystal, manufactured by Graseby Specac, Inc., located in Fairfield, CT. Data is collected at 25°C and analyzed using Grams 386 software, distributed by Galactic Industries Corp., located in Salem, New Hampshire. Prior to measurement the crystal is cleaned with a suitable solvent. The sample is placed onto the ATR crystal and held under constant 4.53 kg (10 lb) weight.

Experimental Procedure:

(1) Measure the reference (background) spectrum: First clean the ATR cell with a suitable solvent (e.g., isopropyl alcohol). Then air dry the ATR cell. Next measure the background spectrum (typically 100 scans @ 4 $cm^{-1}$ resolution).
(2) Place substrate on top of ATR crystal: First lay the substrate flat on the measuring platform. Then place a 4.53 kg (10 lb). weight on top of the substrate. Then, measure the spectrum (typically 100 scans @ 4 $cm^{-1}$ resolution). The substrate acts as an internal standard because the absorbency of the substrate alone can be identified.
(3) Analyze spectrum for conditioning ingredients by first identifying absorbance due to substrate and measuring peak height. Then, identify the absorbance peaks due to skin conditioning agent and measure peak height. The following contain some examples:

| Substrate | substrate peak ht. | Conditioner | conditioner peak ht. | ratio |
|---|---|---|---|---|
| Dupont 8868[1] | 0.21 | petrolatum[3] | 0.76 | 3.61 |
| Fibrella F310062[2] | 0.37 | glycerin[4] | 0.52 | 1.41 |
| Fibrella F310062[2] | 0.37 | petrolatum[3] | 1.21 | 3.27 |

[1] polyester: C=O stretching mode at 1710 $cm^{-1}$
[2] polypropylene: C-H streatching mode at 2822 $cm^{-1}$
[3] petrolatum: C-H stretching mode at 2923 $cm^{-1}$.
[4] glycerin: C-O stretching mode at 1030 $cm^{-1}$

Obtaining the surface to saturation ratio:

(1) If the ratio of conditioning agent absorbance to substrate absorbance is ≥ 1.25, then the conditioning agent is substantially on the surface of the substrate. This is because the FT-IR absorbance reading measures the amount of conditioning agent up to 7 microns into the substrate.
(2) If the ratio of the conditioning agent absorbance to substrate absorbance is < 1.25 then the conditioning agent is substantially not on the surface.

B. Methods of Maintaining the Conditioning Component Substantially on the Surface of the Substrate

It has been found that certain process and compositional improvements greatly enhance the efficiency and effectiveness of delivering conditioning agents to the skin or hair. These process and compositional improvements allow the same or better effect from the conditioning component at lower levels by maintaining the conditioning component on the surface of the substrate.

The products of the present invention effectively and efficiently deliver conditioning agents to the skin and hair by maintaining the conditioning component, comprised of the conditioning agents, substantially on the surface of the substrate. The following subsections discuss in further detail the processes and compositional improvements which allow the a surface to saturation ratio of greater than or equal to about 1.25. All of the following processing and compositional improvements can be used individually or in combination to maintain the conditioning agent substantially on the surface. The term "chemical component," as used herein, means the conditioning agent or a combination of the conditioning agent and the active ingredient.

Chemical Treatment of the Substrate: One method of substantially maintaining the chemical component on the surface of the substrate is by chemically treating the substrate or the fibers of the substrate with either a hydrophobic or hydrophilic substance. Choosing the appropriate substance (hydrophobic or hydrophilic) is dependent on the chemical component that is meant to be deposited. For example, if a oil soluble conditioning agent is to be deposited onto the skin or hair, the substrate or its fibers would typically be treated with a hydrophilic substance, and vice versa. Because most substrates are hydrophobic by their nature, e.g., usually derived from polyolefins, this section will concentrate on hydrophilic chemical treatment of the substrate.

Any of a wide variety of surfactants, including ionic and nonionic surfactants, may be employed to hydrophilically modify the substrate. Suitable surfactants may be internal modifiers, e.g., the modifying compounds are added to the polymer composition prior to spinning or forming fibers, or topical modifiers, e.g., the modifying compounds are topically applied during or subsequent to the formation of fibers or nonwoven webs. An internal modification process is disclosed in U.S. Patent No. 4,578,414 to Sawyer et al, and a topical modification process is disclosed in U.S. Patent No. 5,057,361 to Sayovitz et al..

Nonlimiting examples of suitable surfactants include silicone based surfactants, e.g., polyalkelene-oxide modified polydimethyl siloxane; fluoroaliphatic surfactants, e.g., perfluoroalkyl polyalkylene oxides; and other surfactants, e.g., actyl-phenoxypolyethoxy ethanol nonionic surfactants, alkylaryl polyether alcohols, and polyethylene oxides. Commercially available surfactants suitable for the present invention include various poly(ethylene oxide) based surfactants available under the tradename Triton, e.g., grade X-102, from Rohm and Haas Corp.; various polyethylene glycol based surfactants available under the tradename Emerest, e.g., grades 2620 and 2650, from Emery Indust.; various polyalkylene oxide modified polydimethylsiloxane based surfactants available under the tradename Silwet, e.g., grade Y12488, from OSI Specialty Chemicals; and alkenyl succinamide surfactants available under the tradename Lubrizol, e.g., grade OS85870, from Lubrizol Corp.; and polyoxyalkylene modified fluoroaliphatic surfactants available from Minnesota Mining and Manufacturing Co. The amount of surfactants required and the hydrophilicity of the modified substrate or fibers of the substrate for each application will vary depending on the type of surfactant selected and the component polymers used. In general, the surfactant may be added, topically or internally, in the range of from about 0.1 to about 5%, preferably from about 0.3% to about 4%, by weight of the substrate or the fibers of the substrate.

Increasing Viscosity: Another method of substantially maintaining the chemical component on the surface of the substrate is by increasing the viscosity before application onto the substrate. This prevents the saturation of the substrate with the chemical component. Generally there are two methods for increasing the viscosity of the chemical component: (i) application onto the substrate at the transition temperature of the chemical component; and (ii) introducing a thickener to the chemical component mixture before application onto the substrate. A combination of these methods is preferable.

Phase transition temperature application to the substrate:. One method of maintaining the chemical component on the surface of the substrate is to apply the chemical component to the substrate at the phase transition temperature of the chemical component. This method can be employed with any chemical component wherein the phase transition temperature of the chemical component is above about 35°C (e.g., viscous at room temperature). Phase transition temperature is defined, as used herein, as the temperature at which the chemical component transforms from a fluid, liquid state to a viscous state. In essence, this method applies the chemical component at the temperature at which the chemical component becomes viscous from a fluid liquid state during the cooling process.

Typically, the chemical component is applied onto the substrate by melting or heating. Alternatively, the chemical component can be heated and dissolved into a solvent before application to the substrate. However, some chemical components may be viscous yet fluid enough to be applied without heating. If a chemical component has a transition temperature at about room temperature or slightly above room temperature the other methods within this section must be employed to maintain the chemical component on the surface of the substrate. The transition temperatures (also known as melting point) of most chemicals may be easily obtained from the Merck Index, Tenth Edition (1983) and the

CTFA Cosmetic Ingredient Handbook, Second Edition, (1992).

A corollary to transition temperature application to the substrate is supercooling the chemical component upon application to the substrate. By supercooling is meant that the cooling rate is artificially increase above the normal ambient temperature cooling rate. This provides the dual benefit of having fluidity of the chemical component during processing yet reaching the phase transition temperature before the substrate is saturated by the chemical component. This method would be used when a chemical component is viscous and plastic at room temperature.

Thickening Agent: If the chemical component is a liquid at room temperature (e.g., not viscous), the chemical component will not remain primarily on the surface of the substrate. Instead, the chemical component will tend to migrate and flow into the void volume of the substrate. The present method provides a solution by introducing a thickening agent into the chemical component. This increases the viscosity of the chemical component thereby achieving an equivalent result as phase transition temperature application to the substrate. Because the viscosity of the chemical component is effectively increased, it remains substantially on the surface of the substrate without saturating the substrate. Generally, the thickening agent must be viscous at room temperature, and it must be miscible in the chemical component. Phase transition temperatures and suitable viscosities of thickening agent will vary drastically upon the particular thickener. However, typically, the phase transition temperature of the thickening agent must be greater than about 35°C, preferably greater than about 40°C.

Generally, anything that is viscous at room temperature can be a thickener. The CTFA Cosmetic Ingredient Handbook, Second Edition, (1992), discloses many appropriate thickeners. In fact, any conditioning agent, disclosed above, that is more viscous than the chemical component and is miscible in the chemical component can be an appropriate thickener.

Nonlimiting examples of useful thickening agents of the present invention are selected from the group consisting of fatty alcohols, fatty acids, fatty alcohol ethoxylates having an average degree of ethoxylation ranging from 2 to about 30, sorbitan esters, glyceryl esters, polyglyceryl esters, methyl glucose esters, sucrose esters, sorbitan ester ethoxylates, natural and synthetic waxes, polyacrylic and hydrophobically modified polyacrylic resins, starches, gums, cellulose ethers, polycationic polymers, nonionic polymers, polyethylene glycols (PEG), and mixtures thereof.

Nonlimiting examples of useful thickening agents in the present invention include stearic acid, behenic acid, stearyl alcohol, cetyl alcohol, sorbitan monooleate, sorbitan sesquioleate, sorbitan monoisostearate, sorbitan stearates, sorbitan triooleate, sorbitan tristearate, sorbitan dipalmitates, sorbitan isostearate, glyceryl oleate, glyceryl monostearate, glyceryl monopalmitate, glyceryl monobehenate, polyglyceryl-4 isostearate, polyglyceryl-3 oleate, diglycerol monooleate, tetraglycerol monooleate, methyl glucose dioleate, methyl glucose sesquiisostearate, sucrose stearate, sucrose trilaurate, sucrose distearate oleth-2, oleth-3, steareth-2, PEG-40 sorbitan peroleate, Polysorbate-80, beeswax, polyethylene wax, Carbopol, Pemulen, corn starch, potato starch, tapioca, guar gum, gum arabic, hydroxypropyl cellulose, hydroxyethyl cellulose, carboxymethyl cellulose, Reten 201, Kymene 557H®, Acco 7112, Carbowax.

Nonuniform Application to the Substrate: Another method of substantially maintaining the chemical component on the surface of the substrate is by applying the chemical component nonuniformly to the surface of the substrate. By "nonuniform" is meant that the amount, pattern of distribution, etc. of the chemical component can vary over the surface of the substrate. For example, some portions of the surface of the substrate can have greater or lesser amounts of the chemical component, including portions of the surface that do not have any chemical component.

Order of Application of Ingredients to the Substrate: Another method of substantially maintaining the chemical component on the surface of the substrate is by determining the order of application of ingredients to the substrate. Generally, the best results are obtained when the chemical component is added onto a dry substrate. Thus, applying the lathering surfactant first, and then drying the surfactant treated substrate before application of the chemical component will greatly enhance the delivery of the chemical component.

## VII. ADDITIONAL INGREDIENTS

The articles of the present invention can comprise a wide range of optional ingredients. Some of these ingredients are listed in more detail herein. Particularly useful are various active ingredients useful for delivering various non-conditioning or non-cleansing benefits of the skin or hair during the cleansing and conditioning process. In these compositions, the article is useful for delivering the active ingredient to the skin or hair.

### A. Active Ingredients

The compositions of the present invention can comprise a safe and effective amount of one or more active ingredients or pharmaceutically-acceptable salts thereof.

The term "safe and effective amount" as used herein, means an amount of an active ingredient high enough to modify the condition to be treated or to deliver the desired skin benefit, but low enough to avoid serious side effects, at a reasonable benefit to risk ratio within the scope of sound medical judgment. What is a safe and effective amount

of the active ingredient will vary with the specific active, the ability of the active to penetrate through the skin, the age, health condition, and skin condition of the user, and other like factors.

The active ingredients useful herein can be categorized by their therapeutic benefit or their postulated mode of action. However, it is to be understood that the active ingredients useful herein can in some instances provide more than one therapeutic benefit or operate via more than one mode of action. Therefore, classifications herein are made for the sake of convenience and are not intended to limit the active ingredient to that particular application or applications listed. Also, pharmaceutically-acceptable salts of these active ingredients are useful herein. The following active ingredients are useful in the compositions of the present invention.

Anti-Acne Actives: Examples of useful anti-acne actives include the keratolytics such as salicylic acid (o-hydroxybenzoic acid), derivatives of salicylic acid such as 5-octanoyl salicylic acid, and resorcinol; retinoids such as retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.

Anti-Wrinkle and Anti-Skin Atrophy Actives: Examples of antiwrinkle and anti-skin atrophy actives include retinoic acid and its derivatives (e.g., cis and trans); retinol; retinyl esters; niacinamide, salicylic acid and derivatives thereof; sulfur-containing D and L amino acids and their derivatives and salts, particularly the N-acetyl derivatives, a preferred example of which is N-acetyl-L-cysteine; thiols, e.g. ethane thiol; hydroxy acids, phytic acid, lipoic acid; lysophosphatidic acid, and skin peel agents (e.g., phenol and the like).

Non-Steroidal Anti-Inflammatory Actives (NSAIDS): Examples of NSAIDS include the following categories: propionic acid derivatives; acetic acid derivatives; fenamic acid derivatives; biphenylcarboxylic acid derivatives; and oxicams. All of these NSAIDS are fully described in U.S. Patent 4,985,459 to Sunshine et al., issued January 15, 1991. Examples of useful NSAIDS include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Also useful are the steroidal anti-inflammatory drugs including hydrocortisone and the like.

Topical Anesthetics: Examples of topical anesthetic drugs include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof.

Artificial Tanning Agents and Accelerators. Examples of artificial tanning agents and accelerators include dihydroxyacetaone, tyrosine, tyrosine' esters such as ethyl tyrosinate, and phospho-DOPA.

Antimicrobial and Antifungal Actives: Examples of antimicrobial and antifungal actives include β-lactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, octopirox, parachlorometa xylenol, nystatin, tolnaftate, zinc pyrithione and clotrimazole.

Preferred examples of actives useful herein include those selected from the group consisting of salicylic acid, benzoyl peroxide, 3-hydroxy benzoic acid, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, phytic acid, N-acetyl-L-cysteine, lipoic acid, azelaic acid, arachidonic acid, benzoylperoxide, tetracycline, ibuprofen, naproxen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, neocycin sulfate, and mixtures thereof.

Sunscreen Actives: Also useful herein are sunscreening actives. A wide variety of sunscreening agents are described in U.S. Patent No. 5,087,445, to Haffey et al., issued February 11, 1992; U.S. Patent No. 5,073,372, to Turner et al., issued December 17, 1991; U.S. Patent No. 5,073,371, to Turner et al. issued December 17, 1991; and Segarin, et al., at Chapter VIII, pages 189 et seq., of Cosmetics Science and Technology. Nonlimiting examples of sunscreens which are useful in the compositions of the present invention are those selected from the group consisting of 2-ethylhexyl p-methoxycinnamate, 2-ethylhexyl N,N-dimethyl-*p*-aminobenzoate, *p*-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof. Still other useful sunscreens are those disclosed in U.S. Patent No. 4,937,370, to Sabatelli, issued June 26, 1990; and U.S. Patent No. 4,999,186, to Sabatelli et al., issued March 12, 1991. Especially preferred examples of these sunscreens include those selected from the group consisting of 4-N, N-(2-ethylhexyl)methylaminobenzoic acid ester of 2,4-dihydroxybenzophenone, 4-N,N-(2-ethylhexyl)methylaminobenzoic acid ester with 4-hydroxydibenzoylmethane, 4-N,N- (2-ethylhexyl)-methylaminobenzoic acid ester of 2-hydroxy-4-(2-hydroxyethoxy)benzophenone, 4-N,N-(2-ethylhexyl)-methylaminobenzoic acid ester of 4-(2-hydroxyethoxy) dibenzoylmethane, and mixtures thereof. Exact amounts of sunscreens which can be employed will vary depending upon the sunscreen chosen and the desired Sun Protection Factor (SPF) to be achieved. SPF is a commonly used measure of photoprotection of a sunscreen against erythema. See Federal Register, Vol. 43, No. 166, pp. 38206-38269, August 25, 1978, which is incorporated herein by reference in its entirety.

Nonlimiting examples of preferred actives useful herein include those selected from the group consisting of salicylic acid, benzoyl peroxide, niacinamide, cis-retinoic acid, trans-retinoic acid, retinol, retinyl palmitate, phytic acid, N-acetyl L-cysteine, azelaic acid, lipoic acid, resorcinol, lactic acid, glycolic acid, ibuprofen, naproxen, hydrocortisone, phenoxyethanol, phenoxypropanol, phenoxyisopropanol, 2,4,4,'-trichloro-2'-hydroxy diphenyl ether, 3,4,4'-trichlorocarbanilide, 2-ethylhexyl *p*-methoxycinnamic acid, oxybenzone, 2-phenylbenzimidozole-5-sulfonic acid, dihydroxyacetone, and mixtures thereof.

B. Cationic Surfactants

The articles of the present invention can also optionally comprise one or more cationic surfactants, provided these materials are selected so as not to interfere with the overall lathering characteristics of the required, lathering surfactants. Cationic surfactants are useful as anti-static agents or as emulsifiers.

Nonlimiting examples of cationic surfactants useful herein are disclosed in McCutcheon's, Detergents and Emulsifiers, North American edition (1986), published by allured Publishing Corporation; and McCutcheon's, Functional Materials, North American Edition (1992).

Nonlimiting examples of cationic surfactants useful herein include cationic alkyl ammonium salts such as those having the formula:

$$R_1R_2R_3R_4N^+ X^-$$

wherein $R_1$, is selected from an alkyl group having from about 12 to about 18 carbon atoms, or aromatic, aryl or alkaryl groups having from about 12 to about 18 carbon atoms; $R_2$, $R_3$, and $R_4$ are independently selected from hydrogen, an alkyl group having from about 1 to about 18 carbon atoms, or aromatic, aryl or alkaryl groups having from about 12 to about 18 carbon atoms; and X is an anion selected from chloride, bromide, iodide, acetate, phosphate, nitrate, sulfate, methyl sulfate, ethyl sulfate, tosylate, lactate, citrate, glycolate, and mixtures thereof. Additionally, the alkyl groups can also contain ether linkages, or hydroxy or amino group substituents (e.g., the alkyl groups can contain polyethylene glycol and polypropylene glycol moieties).

More preferably, $R_1$ is an alkyl group having from about 12 to about 18 carbon atoms; $R_2$ is selected from H or an alkyl group having from about 1 to about 18 carbon atoms; $R_3$ and $R_4$ are independently selected from H or an alkyl group having from about 1 to about 3 carbon atoms; and X is as described in the previous paragraph.

Most preferably, $R_1$ is an alkyl group having from about 12 to about 18 carbon atoms; $R_2$, $R_3$, and $R_4$ are selected from H or an alkyl group having from about 1 to about 3 carbon atoms; and X is as described previously.

Alternatively, other useful cationic surfactants include amino-amides, wherein in the above structure $R_1$ is alternatively $R_5CO-(CH_2)_n$ -, wherein $R_5$ is an alkyl group having from about 12 to about 22 carbon atoms, and 'n is an integer from about 2 to about 6, more preferably from about 2 to about 4, and most preferably from about 2 to about 3. Nonlimiting examples of these cationic emulsifiers include stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

Nonlimiting examples of quaternary ammonium salt cationic surfactants include those selected from the group consisting of cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, lauryl dimethyl ammonium bromide, stearyl dimethyl ammonium chloride, stearyl dimethyl ammonium bromide, cetyl trimethyl ammonium chloride, cetyl trimethyl ammonium bromide,

lauryl trimethyl ammonium chloride, lauryl trimethyl ammonium bromide, stearyl trimethyl ammonium chloride, stearyl trimethyl ammonium bromide, lauryl dimethyl ammonium chloride, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl dimethyl ammonium chloride, distearyl methyl ammonium bromide, and mixtures thereof. Additional quaternary ammonium salts include those wherein the C12 to C22 alkyl carbon chain is derived from a tallow fatty acid or from a coconut fatty acid. The term "tallow" refers to an alkyl group derived from tallow fatty acids (usually hydrogenated tallow fatty acids), which generally have mixtures of alkyl chains in the C16 to C18 range. The term "coconut" refers to an alkyl group derived from a coconut fatty acid, which generally have mixtures of alkyl chains in the C12 to C14 range. Examples of quaternary ammonium salts derived from these tallow and coconut sources include ditallow dimethyl ammonium chloride, ditallow dimethyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, di(coconutalkyl)dimethyl ammonium chloride, di(coconutalkyl)dimethyl ammonium bromide, tallow ammonium chloride, coconut ammonium chloride, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

Preferred cationic surfactants useful herein include those selected from the group consisting of dilauryl dimethyl ammonium chloride, distearyl dimethyl ammonium chloride, dimyristyl dimethyl ammonium chloride, dipalmityl dimethyl ammonium chloride, distearyl dimethyl ammonium chloride, and mixtures thereof.

C. Other Optional Ingredients

The compositions of the present invention can comprise a wide range of other optional components. These additional components should be pharmaceutically acceptable. The CTFA Cosmetic Ingredient Handbook, Second Edition, 1992 describes a wide variety of nonlimiting cosmetic and pharmaceutical ingredients commonly used in the skin care industry, which are suitable for use in the compositions of the present invention. Nonlimiting examples of functional classes of ingredients are described at page 537 of this reference. Examples of these and other functional classes include: abrasives, absorbents, anticaking agents, antioxidants, vitamins, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, preservatives, propellants, reducing agents, skin bleaching agents, and sunscreening agents.

Also useful herein are aesthetic components such as fragrances, pigments, colorings, essential oils, skin sensates, astringents, skin soothing agents, and skin healing agents.

VIII. METHODS OF MANUFACTURE

The disposable, single use personal care cleansing and conditioning articles of the present invention are manufactured by separately or simultaneously adding onto or impregnating into a water insoluble substrate a lathering surfactant, a conditioning component, and a fragrance component, wherein said resulting article is substantially dry. By "separately" is meant that the surfactants and conditioning agents can be added sequentially, in any order without first being combined together. By "simultaneously" is meant that the surfactants and conditioning agents can be added at the same time, with or without first being combined together. Preferably, the fragrance component should be added onto or impregnated into the substrate, or any layer therof, separately from the lathering sufactants, because of the possibility of solubilizing the water soluble encapsulation material. The fragrance component is also preferably added onto or impregnated into the substrate, or any layer thereof, when the substrate is dry.

When there is more than one layer, the lathering surfactant and/or the conditioning component can also be added onto or impregnated into each layer in any sequence. Alternatively, the lathering surfactant and/or the conditioning component can be added onto or impregnated into the substrate. Treatment with the lather surfactant, the conditioning component, and/or the fragrance component can be achieved at anytime before or after joining the first layer and the second layer. Despite the order of treatment, excess surfactant, conditioning component, and/or fragrance component should be removed (e.g., by a nipping process). Thereafter, the treated material (e.g., the first layer 100, the second layer 200, both layers 100 and 200, or joined substrate) should be dried by conventional means.

For example, prior to joining the first layer 100 to the second layer 200, the second second layer can be treated with the lathering surfactant. After joining the two layers, either of the outside surfaces (e.g., the unjoined surfaces) of layers 100 and/or 200 can be treated with the conditioning component and/or fragrance component. Alternatively, the lathering surfactants and conditioning agents can be added onto or impregnated into the second layer

200 at the same time prior to joining the two layers. Alternatively, the lathering surfactants and the conditioning agents can be combined together before adding onto or impregnating into the second layer 200. In either sequence, the fragrance component must not be added in conjuction with the surfactant. If the fragrance component is added after the surfactant, any excess solvents (e.g., water) must be removed before adding the fragrance component.

Alternatively, prior to joing the two layers, the first layer 100 can be treated with the lathering surfactant employing methods which do not cause the first layer to elongate or extend. This can be achieved in the manufacturing of the first layer or by various application methods well known to those of ordinary skill in the art. Nonlimiting examples of applicaiton methods include extrusion coating and slot coating.

The surfactant, conditioning agents, fragrance component, and any optional ingredients can be added onto or impregnated into either layer (100 or 200) or the resulting joined layers (100 and 200) by any means known to those skilled in the art: for example, by spraying, laser printing, splashing, dipping, soaking, or coating.

When water or moisture is used or present in the manufacturing process, the fragrance component is added after the resulting treated substrate is then dried so that it is substantially free of water. The treated substrate can be dried by any means known to those skilled in the art. Nonlimiting examples of known drying means include the use of convection ovens, radiant heat sources, microwave ovens, forced air ovens, and heated rollers or cans. Drying also includes air drying without the addition of heat energy, other than that present in the ambient environment. Also, a combination of various drying methods can be used.

## IX METHODS OF CLEANSING AND CONDITIONING THE SKIN OR HAIR

The present invention also relates to a method of cleansing and conditioning the skin or hair with a personal cleansing article of the present invention. These methods comprise the steps of wetting with water a substantially dry, disposable, single use personal cleansing article comprising a water insoluble substrate, a lathering surfactant, and a conditioning component, and contacting the skin or hair with such wetted article. In further embodiments, the present invention is also useful for delivering various active ingredients to the skin or hair.

The articles of the present invention are substantially dry and are intended to be wetted with water prior to use. The article is wetted by immersion in water or by placing it under a stream of water. Lather is generated from the article by mechanically agitating and/or deforming the article either prior to or during contact of the article with the skin or hair. The resulting lather is useful for cleansing and conditioning the skin or hair. During the cleansing process and subsequent rinsing with water, the conditioning agents and active ingredients are deposited onto the skin or hair. Deposition of conditioning agents and active ingredients are enhanced by the physical contact of the substrate with the skin or hair.

## X. METHOD OF CONSISTENTLY DEPOSITING CONDITIONING AGENTS AND ANY ACTIVE INGREDIENTS ONTO THE SKIN OR HAIR

The articles of the present invention are useful for consistently depositing the conditioning agents of the present invention to the skin or hair. In further embodiments where an active ingredient is present, the compositions are also useful for consistently depositing the active ingredient to the skin or hair.

The articles of the present invention have a deposition consistency of greater than about 60%, preferably greater than about 65%, more preferably greater than about 70%, and most preferably greater than about 75%.

The deposition consistency measurement is the quotient obtained from dividing the deposition of conditioning agents via "non-ideal lathering and use" by deposition of conditioning agents via "ideal lathering and use." Non-ideal lathering, as used herein, means that lathering is achieved by rubbing the surface of the article containing the conditioning agents and then contacting the skin or hair with the same surface. This causes inefficient deposition of the conditioning agents because some of the conditioning agents become emulsified by the surfactant. Ideal lathering, as used herein, means that lathering is achieved by rubbing the surface of the article not containing conditioning agents and then contacting the skin or hair with the surface containing the conditioning component. The same reference points would apply if both surfaces of the substrate are treated with the conditioning agents (e.g. deposition obtained from lathering and contacting the skin with the same lathered surface containing emulsified conditioning agents versus contacting the skin with the non-lathered surface which contains non-emulsified conditioning agents). Deposition consistency is maximized when the lipid hardness value of the conditioning component is greater than about 0.02 kg.

Quantification of the conditioning component deposited on the skin or hair can be measured using a variety of standard analytical techniques well known to the chemist of ordinary skill in the art. Such methods include for instance extraction of an area of the skin or hair with a suitable solvent followed by analysis by chromatography (i.e. gas chromatography, liquid chromatography, supercritical fluid chromatography, etc.), IR spectroscopy, UV/VIS spectroscopy, mass spectrometry, etc. Direct measurements can also be made on the skin or hair by techniques such as IR spectroscopy, UV/VIS spectroscopy, opacity measurements, fluoresce spectroscopy, ESCA spectroscopy, and the like.

In a typical method for measuring deposition, a article of the present invention is wetted with water and squeezed and agitated to generate a lather. The article is then rubbed for approximately 15 seconds on a site, approximately about 25 $cm^2$ to about 300 $cm^2$, preferably about 50 $cm^2$ to about 100 $cm^2$, on the skin or head which has been demarcated using an appropriate indelible marker. The site is then rinsed for approximately 10 seconds and then allowed to air dry for approximately 10 minutes. The site is then either extracted and the extracts analyzed, or analyzed directly using any techniques such as those exemplified above.

## XI EXAMPLES

The following examples further describe and demonstrate embodiments within the scope of the present invention. In the following examples, all ingredients are listed at an active level.

Ingredients are identified by chemical or CTFA name, and all weights are in percent actives.

### Examples 1-5.

### A. The Substrate

A multi-layered substrate, as described in Figures 1, 3A, and 3B, is prepared as herein described. This substrate may be substituted with any nonwoven, e.g., Veratec 104-102 or Chicopee C5763.

### B. Surfactant Phase

In a suitable vessel, the following ingredients are mixed at room temperature. Once the polyquaternium is dispersed, the mixture is heated to 65°C.

| Ingredients | Weight Percent | | | | |
|---|---|---|---|---|---|
| | Example 1 | Example 2 | Example 3 | Example 4 | Example 5 |
| Water | QS 100 | QS 100 | QS 100 | QS 100 | QS 100 |
| Polyquaternium-10 | 0.25 | 0.25 | 0.25 | 0.25 | 0.25 |

While the above mixture is being heated to 65°C the following ingredients are added to the mixture.

| | | | | | |
|---|---|---|---|---|---|
| Disodium EDTA | 0.10 | 0.10 | 0.10 | 0.10 | 0.10 |
| Glycerin | 3.00 | 3.00 | 3.00 | 3.00 | 3.00 |
| Sodium Lauroyl Lactylate | 3.33 | 3.33 | ---- | 3.33 | 3.33 |
| Sodium Caproyl Lactylate | | | 3.33 | | |
| Cocamidopropyl Betaine | 3.33 | 3.33 | 3.33 | 3.33 | 3.33 |
| Decyl Polyglucoside | 3.33 | 3.33 | 3.33 | 3.33 | 3.33 |

Once the above ingredients are thoroughly mixed begin cooling the mixture to 45C. In a senarate mixing vessel add the following:

| | | | | | |
|---|---|---|---|---|---|
| Water | 2.0 | 2.0 | 2.0 | 2.0 | 2.0 |
| Butylene Glycol | 2.0 | 2.0 | 2.0 | 2.0 | 2.0 |
| Glydant Plus | 0.2 | 0.2 | 0.2 | 0.2 | 2.0 |

Once the Glydant Plus is dissolved add this mixture to the first mixing vessel and cool to room temperature. Once cooled, apply 1.5 g of this solution to a non-woven substrate and then dry.

### C. Conditioning Phase:

In a suitable vessel, the following ingredients are mixed at room temperature and heated to 70°C during mixing.

| | | | | | |
|---|---|---|---|---|---|
| SEFA* Cottonate | 48.00 | 75.00 | 33.6 | 70.00 | 80.00 |
| SEFA* Behenate | 12.00 | 25.0 | 8.40 | 10.00 | 10.00 |
| Petrolatum | 10.00 | --- | 7.00 | --- | --- |
| Glyceryl Tribehenate | 5.00 | --- | 3.50 | --- | --- |
| Stearyl Alcohol | --- | --- | --- | 5.00 | --- |
| Paraffin | --- | --- | --- | 15.00 | --- |
| Cholesterol Ester | 25.00 | --- | 17.50 | --- | --- |
| Ozokerite Wax | --- | --- | --- | --- | 10.00 |
| Glycerin | --- | --- | 28.00 | --- | --- |
| Triglyceryl monostearate[2] | --- | --- | 1.90 | --- | --- |
| Decaglyceryl dipalmitate[3] | --- | --- | 0.21 | --- | |

[1] Available as Abil WE-09 from Glodschmidt
[2] Available as PolyAldo TGMS from Lonza Chemical
[3] Available as PolyAldo 10-2P from Lonza Chemical
* SEFA is an acronym for sucrose esters of fatty acids

Cool to Room Temperature while mixing. Then add 0.17 g of this phase to the substrate already containing the surfactants from the Surfactant Phase. The resulting cleansing article is used by wetting with water and is useful for cleansing the skin or hair and for depositing the conditioning agents onto the skin or hair in a consistent manner.
The resulting lipid hardness values and the deposition consistencies are as follows:

| | Example 1 | Example 2 | Example3 | Example4 | Example 5 |
|---|---|---|---|---|---|
| Lipid Hardness Value | 0.20 | 2.00 | 0.025 | 5.00 | 1.00 |
| Deposition Consistency | 66% | 75% | 61% | 82% | 67% |

In alternative manufacturing procedures, the lathering surfactants, conditioning component, and optional ingredients are separately or simultaneously added onto or impregnated into (i) either or both layers prior to combining the layers into a laminate, or (ii) after the layers are combined into a laminate. The process of adding onto or impregnating into the substrate the surfactant and/or conditioning component is achieved by spraying, printing, splashing, dipping, or coating.
Similarly, the lathering surfactant and the conditioning emulsion can be added to the substrate in any order. Nonlimiting examples of the process sequences include (i) first adding surfactant to the second layer, then joining the substrate, then treating with the conditioning component; (ii) first combining surfactant with conditioning component then treating the second layer, then joining the two layers; (iii) prior to joining the two layers, treating the second layer with the surfactant first and then the conditioning component second, then joining the two layers.
In alternative embodiments, other substrates such as woven substrates, hydroentangled substrates, natural sponges, synthetic sponges, or polymeric netted meshes are substituted for the present substrate.

D. Fragrance Phase

About 66.5% of beta cyclodextrin, about 22% water and about 11.5% perfume are added at a total rate of about 450 grams /min to twin-screw extruder with no exit die. The mixer speed is about 400 RPM and the paddle configuration is selected to provide specific mechanical input to the mixer that is greater than 1 horsepower per pound per minute.
In a suitable vessel, the beta cyclodextrin complex is added to molten PEG 4600 (65c) at a ratio of 25% complex to 75% PEG 4600. The resulting mixture is milled with a techmar mill for 2.5 theoretical passes. The mixture is then pumped into suitable storage containers.
The resulting mixture is then added to the dry substrate at a rate of 0.3 grams per sheet.

| | | | | | |
|---|---|---|---|---|---|
| Water | 5.500 | 5.500 | 5.500 | 5.500 | 5.500 |

(continued)

| | | | | | |
|---|---|---|---|---|---|
| PEG 4600 | 75.000 | 75.00 | 75.00 | 75.00 | 75.00 |
| Beta cylcodextrin[1] | 16.625 | 16.625 | 16.625 | 16.625 | 16.625 |
| Selina PG Mod 1 [2] | 2.875 | --- | 2.875 | 2.000 | 2.000 |
| Menthol | --- | 2.875 | --- | 0.875 | 0.875 |

[1] Cerastar Corp
[2] Givaudan Roure

Examples 6-9

A personal care cleansing and conditioning product is prepared as follows:

| Ingredients | Weight Percent | | | | |
|---|---|---|---|---|---|
| | Example 6 | Example 7 | Example 8 | Example 9 | |
| Phase A | | | | | |
| Water | QS 100 | QS 100 | QS 100 | QS 100 | |
| Glycerin | | 10.00 | 10.00 | 10.00 | 10.00 |
| Disodium Lauroamphodiacetate (and) Sodium Trideceth Sulfate | | 4.00 | 4.00 | ---- | ---- |
| Sodium Lauroamphoacetate | ---- | ---- | 2.40 | 2.40 | |
| Sodium Lauroyl Sarcosinate | 4.00 | 4.00 | ---- | ---- | |
| Ammonium Laureth Sulfate | ---- | ---- | 4.20 | 4.20 | |
| Ammonium Lauryl Sulfate | ---- | ---- | 1.40 | 1.40 | |
| Polyquarternium-10 | 0.25 | 0.25 | 0.25 | 0.25 | |
| Disodium EDTA | 0.10 | 0.10 | 0.10 | 0.10 | |
| Phase B | | | | | |
| Sucrose Ester Fatty Acid Cottonate | | 3.00 | 3.00 | 3.00 | 3.00 |
| Petrolatum | ---- | 1.50 | ---- | ---- | |
| Cetyl Dimethicone | --- | ---- | ---- | 2.00 | |
| Phase C | | | | | |
| Butylene Glycol | 2.00 | 2.00 | 2.00 | 2.00 | |
| DMDM Hydantoin (and) Iodopropynyl Carbamate | 0.20 | 0.20 | 0.20 | 0.20 | |
| Phase D | | | | | |
| Water | 5.500 | 5.500 | 5.500 | 5.500 | |
| PEG 4600 | 75.000 | 75.00 | 75.00 | 75.00 | |
| Beta cylcodextrin[1] | 16.625 | 16.625 | 16.625 | 16.625 | |
| Selina PG Mod 1 [2] | 2.875 | --- | 2.875 | 2.000 | |
| Menthol | --- | 2.875 | --- | 0.875 | |

[1] Cerastar Corp
[2] Givaudan Roure

Water Insoluble Substrate

A hydroapertured, nonwoven substrate having a basis weight of about 60 gsy comprising 50% rayon and 50% polyester approximately 17.8 cm by 19.3 cm (6 in. by 7.6 in.) and a thickness of about 0.051 cm (20 mil).

In a suitable vessel., the Phase A ingredients are mixed at room temperature to form a dispersion and heated with stirring to 65°C. The Phase B ingredients are mixed in a separate suitable vessel and heated to 65°C. Once the temperatures are the same, the Phase B ingredients are mixed into the vessel containing the Phase A ingredients and then cooled to 45°C. The Phase C ingredients are then mixed together in a separate vessel at room temperature. Next,

the Phase C mixture is added into the vessel containing the combination of Phases A and B at room temperature. 1.5 grams of the resulting solution is sprayed onto each substrate. Alternatively, the substrate can be dipped into the resulting solution. The treated substrate is then dried in an oven to constant weight. Alternatively, the treated substrate is dried in a convection oven at 45°C to constant weight.

Phase D ingredients are prepared in the following manner. About 66.5% of beta cyclodextrin, about 22% water and about 11.5% perfume are added at a total rate of about 450 grams /min to twin-screw extruder with no exit die. The mixer speed is about 400 RPM and the paddle configuration is selected to provide specific mechanical input to the mixer that is greater than 1 horsepower per pound per minute.

In a suitable vessel, the beta cyclodextrin complex is added to molten PEG 4600 (65c) at a ratio of 25% complex to 75% PEG 4600. The resulting mixture is milled with a techmar mill for 2.5 theoretical passes. The mixture is then pumped into suitable storage containers.

The resulting mixture is then added to the dry substrate at a rate of 0.3 grams per sheet.

The resulting cleansing composition is used by wetting with water and is useful for cleansing the skin or hair and for depositing the conditioning agents onto the skin or hair.

In alternative manufacturing procedures, the lathering surfactants, conditioning agents, and optional ingredients can be separately or simultaneously added onto or impregnated into the water insoluble substrate by spraying, laser printing, splashing, dipping, or coating.

In alternative embodiments, other substrates such as woven substrates, hydroentangled substrates, natural sponges, synthetic sponges, or polymeric netted meshes.

Examples 10-14

Phase A. Surfactant Phase

In a suitable vessel, the following ingredients are mixed at room temperature. Once the polyquaternium is dispersed. the mixture is heated to 65°C.

| Ingredients | Weight Percent | | | | |
|---|---|---|---|---|---|
| | Example 10 | Example 11 | Example 12 | Example 13 | Example 14 |
| Water | QS 100 | QS 100 | QS 100 | QS 100 | QS 100 |
| Polyquaternium-10 | 0.25 | 0.25 | 0.25 | 0.25 | 0.25 |

While the above mixture is being heated to 65°C the following ingredients are added to the mixture.

| | | | | | |
|---|---|---|---|---|---|
| Disodium EDTA | 0.10 | 0.10 | 0.10 | 0.10 | — |
| Ammonium Laureth Sulfate | 4.2 | 4.2 | 4.2 | 4.2 | — |
| Ammonium Lauryl Sulfate | 1.4 | 1.4 | 1.4 | 1.4 | — |
| Sodium Lauroamphoacetate | 2.4 | 2.4 | 2.4 | 2.4 | — |
| Sodium Lauroyl Sarcosinate | — | — | — | — | 4.0 |
| Disodium Lauroamphodiacetate & Sodium Trideceth Sulfate | — | — | — | — | 4.0 |

Once the above ingredients are thoroughly mixed begin cooling the mixture to 45C. In a separate mixing vessel add the following:

| | | | | | |
|---|---|---|---|---|---|
| Water | 2.0 | 2.0 | 2.0 | 2.0 | 2.0 |
| Butylene Glycol | 2.0 | 2.0 | 2.0 | 2.0 | 2.0 |
| Glydant Plus | 0.2 | 0.2 | 0.2 | 0.2 | 2.0 |

Once the Glydant Plus is dissolved add this mixture to the first mixing vessel and cool to room temperature. Once cooled, apply 1.5 g of this solution to a non-woven substrate and then dry.

Phase B. CONDITIONING EMULSION:

In a suitable vessel, the following ingredients are mixed at room temperature and heated to 70°C during mixing.

| | | | | | |
|---|---|---|---|---|---|
| SEFA* Cottonate | 4.65 | 4.00 | 4.65 | 34.40 | 4.65 |
| SEFA* Behenate | 0.35 | — | 0.35 | 2.60 | 0.35 |
| Petrolatum | — | 1.00 | — | — | — |
| Sorbitan Monooleate | — | — | — | 3.00 | — |
| Polyglycerl-4 Isostearate (and) Cetyl Dimethicone (and) Hexyl Laurate[1] | 5.00 | 5.00 | 5.00 | — | 5.00 |

[1] Available as Abil WE-09 from Glodschmidt

* SEFA is an acronym for sucrose esters of fatty acids

Once the mixture reaches 70°C, stop heating and slowly add the following ingredients while continuing to mix:

| | | | | | |
|---|---|---|---|---|---|
| Glycerin | 90.00 | 90.00 | 70.00 | 60.00 | 90.00 |
| Panthenol | — | — | 20.00 | — | — |

Cool to Room Temperature while mixing. Then add 0.17 g of this phase to the substrate already containing the surfactants from the Surfactant Phase.

| Phase C. Fragrance Phase | | | | | |
|---|---|---|---|---|---|
| Water | 5.500 | 5.500 | 5.500 | 5.500 | 5.500 |
| PEG 4600 | 75.000 | 75.00 | 75.00 | 75.00 | 75.00 |
| Beta cylcodextrin[1] | 16.625 | 16.625 | 16.625 | 16.625 | 16.625 |
| Selina PG Mod 1 [2] | 2.875 | --- | 2.875 | 2.000 | 2.000 |
| Menthol | --- | 2.875 | --- | 0.875 | 0.875 |

[1] Cerastar Corp

[2] Givaudan Roure

About 66.5% of beta cyclodextrin, about 22% water and about 11.5% perfume are added at a total rate of about 450 grams /min to twin-screw extruder with no exit die. The mixer speed is about 400 RPM and the paddle configuration is selected to provide specific mechanical input to the mixer that is greater than 1 horsepower per pound per minute.

In a suitable vessel, the beta cyclodextrin complex is added to molten PEG 4600 (65c) at a ratio of 25% complex to 75% PEG 4600. The resulting mixture is milled with a techmar mill for 2.5 theoretical passes. The mixture is then pumped into suitable storage containers.

The resulting mixture is then added to the dry substrate at a rate of 0.3 grams per sheet. The resulting cleansing composition is used by wetting with water and is useful for cleansing the skin or hair and for depositing the conditioning emulsions onto the skin or hair.

In alternative manufacturing procedures, the lathering surfactants, conditioning emulsions, and optional ingredients are separately or simultaneously added onto or impregnated into the water insoluble substrate by spraying, printing, splashing, dipping, or coating.

In alternative embodiments, other substrates such as woven substrates, hydroentangled substrates, natural sponges, synthetic sponges, or polymeric netted meshes are substituted for the present substrate.

## Claims

1. A disposable, single use personal care cleansing and conditioning product which deposits materials that are rinsed from the skin or hair, which has desirable fragrance delivery properties, and which is **characterized in that** it comprises:

   (A) a water insoluble substrate,
   (B) at least one lathering surfactant added onto or impregnated into said substrate, and
   (C) from 0.015% to 15%, by weight of said water insoluble substrate, of a fragrance-releasing complex added

onto or impregnated into said substrate, said fragrance-releasing complex comprising:

(i) from 10% to 90%, by weight of the complex, of a porous fragrance carrier, and
(ii) from 1% to 90%, by weight of the complex, of a fragrance impregnated within said carrier,

wherein said product is substantially dry prior to use.

**2.** A product according to Claim 1 wherein said lathering surfactant comprises from 0.5% to 12.5%, by weight of said water insoluble substrate, and wherein the ratio of said fragrance to said porous fragrance carrier ranges from 5: 1 to 1:10.

**3.** A product according to Claim 1 or Claim 2 wherein said fragrance carrier comprises carriers selected from the group consisting of cyclodextrin, preferably alpha-cylcodextrin, beta-cyclodextrin, gamma-cylcodextrin, or mixtures and derivatives thereof; amorphous silicas; precipitated silicas; fumed silicas; colloidal silicas; spheroidal silicas; aluminosilicates, preferably zeolite, alumina, or mixtures thereof; calcium silicates; porous starches; agglomerated starches; polymethacrylate copolymers; and mixtures thereof.

**4.** A product according to any of Claims 1 to 3 wherein said fragrance is selected from the group consisting of perfume ingredients, cooling ingredients, and mixtures thereof.

**5.** A product according to any of Claims 1 to 4 wherein at least a portion of the fragrance is selected from the group consisting of: highly volatile perfume components having a boiling point up to 250° C; moderately volatile perfumes components having a boiling point from 250° C to 350° C; and mixtures thereof

**6.** A product according to any of Claims 1 to 5 further comprising from 0.01% to 10%, by weight of the substrate, of a neat fragrance.

**7.** A product according to any of Claims 1 to 6 wherein the fragrance-releasing complex is encapsulated with a coating material selected from the group consisting of paraffin waxes; microcrystalline waxes; animal waxes; vegetable waxes; saturated fatty acids and fatty alcohols; fatty esters; cellulose esters; polyalkylene glycol, preferably polyethylene glycols, polypropylene glycols, mixed polyalkylene glycols, and mixed polyalkylene glycol copolymers; polyvinyl alcohol; and mixtures thereof, and wherein the coating material is present in an amount ranging from 2% to 50% of the fragrance-releasing complex.

**8.** A product according to Claim 7 wherein said fragrance carrier is cyclodextrin, and wherein said coating material is polyethylene glycol, preferably having molecular weight from 4,400 to 400,000.

**9.** A product according to any of Claims 1 to 8 further comprising from 3% to 99%, by weight of said water insoluble substrate, of a conditioning component added onto or impregnated into said substrate, said conditioning component comprising materials selected from the group consisting of water soluble conditioning agents, oil soluble conditioning agents, conditioning emulsions, lipid hardening materials, and mixtures thereof.

**10.** A product according to any of Claims 1 to 9 wherein said lathering surfactant is selected from the group consisting of anionic lathering surfactants, nonionic lathering surfactants, amphoteric lathering surfactants, and mixtures thereof.

**11.** A product according to any of Claims 1 to 10 wherein said water insoluble substrate is selected from the group consisting of nonwoven substrates, woven substrates, hydroentangled substrates, natural sponges, synthetic sponges, polymeric netted meshes, formed films, and mixtures thereof.

**12.** A product according to any of Claims 9 to 11 wherein said oil soluble conditioning agent and said lipid hardening material are selected from the group consisting of fatty acids, esters of fatty acids, fatty alcohols, ethoxylated alcohols, polyol polyesters, glycerin mono-esters, glycerin polyesters, epidermal and sebaceous hydrocarbons, lanolin, mineral oil, silicone oil, silicone gum, vegetable oil, vegetable oil adduct, petrolatum, nonionic polymers, hydrogenated vegetable oils, nonionic polymers, natural waxes, synthetic waxes, polyolefinic glycols, polyolefinic monoester, polyolefinic polyesters, cholesterols, cholesterol esters, and mixtures thereof; and wherein said water soluble conditioning agent is selected from the group consisting of glycerin, glycerol, propylene glycol, polypropylene glycols, polyethylene glycols, ethyl hexanediol, hexylene glycols, other aliphatic alcohols, panthenol, urea,

cationic polymers, polyols, glycolic acid, lactic acid, and mixtures thereof.

**13.** A product according to any of Claims 9 to 12 wherein said conditioning emulsion comprises,

(A) an internal phase comprising a water soluble conditioning agent selected from one or more water soluble agents such that the weighted arithmetic mean solubility parameter of said water soluble conditioning agent is greater than 10.5, and
(B) an external phase comprising an oil soluble agent selected from one or more oil soluble agents such that the weighted arithmetic mean solubility parameter of said water soluble conditioning agent is greater than 10.5.

**14.** A product according to Claim 13 further comprising from 0.1% to 20% by weight of said conditioning emulsion of an emulsifier capable of forming an emulsion of said internal and external phases, wherein said emulsifier is selected from one or more emulsifiers such that the weighted arithmetic mean HLB value is from 1 to 7.

**15.** A product according to any of Claims 9 to 14 wherein said conditioning component has a surface to saturation ratio of greater than or equal to 1.25 at any point on the surface of the substrate.

**16.** A product according to any of Claims 9 to 15 wherein said conditioning component has a lipid hardness value of greater than 0.02 kg.

**17.** A product according to any of Claims 1 to 16 wherein said water insoluble substrate comprises at least two sheets of fibers each in turn having different textures.

**18.** A product according to Claim 17 wherein said water insoluble substrate comprises at least one portion that is wet extensible and at least a second portion that is less wet extensible than said first portion.

**19.** An article according to Claim 17 or Claim 18 wherein said water insoluble substrate comprises:

(A) an apertured first layer, the first layer being wet extensible in the plane of the first layer when the first layer is wetted; and
(B) a second layer which is less wet extensible when wetted than is said first layer;

wherein selected portions of said first layer are joined to said second layer in a manner which is sufficient to inhibit wet extension of said first layer in the plane of said first layer.

**20.** A product according to any of Claims 1 to 19 wherein said cleansing product further comprises a safe and effective amount of one or more active ingredients selected from the group consisting of anti-acne actives, anti-wrinkle and anti-skin actives, non-stearoidal anti-inflammatory actives, topical anesthetics, artificial tanning agents and accelerators, anti-microbial and anti-fungal agents, sunscreen actives, antioxidants and mixtures thereof.

**21.** A method of manufacturing a disposable, single use personal care cleansing and conditioning product which deposits materials that are rinsed from the skin or hair and which has desirable fragrance delivery properties, which method is **characterized in that** it comprises the steps of separately or simultaneously adding onto or impregnating into a water insoluble substrate

(A) at least one lathering surfactant added onto or impregnated into said substrate, and
(B) from 0.015% to 15%, by weight of said water insoluble substrate, of a fragrance-releasing complex added onto or impregnated into said substrate, said fragrance-releasing complex comprising:

(i) from 10% to 90%, by weight of the complex, a porous fragrance carrier, and
(ii) from 1% to 90%, by weight of the complex, a fragrance impregnated within said carrier,

wherein said product is substantially dry prior to use.

**22.** A method of manufacturing a product according to Claim 21 wherein said fragrance releasing complex is encapsulated with a coating material selected from the group consisting of paraffin waxes, microcrystalline waxes, animal waxes, vegetable waxes, saturated fatty acids and fatty alcohols, fatty esters, cellulose esters, polyalkylene glycol, polyvinyl alcohol, and mixtures thereof and wherein the coating material is present in an amount ranging from 2%

to 50% of the fragrance-releasing complex.

**23.** A method of manufacturing a product according to Claim 21 or Claim 22 comprising the further step of separately or simultaneously adding onto or impregnating into a water insoluble substrate from 0.1% to 10%, by weight of the substrate, of a neat fragrance.

**Patentansprüche**

**1.** Wegwerfbares, einmal verwendbares Reinigungs- und Konditionierungsprodukt für die persönliche Pflege, welches Materialien, die von der Haut oder dem Haar abgespült werden, abscheidet, welches erwünschte Duftabgabeeigenschaften besitzt, und welches **dadurch gekennzeichnet ist, dass** es umfasst:

(A) ein wasserunlösliches Substrat,
(B) mindestens ein schäumendes Tensid, das auf das Substrat gegeben oder in dieses imprägniert worden ist, und
(C) 0,015 bis 15 Gew.-% des wasserunlöslichen Substrats eines duftstofffreisetzenden Komplexes, der auf das Substrat gegeben oder darin imprägniert worden ist, wobei der duftstofffreisetzende Komplex umfasst:

(i) 10 bis 90 Gew.-% des Komplexes eines porösen Dufstoffträgers, und
(ii) 1 bis 90 Gew.-% des Komplexes eines innerhalb des Trägers imprägnierten Duftstoffs,

wobei das Produkt vor der Verwendung im Wesentlichen trocken ist.

**2.** Produkt nach Anspruch 1, wobei das schäumende Tensid 0,5 bis 12,5 Gew.-% des wasserunlöslichen Substrats umfasst, und wobei das Verhältnis des Duftstoffs zu dem porösen Duftstoffträger im Bereich von 5:1 bis 1:10 liegt.

**3.** Produkt nach Anspruch 1 oder Anspruch 2, wobei der Duftstoffträger Träger umfasst, gewählt aus der Gruppe, bestehend aus Cyclodextrin, vorzugsweise alpha-Cyclodextrin, beta-Cyclodextrin, gamma-Cyclodextrin oder Mischungen und Derivate hiervon; amorphe Silicas; ausgefällte Silicas, Kieselpuder; kolloidale Silicas; sphäroidale Silicas; Alumosilicate, vorzugsweise Zeolith, Aluminiumoxid oder Mischungen hiervon; Calciumsilicate; poröse Stärken, agglomerierte Stärken, Polymethacrylatcopolymere; und Mischungen hiervon.

**4.** Produkt nach mindestens einem der Ansprüche 1 bis 3, wobei der Duftstoff aus der Gruppe gewählt ist, bestehend aus Parfümbestandteilen, kühlenden Bestandteilen und Mischungen hiervon.

**5.** Produkt nach mindestens einem der Ansprüche 1 bis 4, wobei mindestens ein Teil des Duftstoffes aus der Gruppe gewählt ist, bestehend aus hochflüchtigen Parfümkomponenten mit einem Siedepunkt bis zu 250°C; mäßigflüchtigen Parfümkomponenten mit einem Siedepunkt von 250°C bis 350°C; und Mischungen hiervon.

**6.** Produkt nach mindestens einem der Ansprüche 1 bis 5, umfassend weiterhin 0,01 bis 10 Gew.-% des Substrats eines unverdünnten Duftstoffs.

**7.** Produkt nach mindestens einem der Ansprüche 1 bis 6, wobei der duftstofffreisetzende Komplex eingekapselt ist mit einem Beschichtungsmaterial, gewählt aus der Gruppe, bestehend aus Paraffinwachsen, mikrokristallinen Wachsen; tierischen Wachsen; pflanzlichen Wachsen; gesättigten Fettsäuren und Fettalkoholen; Fettsäureestern; Zelluloseestern; Polyalkylenglykol, vorzugsweise Polyethylenglykolen, Polypropylenglykolen, gemischten Polyalkylenglykolen und gemischten Polyalkylenglykolcopolymeren; Polyvinylalkohol; und Mischungen hiervon, und wobei das Beschichtungsmaterial in einer Menge im Bereich von 2% bis 50% des duftstofffreisetzenden Komplexes vorliegt.

**8.** Produkt nach Anspruch 7, wobei der Duftstoffträger Cyclodextrin ist, und
wobei das Beschichtungsmaterial Polyethylenglykol ist, vorzugsweise mit einem Molekulargewicht von 4.400 bis 400.000.

**9.** Produkt nach mindestens einem der Ansprüche 1 bis 8, umfassend weiterhin 3 bis 99 Gew.-% des wasserunlöslichen Substrats einer konditionierenden Komponente, die auf das Substrat gegeben oder darin imprägniert worden ist, wobei die konditionierende Komponente Materialien umfasst, gewählt aus der Gruppe. bestehend aus wasserlöslichen Konditionierungsmitteln, öllöslichen Konditionierungsmitteln, konditionierenden Emulsionen, lipidhärtenden Materialien und Mischungen hiervon.

**10.** Produkt nach mindestens einem der Ansprüche 1 bis 9, wobei das schäumende Tensid aus der Gruppe gewählt ist, bestehend aus anionischen schäumenden Tensiden, nichtionischen schäumenden Tensiden, amphoteren schäumenden Tensiden und Mischungen hiervon.

**11.** Produkt nach mindestens einem der Ansprüche 1 bis 10, wobei das wasserunlösliche Substrat aus der Gruppe gewählt ist, bestehend aus Nonwoven-Substraten, gewebten Substraten, hydroverknäuelten Substraten, natürlichen Schwämmen, synthetischen Schwämmen, polymeren Netzmaschenmaterialien, geformten Folien und Mischungen hiervon.

**12.** Produkt nach mindestens einem der Ansprüche 9 bis 11, wobei das öllösliche konditionierende Mittel und das lipidhärtende Material aus der Gruppe gewählt sind, bestehend aus Fettsäuren, Estern von Fettsäuren, Fettalkoholen, ethoxylierten Alkoholen, Polyolpolyestern, Glyzerinmonoestern. Glyzerinpolyestern, epidermalen und talgartigen Kohlenwasserstoffen, Lanolin, Mineralöl, Siliconöl, Silicongummi, Pflanzenöl, Pflanzenöladdukt, Petrolatum, nichtionischen Polymeren, hydrierten Pflanzenölen, nichtionischen Polymeren, natürlichen Wachsen, synthetischen Wachsen, polyolefinischen Glykolen, polyolefinischem Monoester, polyolefinischen Polyestern, Cholesterolen, Cholesterolestern und Mischungen hiervon; und wobei das wasserlösliche Konditionierungsmittel aus der Gruppe gewählt ist, bestehend aus Glyzerin, Glycerol, Propylenglykol, Polypropylenglykolen, Polyethylenglykolen, Ethylhexandiol, Hexylenglykolen, anderen aliphatischen Alkoholen, Panthenol, Harnstoff, kationischen Polymeren, Polyolen, Glykolsäure, Milchsäure und Mischungen hiervon.

**13.** Produkt nach mindestens einem der Ansprüche 9 bis 12, wobei die konditionierende Emulsion umfasst,

(A) eine innere Phase, umfassend ein wasserlöslichen Konditionierungsmittel, gewählt aus einem oder mehreren wasserlöslichen Mitteln, sodass der gewichtete arithmetische mittlere Löslichkeitsparameter des wasserlöslichen Konditionierungsmittel größer als 10,5 ist, und
(B) eine äußere Phase, umfassend ein öllösliches Mittel, gewählt aus einem oder mehreren öllöslichen Mitteln, sodass der gewichtete arithmetische mittlere Löslichkeitsparameter des wasserlöslichen Konditionierungsmittel größer als 10,5 ist.

**14.** Produkt nach Anspruch 13, umfassend weiterhin 0.1 bis 20 Gew.-% der konditionierenden Emulsion eines Emulgiermittels, das fähig ist, eine Emulsion aus der inneren und äußeren Phase zu bilden, wobei das Emulgiermittel aus einem oder mehreren Emulgiermitteln gewählt ist, sodass der gewichtete, arithmetische mittlere HLB-Wert 1 bis 7 beträgt.

**15.** Produkt nach mindestens einem der Ansprüche 9 bis 14, wobei die konditionierende Komponente ein Oberflächen-zu-Sättigungsverhältnis von größer als oder gleich 1,25 an jedem Punkt der Oberfläche des Substrats besitzt.

**16.** Produkt nach mindestens einem der Ansprüche 9 bis 15, wobei die konditionierende Komponente einen Lipidhärtewert von größer als 0,02 kg besitzt.

**17.** Produkt nach mindestens einem der Ansprüche 1 bis 16, wobei das wasserunlösliche Substrat mindestens zwei Lagen aus Fasern umfasst, die jeweils verschiedene Texturen besitzen.

**18.** Produkt nach Anspruch 17, wobei das wasserunlösliche Substrat mindestens einen Bereich umfasst, der Nassausdehnbar ist, und mindestens einen zweiten Bereich, der weniger nass-ausdehnbar ist, als der erste Bereich.

**19.** Artikel nach Anspruch 17 oder 18, wobei das wasserunlösliche Substrat umfasst:

(A) eine löchrige erste Schicht, wobei die erste Schicht in der Ebene der ersten Schicht nass-ausdehnbar ist, wenn die erste Schicht benetzt wird; und
(B) eine zweite Schicht, die weniger nass-ausdehnbar ist, wenn sie benetzt wird, als die erste Schicht;

wobei ausgewählte Bereiche der ersten Schicht mit der zweiten Schicht verbunden sind, in einer Weise, welche ausreichend ist, um Nass-Ausdehnung der ersten Schicht in der Ebene der ersten Schicht zu verhindern.

**20.** Produkt nach mindestens einem der Ansprüche 1 bis 19, wobei das Reinigungsprodukt weiterhin eine sichere und wirksame Menge eines oder mehrerer Wirkstoffbestandteile umfasst, gewählt aus der Gruppe, bestehend aus Antiakne-Wirkstoffen, Antifalten- und Antihaut-Wirkstoffen, nicht-stearoidalen entzündungshemmenden Wirkstoffen, topischen Anästhetika, künstlichen Bräunungsmitteln und -beschleunigern, antimikrobiellen und Antipilz-Mitteln, Sonnenschutzwirkstoffen, Antioxidantsien und Mischungen hiervon.

**21.** Verfahren zur Herstellung eines wegwerfbaren, einmal verwendbaren Reinigungs- und Konditionierungsprodukts für die persönliche Pflege, das Materialien, welche von der Haut oder dem Haar abgespült werden, abscheidet, und das erwünschte Duftstoffabgabeeigenschaften besitzt, wobei das Verfahren **dadurch gekennzeichnet ist, dass** es die Schritte des separaten oder gleichzeitigen Zugebens auf oder Imprägnierens in ein wasserunlösliches Substrat umfasst

(A) mindestens eines schäumenden Tensids, zugegeben auf oder imprägniert in das Substrat, und
(B) 0,015 bis 15 Gew.-% des wasserunlöslichen Substrats eines duftstofffreisetzenden Komplexes, zugegeben auf oder imprägniert in das Substrat, wobei der duftstofffreisetzende Komplex umfasst:

(i) 10 bis 90 Gew.-% des Komplexes eines porösen Dufstoffträgers, und
(ii) 1 bis 90 Gew.-% des Komplexes eines innerhalb des Trägers imprägnierten Duftstoffs,

wobei das Produkt vor der Anwendung im Wesentlichen trocken ist.

**22.** Verfahren zur Herstellung eines Produkts nach Anspruch 21, wobei der duftstofffreisetzende Komplex eingekapselt ist mit einem Beschichtungsmaterial, gewählt aus der Gruppe, bestehend aus Paraffinwachsen, mikrokristallinen Wachsen, tierischen Wachsen, pflanzlichen Wachsen, gesättigten Fettsäuren und Fettalkoholen, Fettsäureestern, Zelluloseestern, Polyalkylenglykol, Polyvinylalkohol und Mischungen hiervon, und wobei das Beschichtungsmaterial in einer Menge im Bereich von 2% bis 50% des duftstofffreisetzenden Komplexes vorliegt.

**23.** Verfahren zur Herstellung eines Produkts nach Anspruch 21 oder Anspruch 22, umfassend den weiteren Schritt des separaten oder gleichzeitigen Zugebens auf oder Imprägnierens in ein wasserunlösliches Substrat von 0,1 bis 10 Gew.-% des Substrats eines unverdünnten Duftstoffs.

## Revendications

**1.** Produit cosmétique nettoyant et conditionneur à usage unique, jetable, qui dépose des substances éliminées de la peau ou des cheveux par rinçage, qui possède des propriétés souhaitables de diffusion de parfum et qui est **caractérisé en ce qu'**il comprend:

(A) un substrat insoluble dans l'eau,
(B) au moins un tensioactif moussant ajouté audit substrat ou imprégné dans celui-ci, et
(C) de 0,015 à 15%, en poids dudit substrat insoluble dans l'eau, d'un complexe libérateur de parfum ajouté audit substrat ou imprégné dans celui-ci, ledit complexe libérateur de parfum comprenant:

(i) de 10 à 90%, en poids du complexe, d'un support de parfum poreux, et
(ii) de 1 à 90%, en poids du complexe, d'un parfum imprégné dans ledit support,

ledit produit étant substantiellement sec avant l'utilisation.

**2.** Produit selon la revendication 1, dans lequel ledit tensioactif moussant constitue de 0,5 à 12,5% en poids dudit substrat insoluble dans l'eau, et dans lequel le rapport dudit parfum audit support de parfum poreux va de 5:1 à 1:10.

**3.** Produit selon la revendication 1 ou la revendication 2, dans lequel ledit support de parfum comprend des supports choisis dans le groupe constitué par la cyclodextrine, de préférence l'$\alpha$-cyclodextrine, la $\beta$-cyclodextrine, la $\gamma$-cyclodextrine ou des mélanges et des dérivés de celles-ci; les silices amorphes; les silices précipitées; les fumées de silice ; les silices colloïdales; les silices sphéroïdales; les aluminosilicates, de préférence, la zéolite, l'alumine ou leurs mélanges; les silicates de calcium; les amidons poreux; les amidons agglomérés; les copolymères de polyméthacrylate; et leurs mélanges.

**4.** Produit selon l'une quelconque des revendications 1 à 3, dans lequel ledit parfum est choisi dans le groupe constitué par les ingrédients de parfum, les ingrédients rafraîchissants, et leurs mélanges.

**5.** Produit selon l'une quelconque des revendications 1 à 4, dans lequel au moins une partie du parfum est choisie dans le groupe constitué par les composants de parfum hautement volatils ayant un point d'ébullition allant jusqu'à 250°C; des composants de parfum modérément volatils ayant un point d'ébullition de 250 à 350°C; et leurs mélanges.

**6.** Produit selon l'une quelconque des revendications 1 à 5, comprenant en outre de 0,01 à 10%, en poids du substrat, d'un parfum pur.

**7.** Produit selon l'une quelconque des revendications 1 à 6, dans lequel le complexe libérateur de parfum est encapsulé avec un matériau d'enrobage choisi dans le groupe constitué par les cires de paraffine; les cires microcristallines; les cires animales; les cires végétales; les acides gras et les alcools gras saturés; les esters gras; les esters de cellulose; les polyalkylèneglycols, de préférence, les polyéthylèneglycols, les polypropylèneglycols, les polyalkylèneglycols mixtes, et les copolymères de polyalkylèneglycol mixte; l'alcool polyvinylique; et leurs mélanges, et dans lequel le matériau d'enrobage est présent en une quantité allant de 2 à 50% du complexe libérateur de parfum.

**8.** Produit selon la revendication 7, dans lequel ledit support de parfum est la cyclodextrine, et dans lequel ledit matériau d'enrobage est le polyéthylèneglycol, de préférence ayant un poids moléculaire de 4400 à 400 000.

**9.** Produit selon l'une quelconque des revendications 1 à 8, comprenant en outre de 3 à 99%, en poids dudit substrat insoluble dans l'eau, d'un composant de conditionnement ajouté audit substrat ou imprégné dans celui-ci, ledit composant de conditionnement comprenant des substances choisies dans le groupe constitué par les agents de conditionnement solubles dans l'eau, les agents de conditionnement solubles dans l'huile, les émulsions de conditionnement, les durcisseurs de lipides et leurs mélanges.

**10.** Produit selon l'une quelconque des revendications 1 à 9, dans lequel ledit tensioactif moussant est choisi dans le groupe constitué par les tensioactifs anioniques moussants, les tensioactifs non ioniques moussants, les tensioactifs amphotères moussants, et leurs mélanges.

**11.** Produit selon l'une quelconque des revendications 1 à 10, dans lequel ledit substrat insoluble dans l'eau est choisi dans le groupe constitué par les substrats non tissés, les substrats tissés, les substrats hydroenchevêtrés, les éponges naturelles, les éponges synthétiques, les tamis polymères réticulés, les films traités, et leurs mélanges.

**12.** Produit selon l'une quelconque des revendications 9 à 11, dans lequel ledit agent de conditionnement soluble dans l'huile et ledit durcisseur de lipides sont choisis dans le groupe constitué par les acides gras, les esters d'acides gras, les alcools gras, les alcools éthoxylés, les polyesters de polyol, les monoesters de glycérine, les polyesters de glycérine, les hydrocarbures épidermiques et sébacés, la lanoline, l'huile minérale, l'huile de silicone, la gomme de silicone, les huiles végétales, les produits d'addition d'huile végétale, la vaseline, les polymères non ioniques, les huiles végétales hydrogénées, les polymères non ioniques, les cires naturelles, les cires synthétiques, les glycols polyoléfiniques, les monoesters polyoléfiniques, les polyesters polyoléfiniques, les cholestérols, les esters de cholestérol, et leurs mélanges; et dans lequel ledit agent de conditionnement soluble dans l'eau est choisi dans le groupe constitué par la glycérine, le glycérol, le propylèneglycol, les polypropylèneglycols, les polyéthylèneglycols, l'éthylhexanediol, les hexylèneglycols, d'autres alcools aliphatiques, le panthénol, l'urée, les polymères cationiques, les polyols, l'acide glycolique, l'acide lactique, et leurs mélanges.

**13.** Produit selon l'une quelconque des revendications 9 à 12, dans lequel ladite émulsion de conditionnement comprend:

(A) une phase interne comprenant un agent de conditionnement soluble dans l'eau choisi parmi un ou plusieurs agents solubles dans l'eau, de sorte que la moyenne arithmétique pondérée du paramètre de solubilité dudit agent de conditionnement soluble dans l'eau est supérieure à 10,5 et
(B) une phase externe comprenant un agent soluble dans l'huile choisi parmi un ou plusieurs agents solubles dans l'huile, de sorte que la moyenne arithmétique pondérée du paramètre de solubilité dudit agent de conditionnement soluble dans l'eau est supérieure à 10,5.

**14.** Produit selon la revendication 13, comprenant en outre de 0,1 à 20%, en poids de ladite émulsion de conditionnement, d'un émulsifiant capable de former une émulsion de ladite phase interne et de ladite phase externe, dans lequel ledit émulsifiant est choisi parmi un ou plusieurs émulsifiants, de sorte que la moyenne arithmétique pondérée de la valeur de HLB est de 1 à 7.

**15.** Produit selon l'une quelconque des revendications 9 à 14, dans lequel ledit composant de conditionnement a un rapport surface/saturation supérieur ou égal à 1,25 à un point quelconque de la surface du substrat.

**16.** Produit selon l'une quelconque des revendications 9 à 15, dans lequel ledit composant de conditionnement a une dureté lipidique supérieure à 0,02 kg.

**17.** Produit selon l'une quelconque des revendications 1 à 16, dans lequel ledit substrat insoluble dans l'eau comprend au moins deux feuilles de fibres ayant chacune des textures différentes.

**18.** Produit selon la revendication 17, dans lequel ledit substrat insoluble dans l'eau comprend au moins une partie qui est extensible à l'état humide et au moins une seconde partie qui est moins extensible à l'état humide que ladite première partie.

**19.** Produit selon la revendication 17 ou la revendication 18, dans lequel ledit substrat insoluble dans l'eau comprend:

(A) une première couche perforée, ladite première couche étant extensible à l'état humide dans le plan de la première couche lorsque celle-ci est mouillée; et
(B) une seconde couche qui est moins extensible à l'état humide une fois mouillée que ladite première couche;

dans lequel, des parties choisies de ladite première couche sont réunies à ladite seconde couche d'une manière suffisante pour empêcher l'extension à l'état humide de ladite première couche dans le plan de ladite première couche.

**20.** Produit selon l'une quelconque des revendications 1 à 19, dans lequel ledit produit nettoyant comprend en outre une quantité sans danger et efficace d'un ou de plusieurs ingrédients actifs choisis dans le groupe constitué par les agents actifs anti-acnéiques, les agents actifs antirides et de protection de la peau, les agents actifs anti-inflammatoires non stéroïdiens, les anesthésiques locaux, les agents et accélérateurs de bronzage artificiel, les agents antimicrobiens et antifongiques, les agents actifs de protection solaire, les antioxydants et leurs mélanges.

**21.** Procédé de fabrication d'un produit cosmétique nettoyant et conditionneur à usage unique, jetable, qui dépose des substances éliminées de la peau ou des cheveux par rinçage et qui possède des propriétés souhaitables de diffusion de parfum, lequel procédé est **caractérisé en ce qu'**il comprend les étapes consistant à ajouter sur ou imprégner dans un substrat insoluble dans l'eau, séparément ou simultanément:

(A) au moins un tensioactif moussant ajouté sur ledit substrat ou imprégné dans celui-ci, et
(B) de 0,015% à 15%, en poids dudit substrat insoluble dans l'eau, d'un complexe libérateur de parfum ajouté audit substrat ou imprégné dans celui-ci, ledit complexe libérateur de parfum comprenant:

(i) de 10 à 90%, en poids du complexe, d'un support de parfum poreux, et
(ii) de 1 à 90%, en poids du complexe, d'un parfum imprégné dans ledit support,

ledit produit étant substantiellement sec avant l'utilisation.

**22.** Procédé de fabrication d'un produit selon la revendication 21, dans lequel ledit complexe libérateur de parfum est encapsulé avec un matériau d'enrobage choisi dans le groupe constitué par les cires de paraffine, les cires microcristallines, les cires animales, les cires végétales, les acides gras et les alcools gras saturés, les esters gras, les esters cellulosiques, les polyalkylèneglycols, l'alcool polyvinylique, et leurs mélanges, et dans lequel le matériau d'enrobage est présent en une quantité allant de 2% à 50% du complexe libérateur de parfum.

**23.** Procédé de fabrication d'un produit selon la revendication 21 ou la revendication 22, comprenant l'étape supplémentaire qui consiste à ajouter sur ou imprégner dans un substrat insoluble dans l'eau, séparément ou simultanément, de 0,1 à 10%, en poids du substrat, d'un parfum pur.

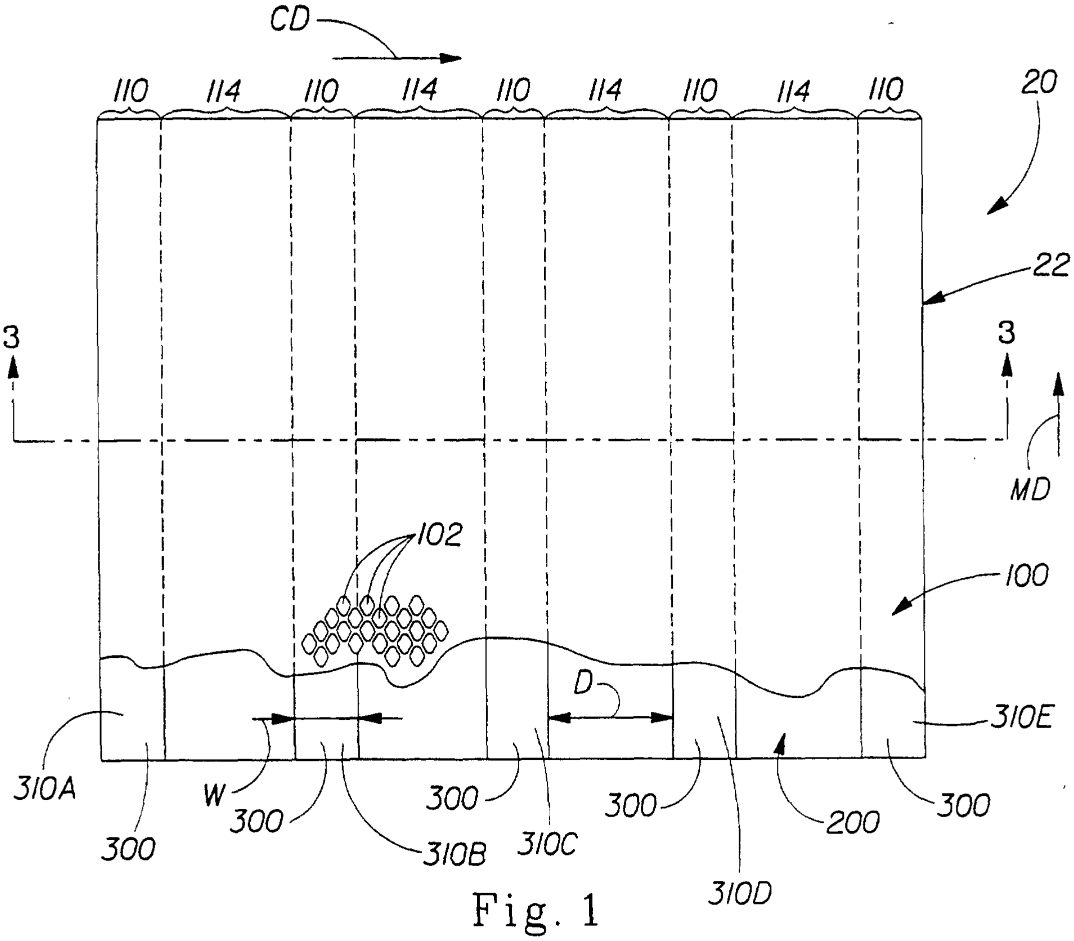
CD
110
114
110
114
110
114
110
114
110
20
22
3
3
MD
102
100
D
310E
310A
W
300
300
300
310B
310C
300
300
200
300
310D

Fig. 1

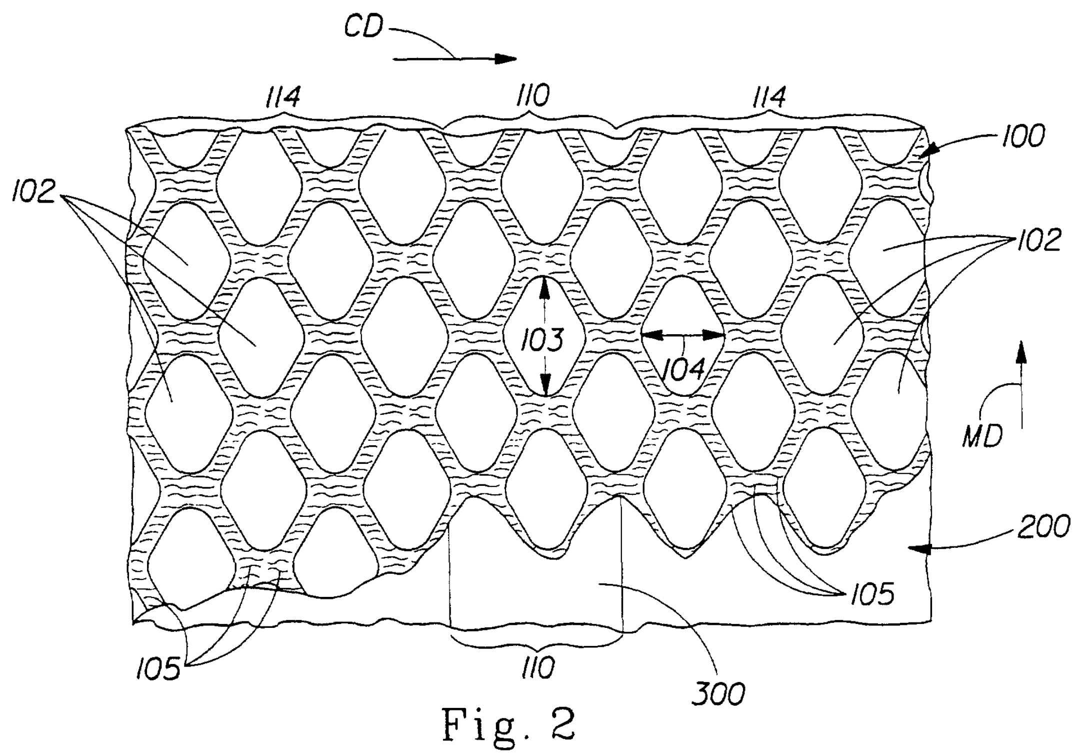
CD
114
110
114
100
102
102
103
104
MD
200
105
105
110
300

Fig. 2

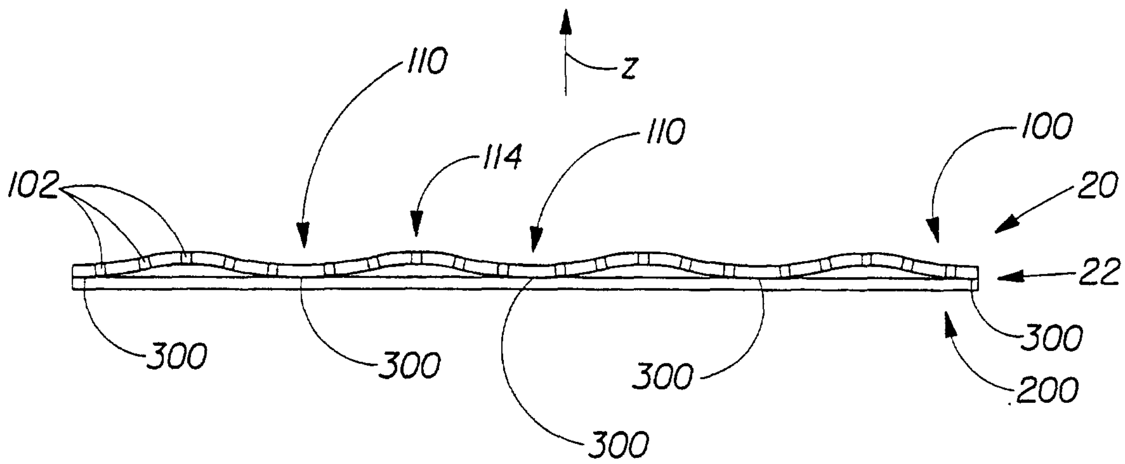
z
110
110
114
100
20
102
22
300
300
300
300
200
300

Fig. 3A

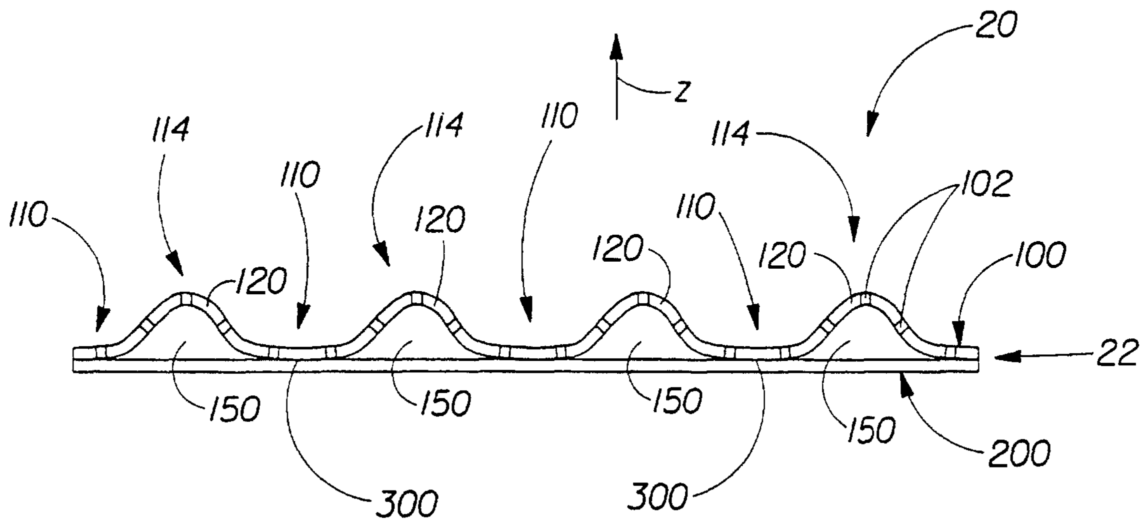
20
z
114
114
110
114
110
110
110
102
120
120
120
120
100
22
150
150
150
150
200
300
300

Fig. 3B

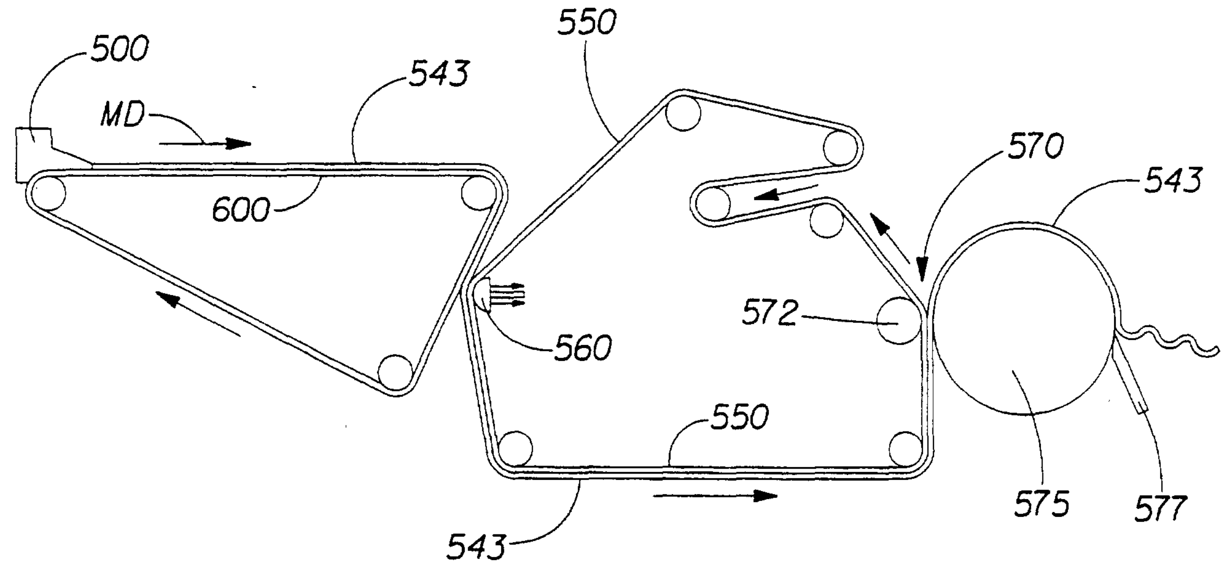
500
MD
543
550
570
543
600
560
572
550
575
577
543

Fig. 4

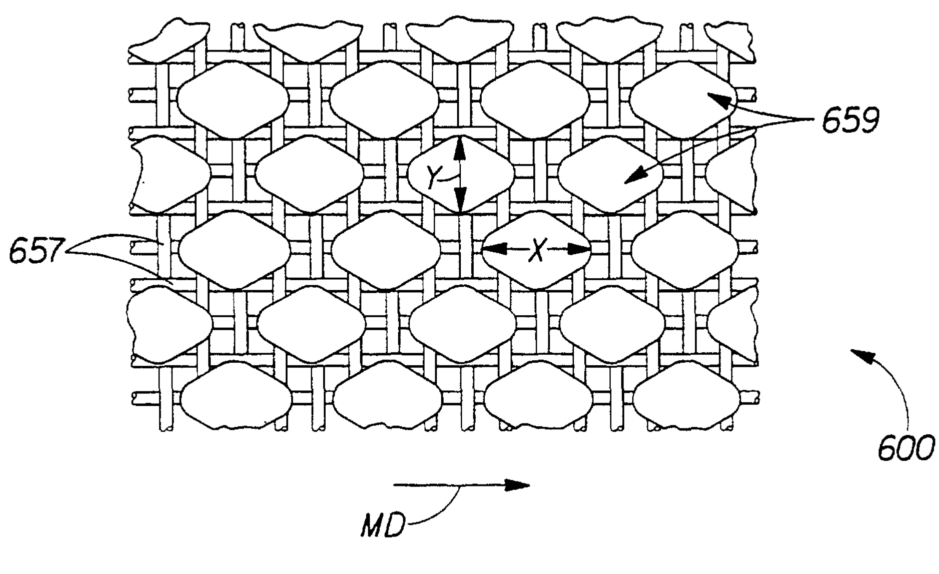
659
Y
X
657
600
MD

Fig. 5